(19) Europäisches Patentamt
European Patent Office
Office européen des brevets

(11) Veröffentlichungsnummer: **0 222 961 B1**

# (12) EUROPÄISCHE PATENTSCHRIFT

(45) Veröffentlichungstag der Patentschrift: **20.09.89**

(51) Int. Cl.⁴: **C07D 257/04**, C07C 103/38, C07C 93/14, A61K 31/41, A61K 31/19, A61K 31/215, C07C 39/24, C07C 49/825

(21) Anmeldenummer: **85810550.5**

(22) Anmeldetag: **18.11.85**

## (54) Neue fluorierte Resorcinether.

(43) Veröffentlichungstag der Anmeldung: **27.05.87 Patentblatt 87/22**

(45) Bekanntmachung des Hinweises auf die Patenterteilung: **20.09.89 Patentblatt 89/38**

(84) Benannte Vertragsstaaten: **AT BE CH DE FR GB IT LI LU NL SE**

(56) Entgegenhaltungen:
**EP-A- 0 086 746**
**EP-A- 0 123 541**
**EP-A- 0 132 366**
**EP-A- 0 132 367**
**EP-A- 0 147 973**
**DE-A- 2 616 479**
**DE-A- 2 653 601**
**DE-A- 2 930 728**
**US-A- 4 448 729**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel(CH)**

(72) Erfinder: **Beck, Andreas, Dr., Kohlerweg 23, D-7800 Freiburg(DE)**
Erfinder: **Sallmann, Alfred, Dr., Joachimsackerstrasse 12, CH-4103 Bottmingen(CH)**
Erfinder: **Lang, Robert Werner, Dr., Hagenbachweg 10, CH-4133 Pratteln(CH)**
Erfinder: **Wenk, Paul, Dr., Quellenweg 7, CH-4123 Allschwil(CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue 2-fluoralkylierte 4-Acylresorcinether der Formel

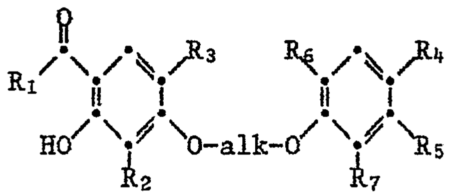

(I),

worin $R_1$ Niederalkyl bedeutet, $R_2$ fluoriertes Niederalkyl darstellt, $R_3$ Wasserstoff, Niederalkoxy, Trifluormethyl oder Halogen bedeutet, alk einen Alkylenrest mit 2 bis und mit 9 Kettengliedern und mit 2 bis und mit 9 C-Atomen, einen Hydroxyalkylenrest mit 3 bis und mit 7 Kettengliedern und mit 3 bis und mit 7 C-Atomen, einen durch Sauerstoff unterbrochenen Alkylenrest mit 5 bis und mit 19 Kettengliedern und mit 4 bis und mit 16 C-Atomen oder einen durch Sauerstoff unterbrochenen Hydroxyalkylenrest mit 6 bis und mit 11 Kettengliedern und mit 5 bis und mit 9 C-Atomen darstellt, einen der Reste $R_4$, $R_5$ und $R_7$ für eine Gruppe der Formel $-NH-C(=O)-R_8$, ein davon verschiedener Rest $R_4$ oder $R_5$ für einen Rest $R_9$ und ein davon verschiedener Rest $R_7$ für einen Rest $R_{10}$ steht, $R_6$ Wasserstoff, Niederalkyl, Trifluormethyl, Halogen, gegebenenfalls verestertes oder amidiertes Carboxy, Cyano oder Niederalkanoyl darstellt, $R_8$ gegebenenfalls verestertes oder amidiertes Carboxy oder 5-Tetrazolyl bedeutet, $R_9$ Wasserstoff, Niederalkyl, Niederalkoxy, Halogen oder Trifluormethyl bedeutet und $R_{10}$ für Wasserstoff, Niederalkyl, Niederalkoxy, Halogen, Trifluormethyl, Cyano oder gegebenenfalls verestertes oder amidiertes Carboxy steht, wobei Halogen eine Atomnummer bis und mit 53 aufweisen kann und "niedere" Gruppen bis und mit 7 C-Atome haben können, und ihre Salze.

Alkylenreste alk können bis und mit 9 Kettenglieder aufweisen und sind beispielsweise geradkettige Alkylenreste der Formel

$$-(CH_2)_m- \qquad (Ia),$$

worin m eine ganze Zahl von 2 bis und mit 9 bedeutet, können aber auch verzweigte, insbesondere in höherer als der α- und in niedrigerer als der ω-Stellung verzweigte, Alkylenreste sein und sind vorzugsweise Niederalkylenreste (geradkettig oder verzweigt) der genannten Art.

Durch Sauerstoff unterbrochene Alkylenreste sind beispielsweise Mono-, Di- oder Trioxaalkylenreste, z.B. der Formel

$$-(CH_2)_{n'}-[O-(CH_2)_n]_k- \qquad (Ib),$$

worin n und n' unabhängig voneinander 2, 3 oder 4 bedeuten und k für 1, 2 oder 3 steht, und bedeuten insbesondere Oxa- oder Dioxaniederalkylenreste, z.B der Formel Ib, worin n und n' für 2 und k für 1 oder 2 steht.

Ggf. durch Sauerstoff unterbrochene Hydroxyalkylenreste sind beispielsweise Hydroxyalkylen-, Hydroxy(oxa)alkylen- oder Hydroxy(dioxa)alkylenreste, in denen die Hydroxygruppe in höherer als der α- und niedrigerer als der ω-Stellung gebunden ist, z.B. Reste der Formel

$$-[(CH_2)_{l'}-O]_o-CH_2CH(OH)CH_2-[O-(CH_2)_l]_p- \qquad (Ic),$$

worin 1 und 1' unabhängig voneinander 2 oder 3 bedeuten und o und p unabhängig voneinander für 0 oder 1 stehen, und stellen insbesondere entsprechende Hydroxyniederalkylenreste, ferner Hydroxy(oxa)niederalkylenreste dar.

Verestertes Carboxy ist beispielsweise Niederalkoxycarbonyl, kann im Falle von $R_8$ aber auch N,N-Diniederalkylaminoniederalkoxycarbonyl, N,N-Niederalkylenaminoniederalkoxycarbonyl, gegebenenfalls substituiertes N,N-(Aza)niederalkylenaminoniederalkoxycarbonyl, N,N-(Oxa)niederalkylenaminoniederalkoxycarbonyl oder N,N-(Thia)niederalkylenaminoniederalkoxycarbonyl sein.

Amidiertes Carboxy ist beispielsweise Carbamyl, N-Mono- oder N,N-Diniederalkylcarbamyl, ferner N,N-Niederalkylen- bzw. N,N-(Aza)niederalkylen-, N,N-(Oxa)niederalkylen- oder N,N-(Thia)niederalkylencarbamyl.

Vor- und nachstehend sind unter "niederen" organischen Verbindungen und davon abgeleiteten Gruppen beispielsweise solche zu verstehen, die bis und mit 7, insbesondere bis und mit 4, Kohlenstoffatome (C-Atome) aufweisen.

Fluoriertes Niederalkyl weist beispielsweise bis und mit 3, insbesondere terminal gebundene, Fluoratome auf und bedeutet beispielsweise Mono-, Di- oder Trifluor-$C_1$–$C_4$-alkyl, wie Trifluormethyl, 3-Fluorpropyl, 3,3-Difluorpropyl oder 3,3,3-Trifluorpropyl.

Niederalkyl bedeutet beispielsweise $C_1$–$C_4$-Alkyl, wie Methyl, Ethyl, Propyl, Isopropyl oder Butyl, ferner Sekundär- oder Tertiärbutyl.

Niederalkoxy bedeutet beispielsweise $C_1$–$C_4$-Alkoxy, wie Methoxy, Ethoxy, Propyloxy, Isoporoxy oder Butyloxy.

Halogen weist beispielsweise eine Atomnummer bis und mit 53, insbesondere von 17 bis und mit 53, auf und bedeutet beispielsweise Fluor, Chlor, Brom oder Iod.

Niederalkanoyl ist beispielsweise $C_1$–$C_7$-Alkanoyl, wie Formyl, Acetyl, Propionyl, Butyryl, Valeroyl oder Pivaloyl.

Niederalkylen ist beispielsweise geradkettiges $C_2$–$C_7$-Alkylen, wie 1,2-Ethylen, 1,3-Propylen, 1,4-Butylen, 1,5-Pentylen, 1,6-Hexylen oder 1,7-Heptylen, kann aber auch verzweigtes $C_3$–$C_6$-Alkylen, wie 1,2-Propylen, 1,3-butylen, 2,4-Pentylen, 1,3-(2-Methyl)-propylen oder 1,3-(2,2Dimethyl)-propylen, sein.

Oxaniederalkylen ist beispielsweise 3- oder 4-Oxa-$C_5$–$C_7$-alkylen, wie 1,5-(3-Oxa)pentylen oder 1,7-(4-Oxa)heptylen; Dioxaniederalkylen ist beispielsweise 1,7-(3,5-dioxa)heptylen.

Hydroxyniederalkylen ist beispielsweise 2-, 3- oder 4-Hydroxy-$C_3$–$C_7$-alkylen, wie 1,3-(2-Hydroxy)propylen, 1,4-(2-Hydroxy)butylen, 1,5-(3-Hydroxy)pentylen, 1,6-(2-Hydroxy)hexylen oder 1,7-(4-Hydroxy)heptylen. Hydroxy(oxa)niederalkylen ist beispielsweise 2-Hydroxy-4-oxa-$C_6$–$C_7$-alkylen, wie 1,6-(2-Hydroxy-4-oxa)hexylen oder 1,7-(2-Hydroxy-4-oxa)heptylen.

Niederalkoxycarbonyl ist beispielsweise $C_1$–$C_4$-Alkoxycarbonyl, wie Methoxy-, Ethoxy-, Propyloxy-, Isopropyloxy- oder Butyloxycarbonyl.

N,N-Diniederalkylaminoniederalkoxycarbonyl ist beispielsweise N,N-Di-$C_1$–$C_4$-alkylamino-$C_2$–$C_4$-alkoxycarbonyl, wie 2-(Dimethylamino)ethoxycarbonyl, 2-(Diethylamino)ethoxycarbonyl oder 3-(Dimethylamino)propyloxycarbonyl.

N,N-Niederalkylenaminoniederalkoxycarbonyl ist beispielsweise 5- bis 7-gliedriges N,N-Alkylenamino-$C_2$–$C_4$-alkoxycarbonyl, wie 2-(Pyrrolidino)-, 2-(Piperidino)- oder 2-(Tetrahydroazepino)ethoxycarbonyl.

Gegebenenfalls substituiertes N,N-(Aza)niederalkylenaminoniederalkoxycarbonyl ist beispielsweise 5- bis 7-gliedriges N,N-(Aza)alkylenamino-$C_2$–$C_4$-alkoxycarbonyl, wie 2-(Piperazino)ethoxycarbonyl oder 2-(4-Methylpiperazino)ethoxycarbonyl.

N,N-(Oxa)- bzw. N,N-(Thia)niederalkylaminoniederalkoxycarbonyl ist beispielsweise 5- bis 7- gliedriges N,N-(Oxa)- bzw. N,N-(Thia)alkylenamino-$C_2$–$C_4$-alkoxycarbonyl, wie 2-(Morpholino)-bzw. 2-(Thiomorpholino)-ethoxycarbonyl.

N-Mono- oder N,N-Diniederalkylcarbamyl ist beispielsweise N–$C_1$–$C_7$-Alkyl- oder N,N-Di-$C_1$–$C_4$-Alkylcarbamyl, wie N-Methyl-, N-Ethyl- oder N,N-Dimethylcarbamyl.

N,N-Niederalkylencarbamyl bzw. N,N-(Aza)-, N,N-(Oxa)- oder N,N-(Thia)-niederalkylencarbamyl ist beispielsweise 5- bis 7-gliedriges N,N-Alkylen-, N,N-(Aza)alkylen-, N,N-(Oxa)alkylen- oder N,N-(Thia)alkylencarbamyl, wie Pyrrolidino-, Piperidino-, Pyridazino-, (4-Methyl)-piperazino-, Morpholino- oder Thiomorpholinocarbonyl.

Als Salze von Verbindungen der Formel I kommen vorzugsweise pharmazeutisch verwendbare Salze in Frage, wie Metallsalze, Ammoniumsalze oder Salze mit organischen Basen. Metallsalze sind beispielsweise entsprechende Alkali-, Erdalkalimetallsalze, z.B. Lithium-, Natrium-, Kalium-, Magnesium- oder Calciumsalze, ferner pharmazeutisch verwendbare Übergangsmetall-, wie Zink- oder Kupfersalze. Salze mit organischen Basen werden beispielsweise von Verbindungen der Formel I, worin $R_6$ und/oder $R_7$ Carboxy und/oder $R_8$ Carboxy oder 5-Tetrazolyl ist, mit mono-, di- oder trisubstituierten organischen Aminen, wie entsprechenden Alkylaminen, Hydroxyalkylaminen, geeigneten, mindestens ein N-Atom aufweisenden Heterocyclen, wie Morpholin, Thiomorpholin, Piperidin oder Pyrrolidin, gegebenenfalls N-substituierten Amino-sacchariden, z.B. mit N-Methyl-D-glucamin, oder basischen Aminosäuren, wie Lysin, Arginin, Histidin oder Ornithin, wobei jene mit L-Konfiguration bevorzugt sind, gebildet. Als Alkylamine kommen z.B. Mono-, Di- oder Triniederalkylamine, wie Ethyl-, tert-Butyl, Diethyl-, Diisopropyl-, Trimethyl- oder Triethylamin, in Betracht. Hydroxyalkylamine sind beispielsweise Mono-, Di- oder Trihydroxyalkylamine, wie Mono-, Di- bzw. Triethanolamin oder Diisopropanolamin, oder Hydroxyniederalkylniederalkylamine, wie N,N-Dimethyl- bzw. N,N-Diethylamino-ethanol oder Tris(hydroxymethyl)methylamin.

Als weitere Salze sind pharmazeutisch verwendbare Säureadditionssalze, wie Hydrohalogenide, Methansulfonate, N-Cyclohexylsulfaminate, Maleinate, Fumarate, Maleate oder Tartrate, von Verbindungen der Formel I, worin der Rest $R_8$ zur Bildung entsprechender Salze befähigt ist, zu nennen.

Die Verbindungen der Formel I mit chiralen C-Atomen können je nach der Anzahl derselben in zueinander enantiomeren bzw. diastereomeren Formen oder als Gemische derselben, wie Diastereomerengemische, Racemate oder Racematgemische, vorliegen.

Die neuen Verbindungen zeichnen sich durch wertvolle pharmakologische Eigenschaften aus.

So weisen sie eine auf einem ausgeprägten $LTD_4$-(Leukotrien-$D_4$)- sowie PAF-(PAF-Acetether)-Antagonismus beruhende antiallergische Wirkung auf. Die $LTD_4$-antagonistischen Eigenschaften der erfindungsgemässen Verbindungen können beispielsweise <u>in vitro</u> anhand ihrer in Konzentrationen ab etwa 0,03 bis etwa 0,10 µmol/l nachweisbaren Hemmwirkung auf durch $LTD_4$ ausgelöste Kontraktionen des isolierten Meerschweinchen-Ileums und in vivo anhand ihrer bei intravenöser Behandlung in Dosen ab etwa 0,08 mg/kg bzw. bei Aerosolbehandlung ab einem Wirkstoffgehalt von etwa 0,025 Gew.-% nachweisbaren Hemmwirkung auf durch $LTD_4$ ausgelöste Bronchospasmen des Meerschweinchens gezeigt werden. Sie weisen ferner eine ausgeprägte $LTD_4$-Synthesehemmung auf, die in vitro anhand der Aggregationshemmung von peritonealen PMN der Ratte gezeigt werden kann.

In der US-Patentschrift Nr. 4 448 729 werden unter anderem schon Verbindungen mit antiallergischer Wirksamkeit offenbart, die von der Grundstruktur her den Verbindungen der Formel I ähneln, jedoch anstelle der fluorierten $C_1$–$C_7$-Alkylgruppe $R_2$ eine $C_1$–$C_6$-Alkylgruppe $R_1$ aufweisen, und in EP-A 147 973 werden unter anderem bereits Verbindungen mit antiallergischer und antiinflammatorischer Wirksamkeit sowie Leukotriensynthese-hemmenden Eigenschaften beschrieben, die ebenfalls eine ähnliche Grundstruktur wie die Verbindungen der Formel I besitzen, sich von diesen jedoch ebenfalls dadurch unterscheiden, dass sie anstelle der fluorierten $C_1$–$C_7$-Alkylgruppe $R_2$ eine unsubstituierte $C_1$–$C_6$-Alkylgruppe aufweisen.

Der Vorteil der erfindungsgemässen Verbindungen der Formel I ist demgegenüber insbesondere darin zu sehen, dass sie, bei teilweise längerer Wirkungsdauer, eine erheblich grössere antiallergische Aktivität sowie zusätzlich zu der erwähnten $LTD_4$-antagonistischen Wirkung für diese Verbindungsklasse neuartige Phospholipasen-hemmende Eigenschaften aufweisen.

Die erfindungsgemässen Verbindungen der Formel I besitzen weiterhin eine ausgeprägte anti-inflammatorische bzw. hautphlogistatische Wirkung und ebenso eine ausgeprägte Hemmwirkung auf die Leukotrien-$B_4$-biosynthese, wie sich in vitro in Konzentrationen ab etwa 0,5 µmol/l zeigen lässt, die selbst schon sehr wertvoll sind und zudem die antiallergische Wirkung in wünschenswerter Weise ergänzen. Die Phospholipasen-hemmenden Eigenschaften können beispielsweise in vitro anhand der in Konzentrationen ab etwa 10 µmol/l nachweisbaren Hemmung der Aktivität von Phospholipasen $A_2$ (aus menschlichen Leukozyten) und C (aus menschlichen Thrombozyten) und die antiinflammatorischen bzw. hautphlogistischen Eigenschaften in vivo anhand der Hemmwirkung auf das experimentelle Crotonöl-Ohroedem der Ratte in Konzentrationen ab etwa 10 mg/ml gezeigt werden.

Die erfindungsgemässen Verbindungen können dementsprechend als Antiallergika, beispielsweise zur Behandlung von Asthma, Heufieber, Rhinitis und Hautallergien, insbesondere aber als Antiinflammatorika, vor allem zur Behandlung entzündlicher Erkrankungen des rheumatischen Formenkreises, sowie als Haut- bzw. Schleimhautphlogistatika, zur Behandlung entzündlicher Dermatosen verschiedenartiger, insbesondere jedoch allergischer, Genese, beispielsweise zur Behandlung entzündlicher Hautirritationen, Kontaktdermatiden, Exanthemen, Verbrennungen und von Mukosaentzündungen der Augen, Lippen, des Mundes sowie der Genital- bzw. Analregion, eingesetzt werden.

Die Hemmwirkung auf das experimentelle Ohroedem der Ratte kann nach G. Tonelli und L. Thibault, Endocrinology 77, 625 (1965) erfolgen. Zur Ermittlung der übrigen der erwähnten Eigenschaften können z.B. die nachfolgend beschriebenen Versuchsanordnungen herangezogen werden.

Hemmwirkung auf $LTD_4$-induzierte Kontraktionen des Meerschweinchen-Ileums

An Ileumsegmenten, die aus Meerschweinchen von 300 bis 400 g Körpergewicht entnommen, in einem Organbad in Tyrodelösung (38°C, Begasung mit einem Gemisch aus 95% $O_2$ und 5% $CO_2$) befestigt und mit 1p belastet wurden, werden durch synthetisches $LTD_4$ (Leukotrien $D_4$, Kaliumsalz) Kontraktionen ausgelöst, deren Ausmass registriert wird. Das Ausmass der auf die $LTD_4$-antagonistische Wirkung der Testsubstanz zurückzuführenden Hemmung dieser Kontraktionen wird gemessen. Man ermittelt diejenige, als $IC_{50}$ bezeichnete, Konzentration der Testsubstanz, die durch $LTD_4$ induzierte Kontraktionen auf 50% des Ausgangswertes reduziert. In dieser Versuchsanordnung wurde beispielsweise für das Natriumsalz der N-{3-[3-(4-Acetyl-3-hydroxy-2-propyl-phenoxy)-2-hydroxy-propyloxy]-2-cyanophenyl}-oxaminsäure gemäss US-Patentschrift Nr. 4 448 729 ein $IC_{50}$-Wert von 0,087 µmol/l und für das erfindungsgemässe Natriumsalz des N-{{3-{3-[4-Acetyl-3-hydroxy-2-(3,3,3-trifluorpropyl)-phenoxy]propyloxy}-4-brom-6-methyl-phenyl}}-1H-tetrazol-5-carboxamids ein $IC_{50}$-Wert von 0,012 µmol/l erhalten.

In vitro-Test zur Bestimmung der Hemmung von Phospholipase $A_2$ aus menschlichen Leukozyten

Neutrophile polymorphkernige menschliche Leukozyten werden, ausgehend von "buffy coats", durch mehrstufige fraktionierte Sedimentation isoliert und tiefgefroren. Die Phospholipase $A_2$ wird aus der Zellsuspension durch Homogenisieren unter Zusatz von eiskalter 0,36 N–$H_2SO_4$ in 2M–NaCl extrahiert und der nach Zentrifugieren bei 10 000 g erhaltene Überstand gegen Natriumacetatpuffer (pH 4,5) dialysiert.

Für die Bestimmung der Enzymaktivität wird Enzym (10–39 µg Protein) in 0,1 M-Tris/HCl-Puffer (pH 7) unter Zusatz von 1 mM $CaCl_2$ und Substrat, bestehend aus biosynthetisch mit $^{14}C$-Ölsäure radioaktiv markierten Phospholipiden (2 µM) von Escherichia coli, bei 37°C während 1 Stunde inkubiert. Die Reaktion wird abgestoppt durch Zugabe von Dole-Reagens (Isopropanol/Heptan/1N–$H_2SO_4$ 40:10:1, v/v) und die durch Phospholipase $A_2$ selektiv freigesetzte $^{14}C$-Ölsäure extrahiert. Ebenfalls mitextrahiertes

Substrat wird durch Filtration des Extraktes durch eine Säule von Kieselgel vollständig entfernt. Die Bestimmung der $^{14}C$-Ölsäure im Eluat erfolgt durch Radiometrie.

Zur Ermittlung einer Hemmwirkung von Prüfsubstanzen auf die Phospholipase $A_2$ werden diese als Lösungen in Wasser, Dimethylsulfoxid (Endkonzentration im Ansatz bis 5 Vol-%) oder Ethanol (Endkonzentration im Ansatz bis 2,5 Vol-%) dem Inkubationsansatz zugesetzt. Die Wirkungsstärke der Prüfsubstanzen wird ausgedrückt durch die $IC_{50}$, d.h. die Konzentration, welche eine Hemmung von 50% der Kontrollaktivität bewirkt. Die $IC_{50}$ wird graphisch ermittelt durch Auftragen der prozentualen Hemmung auf der Ordinate gegen den Logarithmus der Konzentration (µM) auf der Abszisse.

In dieser Versuchsanordnung wurde beispielsweise für das erfindungsgemässe Natriumsalz von N-{{3-{3-[4-Acetyl-3-hydroxy-2-(3,3,3-trifluorpropyl)-phenoxy]-propyloxy}-4-brom-6-methyl-phenyl}}-1H-tetrazol-5-carboxamid ein $IC_{50}$-Wert von 12 µmol/l und für das erfindungsgemässe N-{{3-{3-[4-Acetyl-3-hydroxy-2-(3,3,3-trifluorpropyl)-phenoxy]propyloxy}-4-chlor-6-methyl-phenyl}}-1H-tetrazol-5-carboxamid ein $IC_{50}$-Wert von 13 µmol/l erhalten, wohingegen das Natriumsalz der N-{3-[3-(4-Acetyl-3-hydroxy-2-propyl-phenoxy)-2-hydroxy-propyloxy]-2-cyano-phenyl}-oxaminsäure gemäss US-Patentschrift Nr. 4 448 729 auch bei 100 µm/l noch keine Wirkung zeigte.

Calcium-Ionophor-induzierte Synthese von $LTB_4$

Peritoneal-Exsudat (Neutrophile) von Wistar Ratten (RA 25, männlich) gewinnt man 24 Stunden nach i.p.-Injektion von 12% Natriumcaseinat. Zellen (1 x $10^7$/ml) werden durch Ca-Ionophor A 23187 (1 x $10^{-6}$ M) während 4 Minuten stimuliert. Überstände werden auf ihre Fähigkeit, PMN-Aggregation in vitro auszulösen, untersucht. Hemmsubstanzen in DMSO werden 5 Minuten vor der Zugabe des Ionophors zugegeben.

Die Resultate werden in % Hemmung der Kontrolle (ohne Hemmer) und als $IC_{50}$ angegeben.

Die Erfindung betrifft in erster Linie Verbindungen der Formel I, worin $R_1$ Niederalkyl bedeutet, $R_2$ fluoriertes Niederalkyl mit bis und mit 3 Fluoratomen bedeutet, $R_3$ Wasserstoff, Niederalkoxy, Trifluormethyl oder Halogen darstellt, alk für gegebenenfalls durch Sauerstoff unterbrochenes Alkylen bzw. Hydroxyalkylen der Formeln $-(CH_2)_m-$ (Ia),

$$-(CH_2)_{n'}-[O-(CH_2)_n]_k- \text{ (Ib) oder}$$
$$-[(CH_2)_{l'}-O]_o-CH_2-CH(OH)-CH_2-[O-(CH_2)_l]_p- \text{ (Ic)}$$

steht, worin k für 1, 2 oder 3 steht, l und l' unabhängig voneinander 2 oder 3 bedeuten, m eine ganze Zahl von 2 bis und mit 9 bedeutet, n und n' unabhängig voneinander 2, 3 oder 4 bedeuten und o und p unabhängig voneinander für 0 oder 1 stehen, einer der Reste $R_4$, $R_5$ und $R_7$ für eine Gruppe der Formel $-NH-C(=O)-R_8$, ein davon verschiedener Rest $R_4$ oder $R_5$ für einen Rest $R_9$ und ein davon verschiedener Rest $R_7$ für einen Rest $R_{10}$ steht, $R_6$ Wasserstoff, Niederalkyl, Trifluormethyl, Halogen, Niederalkanoyl, Carboxy, Niederalkoxycarbonyl, Cyano, Carbamyl, N-Mono- oder N,N-Diniederalkylcarbamyl bedeutet, $R_8$ einerseits Carboxy, Niederalkoxycarbonyl, N,N-Diniederalkylaminoniederalkoxycarbonyl, N,N-Niederalkylenaminoniederalkoxycarbonyl, Carbamyl, N-Mono- oder N,N-Diniederalkylcarbamyl, N,N-Niederalkylen- oder N,N-(Aza)niederalkylen-, N,N-(Oxa)niederalkylen- bzw. N,N-(Thia)niederalkylencarbamyl oder andererseits 5-Tetrazolyl bedeutet, $R_9$ Wasserstoff, Niederalkyl, Niederalkoxy, Halogen oder Trifluormethyl bedeutet und $R_{10}$ für Wasserstoff, Niederalkyl, Niederalkoxy, Halogen, Trifluormethyl, Carboxy, Niederalkoxycarbonyl, Cyano, Carbamyl, N-Mono- oder N,N-Diniederalkylcarbamyl steht, wobei Halogen eine Atomnummer bis und mit 53 aufweisen kann und "niedere" Gruppen bis und mit 7 C-Atome haben können, und ihrer Salze, insbesondere pharmazeutisch verwendbaren Salze.

Die Erfindung betrifft vorzugsweise Verbindungen der Formel I, worin $R_1$ Niederalkyl mit bis und mit 4 C-Atomen, wie Methyl, ist, $R_2$ ω-Fluor-, ω,ω-Difluor- oder ω,ω,ω-Trifluorniederalkyl mit bis und mit 4 C-Atomen, wie Trifluormethyl, 3,3,3-Trifluorpropyl, ferner 3-Fluorpropyl oder 3,3 Difluorpropyl bedeutet, $R_3$ für Wasserstoff steht, $R_4$ Wasserstoff, Niederalkyl mit bis und mit 4 C-Atomen, wie Methyl, Trifluormethyl oder Halogen mit einer Atomnummer von 17 bis und mit 53, wie Chlor oder Brom, bedeutet, $R_5$ eine Gruppe der Formel $-NH-C(=O)-R_8$ darstellt, $R_6$ für Wasserstoff, Niederalkyl mit bis und mit 4 C-Atomen, wie Methyl, Halogen mit einer Atomnummer von 17 bis und mit 53, wie Chlor oder Brom, Trifluormethyl, Niederalkoxycarbonyl mit bis und mit 5 C-Atomen, wie Ethoxycarbonyl, Cyano oder Carboxy steht, $R_7$ Wasserstoff, Niederalkyl mit bis und mit 4 C-Atomen, wie Methyl, Halogen mit einer Atomnummer von 17 bis und mit 53, wie Chlor, Carbamyl oder Cyano bedeutet, $R_8$ für Carboxy, Niederalkoxycarbonyl mit bis und mit 5 C-Atomen, wie Methoxy- oder Ethoxycarbonyl, oder 5-Tetrazolyl steht und alk geradkettiges Niederalkylen mit 2 bis und mit 7 C-Atomen, wie 1,3-Propylen, oder Hydroxyniederalkylen mit 3 bis und mit 7 C-Atomen, in dem die Hydroxygruppe in höherer als der α-Stellung und in niedrigerer als der ω-Stellung gebunden ist, wie 1,3-(2-Hydroxy)propylen, bedeutet, und ihre Salze, insbesondere pharmazeutisch verwendbaren Salze, mit Basen.

Die Erfindung betrifft insbesondere Verbindungen der Formel I, worin $R_1$ Niederalkyl mit bis und mit 4 C-Atomen, wie Methyl, ist, $R_2$ ω-Fluor-, ω,ω-Difluor oder ω,ω,ω-Trifluorniederalkyl mit bis und mit 4

C-Atomen, wie 3,3,3-Trifluorpropyl, ferner 3-Fluorpropyl oder 3,3-Difluorpropyl, bedeutet, $R_3$ für Wasserstoff steht, $R_4$ Wasserstoff, Niederalkyl mit bis und mit 4 C-Atomen, wie Methyl, Trifluormethyl oder Halogen mit einer Atomnummer von 17 bis und mit 53, wie Chlor oder Brom, bedeutet, $R_5$ eine Gruppe der Formel $-NH-C(=O)-R_8$ ist, $R_6$ für Wasserstoff, Niederalkyl mit bis und mit 4 C-Atomen, wie Methyl, Halogen mit einer Atomnummer von 17 bis und mit 53, wie Chlor oder Brom, Trifluormethyl oder Cyano steht, $R_7$ Wasserstoff, Niederalkyl mit bis und mit 4 C-Atomen, wie Methyl, Cyano oder Halogen mit einer Atomnummer von 17 bis und mit 53, wie Chlor oder Brom, darstellt, $R_8$ für Carboxy, Niederalkoxycarbonyl mit bis und mit 5 C-Atomen, wie Methoxy- oder Ethoxycarbonyl, oder 5-Tetrazolyl steht und alk geradkettiges Niederalkylen mit 2 bis und mit 7 C-Atomen, wie 1,3-Propylen, oder Hydroxyniederalkylen mit 3 bis und mit 7 C-Atomen, in dem die Hydroxygruppe in höherer als der α-Stellung und in niedrigerer als der ω-Stellung gebunden ist, wie 1,3-(2-Hydroxy)propylen, bedeutet, und ihre Salze, insbesondere pharmazeutisch verwendbaren Salze, mit Basen.

Die Erfindung betrifft ganz besonders Verbindungen der Formel I, worin $R_1$ Niederalkyl mit bis und mit 4 C-Atomen, wie Methyl, bedeutet, $R_2$ ω,ω,ω-Trifluorniederalkyl mit bis und mit 3 C-Atomen, wie 3,3,3-Trifluorpropyl bedeutet, $R_3$ Wasserstoff bedeutet, $R_5$ eine Gruppe der Formel $-NH-C(=O)-R_8$ darstellt, $R_4$ für Niederalkyl mit bis und mit 4 C-Atomen, wie Methyl, steht, $R_6$ Halogen mit einer Atomnummer von 17 bis und mit 53, wie Chlor oder Brom, oder Cyano darstellt, $R_7$ Wasserstoff ist, $R_8$ für Carboxy, Niederalkoxycarbonyl mit bis und mit 5 C-Atomen, wie Methoxy- oder Ethoxycarbonyl, oder 5-Tetrazolyl steht und alk geradkettiges Niederalkylen mit 2 bis und mit 7 C-Atomen, wie 1,3-Propylen, oder Hydroxyniederalkylen mit 3 bis und mit 7 C-Atomen, in dem die Hydroxygruppe in höhrere als der α-Stellung und in niedrigerer als der ω-Stellung gebunden ist, wie 1,3-(2-Hydroxy)propylen, darstellt, und ihre Salze, insbesondere pharmazeutisch verwendbaren Salze, mit Basen.

Die Erfindung betrifft in allererster Linie Verbindungen der Formel I, worin $R_1$ Niederalkyl mit bis und mit 4 C-Atomen, wie Methyl, bedeutet, $R_2$ ω-Fluor, ω,ω-Difluor- oder ω,ω,ω-Trifluorniederalkyl mit bis und mit 3 C-Atomen, wie 3,3,3-Trifluorpropyl, bedeutet, $R_3$ und $R_7$ für Wasserstoff stehen, einer der Reste $R_4$ und $R_6$ Niederalkyl mit bis und mit 4 C-Atomen, wie Methyl, und der andere Halogen mit einer Atomnummer von 17 bis und mit 53, wie Fluor, Chlor oder Brom, bedeutet, $R_5$ eine Gruppe der Formel $-NH-C(=O)-R_8$ ist, $R_8$ für Carboxy oder 5-Tetrazolyl steht und alk geradkettiges Niederalkylen mit 2 bis und mit 7 C-Atomen, wie 1,3-Propylen, oder Hydroxyniederalkylen mit 3 bis und mit 7 C-Atomen, in dem die Hydroxygruppe in höherer als der α-Stellung und in niedrigerer als der ω-Stellung gebunden ist, wie 1,3-(2-Hydroxy)propylen, darstellt, und ihre Salze, insbesondere pharmazeutisch verwendbaren Salze, mit Basen.

Die Erfindung betrifft namentlich die in den Beispielen genannten Verbindungen der Formel I und ihre Salze, insbesondere pharmazeutisch verwendbaren Salze, mit Basen.

Die Erfindung betrifft ferner ein auf an sich bekannten Methoden beruhendes Verfahren zur Herstellung von Verbindungen der Formel I oder ihrer Salze. Dieses ist dadurch gekennzeichnet, dass man

a) eine Verbindung der Formel

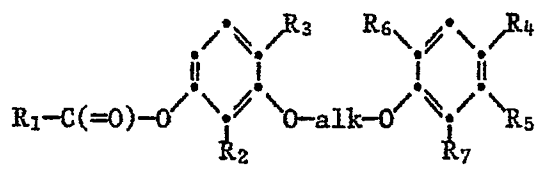

(II)

umlagert oder

b) eine Verbindung der Formel

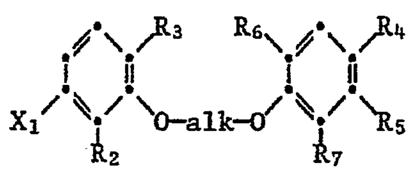

(III),

worin $X_1$ ggf. verethertes Hydroxy bedeutet, mit einer Verbindung der Formel $R_1-X_2$ (IV) umsetzt, worin $X_2$ ggf. funktionell abgewandeltes Carboxy bedeutet, oder

c) in einer Verbindung der Formel

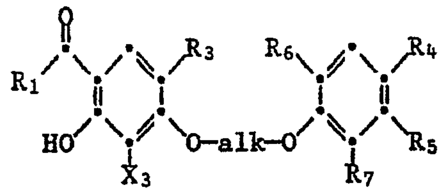

(V),

worin $X_3$ einen in $R_2$ überführbaren Rest bedeutet, oder in einem Salz davon $X_3$ in $R_2$ überführt oder
d) in einer Verbindung der Formel

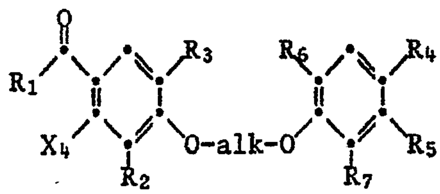

(VI),

worin $X_4$ einen in Hydroxy überführbaren Rest bedeutet, $X_4$ in Hydroxy überführt oder
e) Verbindungen der Formeln

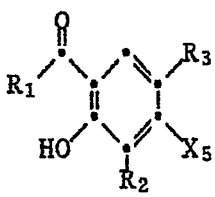

(VII) und

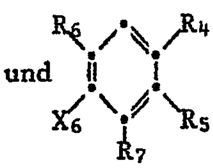

(VIII)

miteinander umsetzt, worin einer der Reste $X_5$ und $X_6$ gegebenenfalls in Salzform vorliegendes Hydroxy und der andere einen durch reaktionsfähiges verestertes Hydroxy oder Epoxy substituiertes Rest –O–alkH, d.h. einen durch reaktionsfähiges verestertes Hydroxy substituiertes Alkoxy- oder Mono-, Di- oder Trioxaalkoxyrest oder einen durch Epoxy substituerten Alkoxy- oder Mono- oder Dioxaalkoxyrest, darstellt, oder
f) eine Verbindung der Formel

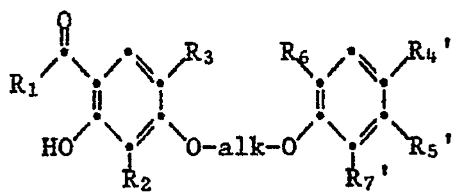

(IX) ,

worin einer der Reste $R_4'$, $R_5'$ und $R_7'$ die Aminogruppe, ein davon verschiedener Rest $R_4'$ bzw $R_5'$ einen Rest $R_9$ und ein davon verschiedener Rest $R_7'$ einen Rest $R_{10}$ darstellt, oder ein Salz davon mit einer Verbindung der Formel

$X_7 - R_8'$ (X)

umsetzt, worin $R_8'$ gegebenenfalls veresterte oder amidierte Carboxygruppe oder gegebenenfalls in 1-Stellung geschütztes 5-Tetrazolyl und $X_7$ eine gegebenenfalls veresterte, amidierte, anhydridisierte oder, sofern $R_8'$ für in 1-Stellung geschütztes 5-Tetrazolyl steht, in Salzform vorliegende Carboxygruppe bedeutet, und gegebenenfalls die Schutzgruppe in 1-Stellung einer 5-Tetrazolylgruppe $R_8$ abspaltet oder
g) in einer Verbindung der Formel

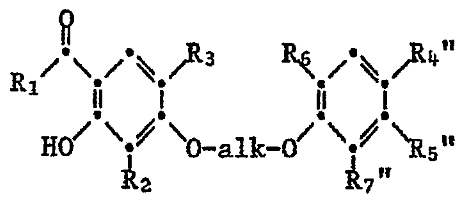 (XI),

worin einer der Reste $R_4$", $R_5$" und $R_7$" einen Rest $X_8$, ein davon verschiedener Rest $R_4$" bzw. $R_5$" einen Rest $R_9$ und ein davon verschiedener Rest $R_7$" einen Rest $R_{10}$ darstellt und $X_8$ einen in die Gruppe der Formel $-NH-C(=O)-R_8$ überführbaren Rest bedeutet, $X_8$ in diese Gruppe überführt oder
h) in einer Verbindung der Formel

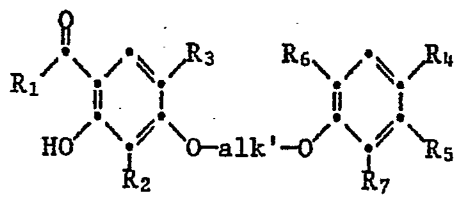 (I'),

worin alk' einen in die Gruppe alk überführbaren Rest bedeutet, alk' in alk überführt und gewünschtenfalls jeweils eine verfahrensgemäss erhältliche Verbindung der Formel I in eine andere Verbindung der Formel I überführt, ein verfahrensgemäss erhältliches Isomerengemisch auftrennt und das oder die gewünschte(n) Isomere(n) der Formel I isoliert und/oder eine verfahrensgemäss erhältliche freie Verbindung der Formel I in ein Salz oder ein verfahrensgemäss erhältliches Salz in die freie Verbindung der Formel I oder in ein anderes Salz umwandelt.

Ggf. verethertes Hydroxy $X_1$ in Formel III bedeutet beispielsweise Niederalkoxy, wie Methoxy.

Ggf. funktionell abgewandeltes Carboxy $X_2$ in Formel IV bedeutet beispielsweise freies, verestertes oder anhydridisiertes Carboxy, wie Carboxy, Niederalkoxycarbonyl, z.B. Methoxy- oder Ethoxycarbonyl, Halogencarbonyl, z.B. Chlorcarbonyl, oder anhydridisiertes Carboxy der Formel $-C(=O)-O-C(=O)-R_1$ (IVa).

In fluorierte Niederalkylreste $R_2$ überführbare Reste $X_3$ in Formel V sind beispielsweise fluorierte Niederalkenylrest, wie 3-Fluor-, 3,3-Difluor- oder 3,3,3-Trifluor-prop-1-enyl bzw. 3-Fluor- oder 3,3,-Difluor-prop-2-enyl, fluorierte Niederalkinylreste, wie 3-Fluor, 3,3-Difluor- oder 3,3,3-Trifluor-prop-1-inyl, ferner fluorierte Hydroxyniederalkylreste, wie 3-Fluor-, 3,3-Difluor- oder 3,3,3-Trifluor-1-hydroxy-propyl, ferner Halogen der Atomnummer 19 bis und mit 53, wie Brom oder Jod, ferner Chlor.

In Hydroxy überführbare Reste $X_4$ in Formel VI sind beispielsweise veretherte oder veresterte Hydroxygruppen. Als verethertes Hydroxy $X_4$ kommen beispielsweise aliphatisch verethertes Hydroxy, z.B. Niederalkoxy, wie Methoxy, oder Niederalkenyloxy, wie Allyloxy, Phenylniederalkoxy, insbesondere gegebenenfalls substituiertes Phenylniederalkoxy, wie Benzyloxy, ferner Teterahydropyran-2-yloxy oder Silyloxy, insbesondere Triniederalkylsilyloxy, z.B. Trimethylsilyloxy, in Betracht. Verestertes Hydroxy $X_4$ ist beispielsweise mit einer Carbonsäure, wie einer aliphatischen oder aromatischen Carbonsäure oder mit einem aliphatischen oder aromatischen Halbester der Kohlensäure verestertes Hydroxy, wie Niederalkanoyloxy, z.B. Acetoxy, gegebenenfalls substituiertes Benzoyloxy, z.B. der Formel $R_1-C(=O)-O-$, gegebenenfalls halogeniertes Niederalkoxycarbonyloxy, z.B. Methoxy-, Ethoxy- oder Tertiärbutyloxycarbonyloxy, 2,2,2-Trijodethoxy- oder 2,2,2-Trichlorethoxycarbonyloxy, gegebenenfalls substituiertes Phenylniederalkoxycarbonyloxy, insbesondere 1-Phenylniederalkoxycarbonyloxy, z.B. Benzyloxycarbonyloxy, oder gegebenenfalls substituiertes Phenoxycarbonyloxy.

In Salzform vorliegende Hydroxy $X_5$ in Formel VII bzw. $X_6$ in Formel VIII bzw. Carboxy $X_7$ in Formel X liegt insbesondere in einer Alkalimetallsalzform, z.B. als Natrium- oder Kaliumsalz, vor.

Durch reaktionfähiges verestertes Hydroxy oder Epoxy substituierte Alkoxyreste $X_5$ in Formel VII bzw. $X_6$ in Formel VIII sind beispielsweise Reste der Formeln

$$-O-(CH_2)_m-X,\quad -O-[(CH_2)_n-O]_k-(CH_2)_{n'}-X,$$

$$-O-[(CH_2)_l-O]_p-CH_2CH(OH)CH_2-[O-(CH_2)_{l'}]_o-X \text{ bzw.}$$

$$-O-[(CH_2)_l-O]_p-CH_2-X',$$

worin X reaktionsfähiges verestertes Hydroxy und X' 1,2-Epoxyethyl ist. Reaktionsfähiges verestertes Hydroxy ist dabei beispielsweise Halogen, wie Chlor oder Jod, oder organisches Sulfonyloxy, wie Niederalkansulfonyloxy, z.B. Methansulfonyloxy, oder gegebenenfalls substituiertes Benzolsulfonyloxy, z.B. Benzol-, p-Brombenzol- oder p-Toluolsulfonyloxy.

Gegebenenfalls verestertes, amidiertes oder anhydridisiertes Carboxy $X_7$ in Formel X ist beispielsweise freies Carboxy, verestertes Carboxy $R_8$ oder mit einem gegebenenfalls substituierten Phenol verestertes Carboxy, wie Phenoxy-, 4-Nitrophenoxy- oder 2,4-Dinitrophenoxycarbonyl, amidiertes Carboxy $R_8$ oder aktiviertes Carbamyl, wie 1-Imidazolyl- bzw. 1-(2,5-Dimethylimidazolyl)-carbonyl, oder mit einer Halogenwasserstoffsäure anhydridisiertes Carboxy, wie Halogencarbonyl, z.B. der Formel Hal–C(=O)–, worin Hal Chlor, Brom oder Jod, vor allem Chlor ist.

Als Ausgangsstoffe X kommen insbesondere solche der Formeln $R_8$"–$R_8$" (Xa), worin $R_8$" gegebenenfalls verestertes Carboxy bedeutet, oder Hal–C(=O)–$R_8$' (Xb) in Betracht. In geschützter Form vorliegende 5-Tetrazolylreste $R_8$' sind beispielsweise gegebenenfalls im Arylteil substituierte 1-(α-Aralkyl)-tetrazolyl-(5)-reste, wie Benzyltetrazolyl-(5) oder 1-(p-Methoxybenzyl)-tetrazolyl-(5).

Ein in die Gruppe der Formel –NH–C(=O)–$R_8$ überführbarer Rest $X_8$ in Formel XI beispielsweise ein durch Solvolyse, d.h. Hydrolyse, Alkoholyse (Umsetzung mit dem der gewünschten veresterten Carboxygruppe $R_8$ entsprechenden Alkohol) und/oder Aminolyse (Umsetzung mit Ammoniak oder einem der gewünschten amidierten Carboxygruppe $R_8$ entsprechenden Amin) in diese überführbarer Rest, beispielsweise eine Gruppe der Formel –NH–$X_A$, in der $X_A$ eine von einer gegebenenfalls veresterten oder amidierten Oxalogruppe verschiedene und in diese überführbare funktionell abgewandelte Oxalogruppe bedeutet. Derartige funktionell abgewandelte Oxalogruppen sind vorzugsweise solche, die als funktionell abgewandelte a-Carbonylgruppierung Thioxomethylen, Iminomethylen oder eine veresterte und/oder veretherte Dihydroxymethylengruppierung und/oder als funktionell abgewandelte Carboxygruppe eine von einer veresterten oder amidierten Carboxygruppe verschiedene funktionell abgewandelte Carboxygruppe aufweisen. Veresterte und/oder veretherte Dihydroxymethylengruppierungen sind beispielsweise mit einer Halogenwasserstoffsäure, wie Chlorwasserstoffsäure, veresterte und/oder mit einem Niederalkanol, wie Methanol oder Ethanol, veretherte Dihydroxymethylengruppen. Als Beispiele seinen vor allem Dihalogenmethylengruppierungen, wie Dichlormethylen, Niederalkoxyhalogenmethylengruppierungen, wie Methoxy- oder Ethoxychlormethylen, oder Diniederalkoxymethylengruppierungen, wie Dimethoxy- oder Diethoxymethylen, genannt. Von veresterten oder amidierten Carboxylgruppen verschiedene funktionell abgewandelte Carboxygruppierungen sind beispielsweise die Cyanogruppe, anhydridisierte Carboxygruppen, wie Halogen-, z.B. Chlorcarbonyl, Iminoester-, wie Imid- bzw. Aminhalogenidgruppierungen, z.B. Iminochlor- oder Aminodichlormethyl, Iminoethergruppierungen, wie Niederalkyl- oder Niederalkyleniminoethergruppierungen, z.B. Methoxy- oder Ethoxyiminomethylen, 4,4- oder 5,5-dimethyloxazolinyl(2) oder 4,4,6-Trimethyl-dihydro-oxazinyl-(2), Amidinogruppen, wie Amidino oder Niederalkyl-, z.B. Methylamidino, mit einer Halogenwasserstoffsäure, wie Chlorwasserstoffsäure, veresterte und/oder mit einem Niederalkanol veretherte Orthosäuregruppierungen, wie Triniederalkoxy-, Niederalkoxyhalogen- oder Trihalogenmethylgruppen, vor allem Trimethoxy- oder Triethoxymethyl, Ethoxydichlormethyl oder Trichlormethyl, oder gegebenenfalls veresterte Thiocarboxylgruppen, wie Niederalkylthiocarbonylgruppen, z.B. Ethylthiocarbonyl.

Ein in die Gruppe der Formel –NH–C(=O)–$R_8$, worin $R_8$ 5-Tetrazolyl bedeutet, überführbarer Rest $X_8$ ist beispielsweise die Gruppe der Formel –NH–C(=O)–CN oder einer Gruppe der Formel –NH–C(=O)–$R_8$', worin $R_8$' in 1-Stellung geschütztes 5-Tetrazolyl ist. In geschützter Form vorliegende 5-Tetrazolylreste $R_8$' sind beispielsweise gegebenenfalls im Arylteil substituierte 1-(α-Aralkyl)-tetrazolyl-(5)-reste, wie 1-Benzyltetrazolyl-(5) oder 1-(p-Methoxybenzyl)-tetrazolyl-(5).

Weitere in Gruppen der Formel –NH–C(=O)–$R_8$ überführbare Reste $X_8$ sind beispielsweise oxydativ in diese überführbare Gruppen der Formel –NH–$X_B$, worin $X_B$ die oxydativ in die Oxalgruppe der Formel –C(=O)–$R_8$, worin $R_8$ Carboxy darstellt, überführbare gegebenenfalls hydratisierte Glyoxylgruppe bedeutet. Diese kann vorteilhaft im Verlaufe der Oxydationsreaktion, z.B. aus der Acylgruppe einer gegebenenfalls α,β-ungesättigten oder α,β-dihydroxylierten aliphatischen oder araliphatischen Carbonsäure, einer gegebenenfalls an der Hydroxygruppe veresterten Glykoloylgruppe oder der Glycylgruppe, in situ gebildet oder aus einer ihrer funktionellen Derivate, z.B. eine ihrer Acetale oder Imine, in Freiheit gesetzt werden. Acylgruppen von gegebenenfalls α,β-ungesättigten oder α,β-dihydroxylierten Carbonsäuren sind beispielsweise Alkanoylgruppen, wie Niederalkanoyl, z.B. Acetyl, Acylgruppen von α,β-ungesättigten aliphatischen Mono- oder Dicarbonsäuren, z.b. Acryloyl, Crotonyl oder die Acylgruppe der gegebenenfalls funktionell abgewandelten Fumar- oder Maleinsäure, Acylgruppen von α,β-ungesättigten aliphatischen Carbonsäuren, z.B. gegebenenfalls substituiertes Cinnamoyl, oder Acylgruppen von aliphatischen, α,β-Dihydroxydicarbonsäuren, wie der Weinsäure, oder monofunktioneller Carboxyderivate, wie Estern oder Amiden, derselben. Veresterte Glykoloylgruppen sind beispielsweise an der Hydroxygruppe mit einer Mineralsäure, wie einer Halogenwasserstoffsäure, z.B. mit Chlor- oder Bromwasserstoffsäure, oder mit einer Carbonsäure, z.B. mit Essigsäure oder der gegebenenfalls substituierten Benzoesäure, veresterte Glykoloylgruppen. Acetalisierte Glyoxyloylgruppen sind beispielsweise mit Niederalkanolen oder einem Niederalkandiol acetalisierte Glyoxyloylgruppen, wie Dimethoxy-, Diethoxy- oder Ethylendioxyacetyl. Imine von Glyoxyloylgruppen sind beispielsweise gegebenenfalls substituierte N-Benzylimine oder N-(2-Benzothiazolyl)-imine derselben oder Imine mit 3,4-Di-tert.-butyl-o-chinon. Weitere oxydativ in die Oxalogruppe überführbare Reste sind z.B. gegebenenfalls substituierte, wie in 5-Stellung eine acetalisierte Formylgruppe, wie Diethoxymethyl, aufweisende 2-Furoylgruppen.

Zu veresterten Oxalogruppen der Formel $-C(=O)-R_8$, worin $R_8$ für verestertes Carboxy steht, oxydierbare Gruppen sind veretherte Glykoloylgruppen, wie Niederalkoxyacetyl. Zu gegebenenfalls veresterten oder amidierten Oxaloaminogruppen oxydierbare Reste $X_B$ sind ferner gegebenenfalls hydratisierte oder acetalisierte Formylmethylaminogruppen oder gegebenenfalls funktionell abgewandelte Carboxymehtylaminogruppen bzw. Carboxymethyleniminogruppen, z.B. der Formel
$-NH-CH_2-CH=O$, $-NH-CH_2-R_8$ bzw. $-N=CH-R_8$.

In Reste alk überführbare Gruppen sind beispielsweise durch Oxo oder verethertes oder verestertes Hydroxy substituierte Reste alk, wie Oxoalkylen-, Oxo(oxa)alkylen- oder Oxo(dioxa)alkylenreste, z.B. der Formel

$$-[(CH_2)_{l'}O]_{o}CH_2C(=O)CH_2-[O-(CH_2)_{l}]_{p}- \quad (Ib'),$$

bzw. veretherte oder veresterte Hydroxyalkylen-, Hydroxy(oxa)alkylen- oder Hydroxy(dioxa)alkylenreste der Formel

$$-[(CH_2)_{l'}O]_{o}CH_2CH(X'')CH_2-[O-(CH_2)_{l}]_{p}- \quad (Ib''),$$

worin X" verethertes oder verestertes Hydroxy bedeutet.

Verethertes Hydroxy X" ist dabei beispielsweise mit einem α-Aralkanol oder einem Silanol verethertes Hydroxy, wie gegebenenfalls substituiertes Benzyloxy oder Triniederalkylsilyloxy, z.B. Trimethylsilyloxy.

Verestertes Hydroxy X" ist beispielweise mit einer Carbonsäure, wie einer Niederalkansäure oder einem Halbester der Kohlensäure verestertes Hydroxy, wie Niederalkanoyloxy, z.B. Acetoxy oder Pivaloyloxy, Niederalkoxycarbonyloxy, z.B. Tertiärbutoxycarbonyloxy, oder gegebenenfalls substituiertes Benzyloxycarbonyloxy, z.B. Benzyloxycarbonyloxy.

Die Durchführung der verfahrensgemässen Reaktionen sowie die Herstellung neuer Ausgangsstoffe bzw. Zwischenprodukte erfolgt in Analogie zur Reaktions- und Bildungsweise bekannter Ausgangsstoffe bzw. Zwischenprodukte. Dabei werden, auch wenn nachstehend nicht ausdrücklich erwähnt, die jeweils üblichen Hilfsmittel, wie Katalysatoren, Kondensations- sowie Solvolysemittel und/oder Lösungs- bzw. Verdünnungsmittel, und Reaktions-, wie Temperatur- und Druckbedingungen, sowie gegebenenfalls Schutzgase verwendet.

Die Umlagerung von Verbindungen II gemäss Verfahrensvariante a) erfolgt beispielsweise photochemisch oder in Gegenwart eines sauren Kondensationsmittels. Geeignete saure Kondensationsmittel sind beispielsweise Lewissäuren, insbesondere komplexe Metallhalogenide der Formel $M^nY_n$ (XIX), worin M ein n-wertiges, koordinativ ungesättigtes Metallatom oder Gruppe IIb, IIIa, IIIb, IVa, IVb, Va oder VIIIb des Periodischen Systems der Elemente, z.B. ein $Zink^{II}$-, $Bor^{III}$-, $Aluminium^{III}$-, $Gallium^{III}$-, $Zinn^{IV}$-, $Titan^{IV}$-, $Antimon^{V}$- oder $Eisen^{III}$-Atom und Y ein Halogenatom, insbesondere der Atomnummer bis und mit 35, wie Fluor, Chlor oder Brom darstellt. Vorzugsweise verwendet man Bortrifluorid, Aluminiumtrichlorid, Galliumchlorid, Zinntetrachlorid oder insbesondere Zinkchlorid. Weitere geeignete saure Kondensationsmittel sind komplexe Sauerstoffsäuren, vor allem des Schwefels oder Phosphors, wie Schwefelsäure, Pyroschwefelsäure, Phosphorsäure, Pyrophosphorsäure oder Polyphosphorsäure. Geeignete inerte Lösungsmittel sind beispielsweise Tetrachlormethan, Tetrachlorethan, Trichlorethylen, Schwefelkohlenstoff oder Nitrobenzol. Erforderlichenfalls arbeitet man unter Kühlen oder Erwärmern, z.B. bei etwa –10 bis etwa 40°C, insbesondere bei +5 bis +30°C.

Ausgangsstoffe II können z.B hergestellt werden, indem man Verbindungen der Formeln

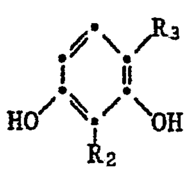 (XII) und 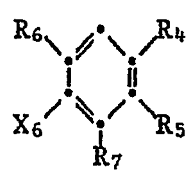 (VIII),

worin $X_6$ einen durch reaktionfähiges verestertes Hydroxy oder Epoxy substituierten Alkoxyrest, z.B. der Formeln

$$-O-(CH_2)_m-X,\quad -O-[(CH_2)_n-O]_k-(CH_2)_{n'}-X,$$

$$-O-[(CH_2)_l-O]_p-CH_2CH(OH)CH_2-[O-(CH_2)_{l'}]_o-X \text{ bzw.}$$

$$-O-[(CH_2)_l-O]_p-CH_2-X',$$

worin X reaktionsfähiges verestertes Hydroxy, z.B. Halogen, und X' 1,2-Epoxyehtyl bedeutet, darstellt, in üblicher Weise miteinander umsetzt und das Reaktionsprodukt der Formel

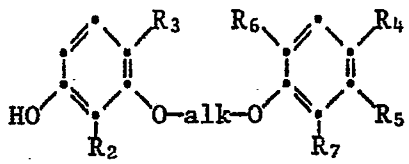

(XIII)

in üblicher Weise, z.B. durch Umsetzung mit einer Verbindung $R_1$–$X_1$ (IV), worin $X_1$, z.B. Halogencarbonyl oder anhydridisiertes Carboxy der Formel –C(=O)–O–C(=O)–$R_1$ bedeutet, O-acyliert.

Verbindungen XII werden beispielsweise erhalten, indem man Verbindungen der Formeln

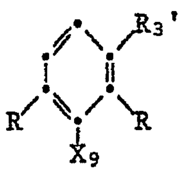

(XIV) und $X_{10}$–$R_2$ (XV),

worin die Reste R gleiche oder verschiedene veretherte Hydroxygruppen bedeuten, $R_3$' Wasserstoff, Niederalkoxy, Trifluormethyl, Fluor oder Nitro bedeutet und eine Gruppe $X_9$ und $X_{10}$ einen metallischen Rest, wie ein Alkalimetallatom, z.B. Natrium oder Lithium, ferner Kupfer oder eine Halogenerdalkalimetallgruppe, z.B. der Formel –Mg–Hal bedeutet, und der andere für eine Gruppe –Hal steht, die Halogen, z.B. Brom oder Jod, darstellt, miteinander umsetzt, beispielsweise in einem Diniederalkyl- oder Niederalkylenether, wie Diethylether, Tertiärbutoxymethan, Dioxan oder Tetrahydrofuran, Nitro $R_3$' zu Amino reduziert und durch Behandeln mit Natriumnitrit und einer Halogenwasserstoffsäure in Halogen $R_3$ überführt und die Gruppen R, z.B. durch Behandeln mit Bromwasserstoff in Dichlormethan, zu Hydroxy spaltet.

In einer Abwandlung dieses Verfahrens kann man die metallorganische Komponente in situ bilden, indem man von der entsprechenden Halogenverbindung ausgeht und die Umsetzung, vorzugsweise unter Erwärmen in Gegenwart des entsprechenden Metalles in feinverteilter Form, z.B. von Kupferpulver, durchführt. In einer anderen Abwandlung dieses Verfahrens gelangt man zu Verbindungen XII, indem man eine Verbindung XIV, worin $X_9$ einen der genannten metallischen Reste, vorzugsweise ein Alkalimetallatom oder eine Gruppe –Mg–Hal ist, mit einem fluorierten Niederalkanal bzw. Niederalkanon umsetzt, in der gebildeten Verbindung der Formel

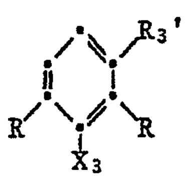

(XVI)

worin $X_3$ fluoriertes Hydroxyniederalkyl ist, die Hydroxyniederalkylgruppe ggf. nach Wasserabspaltung, z.B. durch Acetylierung und anschliessende Behandlung mit Zink und Ammonchlorid, zur entsprechenden fluorierten Niederalkenylgruppe, zu dem gewünschten fluorierten Rest $R_2$ hydriert, Nitro in Halogen umwandelt und R zu Hydroxy spaltet.

In einer weiteren Abwandlung dieses Verfahrens kann man auch eine Verbindung XIV, worin $X_9$ Brom oder Jod ist, in Gegenwart von Kupferpulver mit Trifluorjodmethan zu der entsprechenden Verbindung der Formel

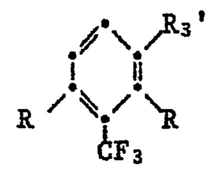

(XVII)

umsetzen und nachfolgend ggf. Nitro in Halogen und die Reste R in Hydroxy umwandeln.

Verbindungen XII, worin $R_2$ 3-Fluor- oder 3,3-Difluorpropyl ist, werden besonders elegant erhalten, indem man eine Verbindung der Formel

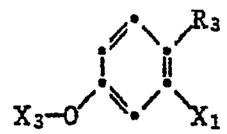

(XVIII)

worin $X_1$ ggf. verethertes Hydroxy und $X_3$ einen 1-Fluor- oder 1,1-Difluorprop-2-enylrest bedeutet, die ihrerseits durch Umsetzung des entsprechenden $R_3$-Resorcins bzw. $R_3$-Resorcinmonoethers mit einem 1-Fluor- oder 1,1-Difluorprop-2-enyl-1-bromid erhältlich ist, der Allylumlagerung unterwirft, beispielsweise durch Erwärmen auf etwa 150 bis 250°C, vorzugsweise auf etwa 190 bis 220°C, vorteilhaft in einem Lösungsmittel, wie Diphenylether oder N,N-Dimethyl- oder N,N-Diethylanilin und in dem Reaktionsprodukt der Formel

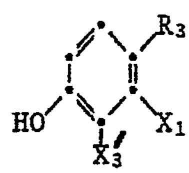

(XIX),

die gebildete 3-Fluor- bzw. 3,3-Difluorprop-2-enylgruppe $X_3'$ zu 3-Fluor- bzw. 3,3-Difluor-propyl hydriert und erforderlichenfalls verethertes Hydroxy $X_1$ zu Hydroxy spaltet.

Verbindungen XII, worin $R_2$ 3,3,3-Trifluorpropyl bedeutet, werden gemäss einer neuartigen Verfahrensweise besonders elegant erhalten, indem man einen Aldehyd der Formel

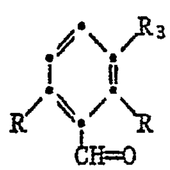

(XX),

worin z.B. Methoxy ist, z.B. in Gegenwart von Dimethylformamid mit der Verbindung der Formel $CF_3CCl_2ZnCl(C_2H_5)_2O$ (XXI) zu der entsprechenden Verbindung der Formel

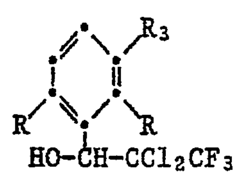

(XXII)

umsetzt, diese, z.B. durch Umsetzung mit Acetanhydrid in Gegenwart von Pyridin, in der Seitenkette acyliert, das Reaktionsprodukt der Formel

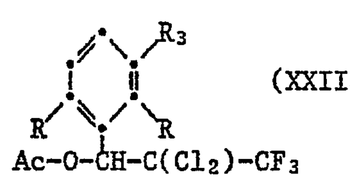

(XXIIa),

worin Ac für Acyl, z.B. Acetyl, steht,

durch Behandlung mit einem metallischen Reduktionsmittel, z.B. mit aktiviertem Zinkstaub in Gegenwart von Ammoniumchlorid, in die entsprechende Verbindung der Formel

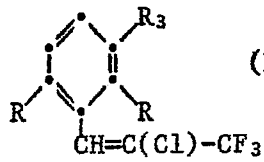

(XXIII)

übeführt, aus dieser, z.B. durch Behandeln mit Kaliumtertiärbutanolat, Chlorwasserstoff abspaltet, in der erhaltenen Verbindung der Formel

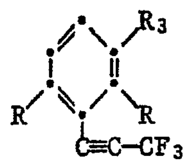

(XXIV)

die Seitenkette, z.B. in Gegenwart von Palladiumkohle, hydriert und in der erhaltenen Verbindung der Formel

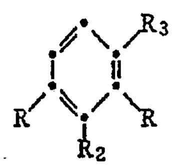

(XXV),

z.B. durch Behandeln mit Bromwasserstoffsäure in Dichlormethan, die Hydroxygruppen freisetzt.

Die Herstellung von Verbindungen VIII ist unter der Verfahrensvariante e) beschrieben.

Die Umsetzung von Verbindungen III und IV, als welche insbesondere solche geeignet sind, in denen $X_2$ Carboxy ist, gemäss Verfahrensvariante b) erfolgt üblicherweise in Gegenwart eines sauren Kondensationsmittels, vorteilhaft in einem inerten Lösungsmittel, erforderlichenfalls unter Kühlen oder Erwärmen, beispielsweise bei etwa +80 bis +140°C, vor allem bei etwa 80 bis etwa 120°C. Als saure Kondensationsmittel kommen beispielsweise die für Verfahrensvariante a) angegebenen in Betracht.

Ausgangsstoffe III können beispielsweise hergestellt werden, indem man Verbindungen der Formeln

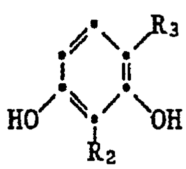

(XII) und

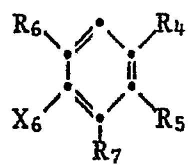

(VIII)

miteinander umsetzt, wobei die Verbindung XII auch in Monoether- und/oder in Salzform vorliegen kann und $X_6$ einen durch reaktionsfähiges verestertes Hydroxy substituierten Rest –O–alkH, d.h. einen reaktionsfähigen veresterten Hydroxyalkoxyrest oder einen Epoxyalkoxyrest, z.B. eine Gruppe der Formeln

$-O-(CH_2)_m-X$, $-O-[(CH_2)_n-O]_k-(CH_2)_{n'}-X$,

$-O-[(CH_2)_l-O]_p-CH_2CH(OH)CH_2-[O-(CH_2)_{l'}]_o-X$ bzw.

$-O-[(CH_2)_l-O]_p-CH_2-X'$,

worin X reaktionsfähiges verestertes Hydroxy, wie Halogen, und X' 1,2-Epoxyethyl bedeutet, darstellt. Die Umsetzung erfolgt z.B. in analoger Weise wie in der Verfahrensvariante e) angegeben.

Die Überführung der genannten Fluor aufweisenden Reste $X_3$ in Gruppen $R_2$ gemäss Verfahrensvariante c) erfolgt beispielsweise durch Reduktion. Als Reduktionsmittel kommt beispielsweise Wasserstoff in Gegenwart eines Hydrierungskatalysators, wie eines Platin-, Palladium- oder Rhodiumkatalysators, z.B. von Platinoxid, in Betracht. Die Behandlung mit katalytisch aktiviertem Wasserstoff (Hydrierung) erfolgt unter normalen oder höchstens geringfügig erhöhten Druck- und Temperaturbedingungen, z.B. unter etwa 0 bis 5 bar Überdruck und/oder im Temperaturbereich von etwa 20° bis etwa 80°C. Die Überführung von Halogen in z.B. Trifluormethyl erfolgt beispielsweise durch Erhitzen mit Trifluorjodmethan und Kupferpulver.

Ausgangsstoffe V, worin $X_3$ einen mono- oder difluorierten Niederalkenylrest bedeutet, werden beispielsweise erhalten, indem man eine Verbindung der Formel

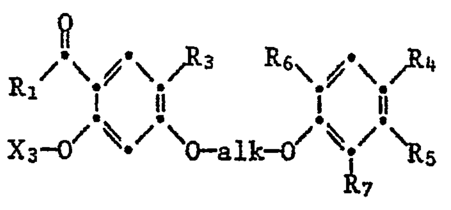
(XXVI)

der Allylumlagerung unterwirft, beispielsweise durch Erwärmen auf etwa 150 bis 250°C, vorzugsweise auf etwa 190° bis 220°C, vorteilhaft in einem Lösungsmittel, wie Diphenylether oder N,N-Dimethyl- oder N,N-Diethylanilin.

Verbindungen XXIV werden ihrerseits erhalten, indem man eine entsprechende Verbindung der Formel

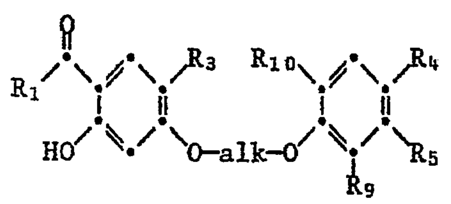
(XXVII)

mit einer in Allylstellung zur Doppelbindung durch Chlor, Brom oder Jod substituierten fluorierten Niederalken der Formel $X_3$–H (XXVIII) umsetzt, z.B. in Gegenwart eines basischen Kondensationsmittels, wie Kaliumcarbonat.

Die Überführung von Gruppen $X_4$ in Hydroxy gemäss der Verfahrensvariante d) erfolgt in üblicher Weise, beispielsweise durch Behandeln mit einem komplexen Metallhalogenid der Formel $M^nY_n$ (XIX), worin M ein n-wertiges, koordinativ ungesättigtes Metalkation oder Gruppe IIa, IIb, IIIa, IIIb, IVa, IVb, Va oder VIIIb des periodischen Systems der Elemente, z.B. Magnesium-, Zink$^{II}$-, Bor$^{III}$-, Aluminium$^{III}$-, Gallium$^{III}$-, Zinn$^{IV}$-, Titan$^{IV}$-, Antimon$^{V}$- oder Eisen$^{III}$ bzw. Eisen VI-ion, Y ein Halogenatom der Atomnummer bis und mit 35, wie Fluor oder Chlor bedeutet, z.B. Aluminiumtrichlorid, oder mit einem tertiären organischen Ammoniumsalz, wie einem Pyridinium- oder Triniederalkylammoniumhalogenid, z.B. mit Pyridiniumchlorid oder -bromid oder Triethylammoniumchlorid, kann aber auch durch Solvolyse, insbesondere durch Hydrolyse, erforderlichenfalls in Gegenwart eines, vorzugsweise sauren, Hydrolysemittels, erfolgen. Hydrolysemittel sind neben üblichen basischen Hydrolysemitteln, wie Alkalimetallhydroxiden als saure Hydrolysemittel beispielsweise Mineralsäuren, z.B. Chlor-, Brom- oder Jodwasserstoffsäure, Schwefelsäure, Phosphorsäure oder Polyphosphorsäure, ebenso komplexe Metallsäuren, z.B. Hexachlorantimonsäure, Tetrafluorborsäure und dergleichen, im Falle von mit organischen Carbonsäuren veresterten Hydroxygruppen $X_4$ ferner Niederalkancarbonsäuren, wie Essigsäure. Lösungsmittel sind bei der Hydrolyse beispielsweise mit Wasser mischbare organische Lösungsmittel. Vorzugsweise arbeitet man jeweils in Gegenwart eines Lösungs- oder Verdünnungsmittels bzw. eines Lösungsvermittlers, unter Kühlen oder Erwärmen, z.B. im Temperaturbereich von etwa 0° bis 120°C, und/oder unter Inertgas.

In als Gruppe $X_4$ eine gegebenenfalls substituierte $\alpha$-Phenylniederalkoxygruppe oder eine andere übliche durch Reduktion spaltbare geschützte Hydroxygruppe aufweisenden Verbindungen VI kann die Hydroxygruppe vorteilhaft reduktiv freigesetzt werden. So kann man beispielsweise hydrieren, d.h. mit Wasserstoff in Gegenwart eines Hydrierungskatalysators, z.B. eines Palladium-, Platin-, Nickel- oder Rhodiumkatalysators, z.B. von Palladium auf Kohle oder von Ransey-Nickel, reduzieren.

Ferner kann man ausgehend von Verbindungen VI, worin $X_4$ mit einer organischen Carbonsäure verestertes Hydroxy ist, die Hydroxygruppe durch Umesterung, d.h. durch Behandlung mit einem Alkohol, z.B. Niederalkanol, in Gegenwart eines sauren oder basischen Mittels, wie einer Mineralsäure, z.B. von Schwefelsäure oder eines Alkalimetallhydroxides oder -alkoholates, z.B. von Natriumhydroxid oder eines Natriumniederalkanolates, freisetzen.

Ausgangsstoffe VI werden beispielsweise hergestellt, indem man Verbindungen der Formeln

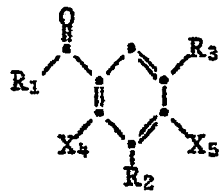 (XXIX) und 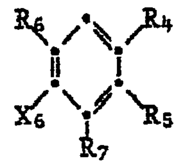 (VIII)

miteinander umsetzt, worin einer der Reste $X_5$ und $X_6$ gegebenenfalls in Salzform voliegendes Hydroxy und der andere einen durch reaktionsfähiges verestertes Hydroxy oder Epoxy substituierten Rest –O–alkH, z.B. eine Gruppe der Formeln

$$-O-(CH_2)_m-X,\quad -O-[(CH_2)_n-O]_k-(CH_2)_{n'}-X,$$

$$-O-[(CH_2)_l-O]_p-CH_2CH(OH)CH_2-[O-(CH_2)_{l'}]_o-X \text{ bzw.}$$

$$-O-[(CH_2)_l-O]_p-CH_2-X',$$

in der X reaktionsfähiges verestertes Hydroxy, z.B. Halogen, und X' 1,2-Epoxyethyl bedeutet, darstellt.

Die Umsetzung von Verbindungen VII und VIII gemäss Verfahrensvariante e) erfolgt in üblicher Weise, beispielsweise in Gegenwart eines basischen Kondensationsmittels, wie eines Hydroxides oder Carbonates eines Alkali- oder Erdalkalimetalles, wie von Natrium- oder Kaliumhydroxid, Kaliumcarbonat oder Calciumcarbonat, vorteilhaft in einem Niederalkanol, z.B. Methanol oder Amylalkohol, Diniederalkylketon, z.B. in Aceton oder Diethylketon, oder N,N-Diniederalkylniederalkansäureamid oder N-Niederalkylniederalkansäurelactam, z.B. in Dimethylformamid oder N-Methylpyrrolidinon.

Die Ausgangsstoffe VII werden beispielsweise hergestellt, indem man eine Verbindung der Formel

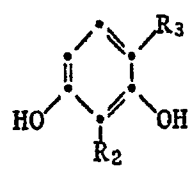 (XII)

in Gegenwart einer Lewissäure, z.B. von Zinkchlorid, mit einer Verbindung der Formel $R_1$–$X_2$ [IV; $X_2$ = Carboxy] umsetzt und gewünschtenfalls in der erhaltenen Verbindung VII, worin $X_5$ Hydroxy ist, die Hydroxygruppe in p-Stellung zu $R_1$–C(=O)– durch Umsetzung mit einem Dihalogenalkan, Epoxyalkan oder Halogenalkanol in einen durch Halogen oder Hydroxy substituierten Alkoxyrest überführt und gegebenenfalls Hydroxyalkoxy reaktionsfähig verestert, z.B. durch Behandeln mit Thionylchlorid Phosphortribromid oder einem Sulfonsäurechlorid.

Verbindungen VIII können beispielsweise erhalten werden, indem man in einer Verbindung der Formel

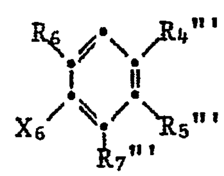 (XXX) ,

worin einer der Reste $R_4'''$, $R_5'''$ und $R_7'''$ die Nitrogruppe, ein davon verschiedener Rest $R_4'''$ oder $R_5'''$ einen Rest $R_9$ und ein davon verschiedener Rest $R_7'''$ einen Rest $R_{10}$ darstellt, die Nitrogruppe zu Amino reduziert, z.B. mit Wasserstoff in Gegenwart von Raney-Nickel, und die Verbindung der Formel

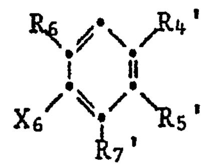 (XXXI) ,

worin einer der Reste $R_4'$, $R_5'$ und $R_7'$ die Aminogruppe, ein davon verschiedener Rest $R_4'$ oder $R_5'$ eine Gruppe $R_9$ und ein davon verschiedener Rest $R_7'$ einen Rest $R_{10}$ bedeutet, in Gegenwart einer Base,

z.B. von Triethylamin oder Pyridin, mit einer Verbindung der Formel Hal–C(=O)–R'$_8$ (Xb; Hal = Halogen) umsetzt und gewünschtenfalls in der erhaltenen Verbindung VIII, worin $X_6$ Hydroxy ist, die Hydroxygruppe durch Umsetzung mit einem Dihalogenalkan, Epoxyalkan oder Halogenalkanol, Dihalogen(hydroxy)alkan, Halogen(epoxy)alkan oder Halogenkandiol in einen durch Halogen, Epoxy oder Hydroxy substituierten Alkoxyrest überführt und gegebenenfalls Hydroxyalkoxy bzw. Dihydroxyalkoxy reaktionsfähig monoverestert, z.B. durch Behandeln mit Thionylchlorid, Phosphortribromid oder einem Sulfonsäurechlorid.

Die Umsetzung von Verbindungen IX und X gemäss Verfahrensvariante f) kann in üblicher, insbesondere in der aus der Literatur für analoge Umsetzungen, bekannten Weise erfolgen, erforderlichenfalls in Gegenwart eines Kondensationsmittels, bei der Umsetzung mit einem Esterhalogenid oder Amidhalogenid der Oxalsäure beispielsweise eines basischen Kondensationsmittels, wie einer tertiären organischen Stickstoffbase, z.B. von Triethylamin oder Pyridin, oder eines Alkalimetallhydroxides oder -carbonates, z.B. von Natrium- oder Kaliumhydroxid, oder bei der Umsetzung mit Oxalsäure beispielsweise eines die Dehydratisierung des primär gebildeten Ammoniumsalzes bewirkenden Kondensationsmittels, wie eines wasserbindenden Mittels, z.B. von Dicyclohexylcarbodiimid oder eines Isonitrils, wie von Tertiärbutylisonitril, oder einer Mineralsäure, z.B. von Chlorwasserstoffsäure, oder eines Säureanhydrids, z.B. von Phophorpentoxid, jeweils in einem inerten Lösungsmittel, wie einem Halogenalkan, z.B. in Dichlormethan, oder einem N,N-Dialkylamid, z.B. in N,N-Dimethylformamid oder -acetamid.

Die 1-Schutzgruppe von 5-Tetrazolylresten R'$_8$ kann anschliessend z.B. durch Acidolyse, d.h. Behandeln mit einer Säure, z.B. mit Trifluoressigsäure/Anisol, oder hydrogenolytisch, insbesondere mittels Wasserstoff und Palladium auf Kohle, abgespalten werden.

Ausgangsstoffe IX können beispielsweise hergestellt werden, indem man Verbindungen der Formeln

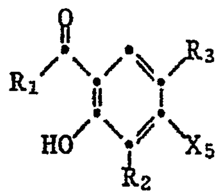 (VII) und 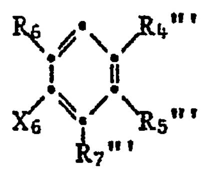 (XXX),

worin einer der Reste $R_4'''$; $R_5'''$ und $R_7'''$ die Nitrogruppe, ein davon verschiedener Rest $R_4'''$ oder $R_5'''$ einen Rest $R_9$ und ein davon verschiedener Rest $R_7'''$ einen Rest $R_{10}$ darstellt, miteinander umsetzt, worin einer der Reste $X_5$ und $X_6$ gegebenenfalls in Salzform vorliegendes Hydroxy und der andere einen durch reaktionsfähiges verestertes Hydroxy oder Epoxy substituierten Rest –O–alkH, z.B. eine Gruppe der Formeln

$$-O-(CH_2)_m-X,\quad -O-[(CH_2)_n-O]_k-(CH_2)_{n'}-X,$$

$$-O-[(CH_2)_l-O]_p-CH_2CH(OH)CH_2-[O-(CH_2)_{l'}]_o-X \text{ bzw.}$$

$$-O-[(CH_2)_l-O]_p-CH_2-X',$$

in der X reaktionsfähiges verestertes Hydroxy, z.B. Halogen, und X' 1,2-Epoxyethyl bedeutet, darstellt, und in der erhaltenen Verbindung der Formel

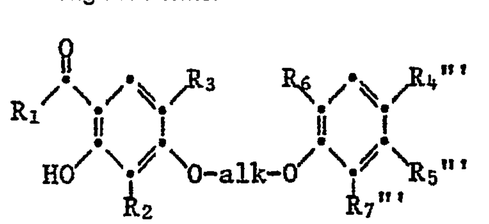 (IXa)

die Nitrogruppe zu Amino reduziert, beispielsweise durch Umsetzung mit Wasserstoff in Gegenwart eines Hydrierungskatalysators, wie von Palladium auf Kohle oder vor allem Raney-Nickel, beispielsweise in Tetrahydrofuran.

In 1-Stellung geschützte Tetrazole X werden vorzugsweise in situ hergestellt, z.B. durch Umsetzung eines Alkalimetall-, z.B. des Kaliumsalzes, einer in 1-Stellung geschützten, z.B. α-aralkylierten, Tetrazol-5-Carbonsäure mit Oxalylchlorid in Gegenwart von Pyridin.

Die Überführung der Gruppe $X_8$ in Verbindung XI in solche der Formel –NH–C(=O)–$R_8$ gemäss Verfahrensvariante g) erfolgt beispielsweise durch Solvolyse oder Oxidation oder ausgehend von Gruppen

$X_8$ der Formel –NH–C(=O)–CN durch Umsetzung mit Stickstoffwasserstoffsäure bzw. ausgehend von Gruppen $X_8$ der Formel –NHC(=O)–$R_8$', worin $R_8$' in 1-Stellung geschütztes 5-Tetrazolyl bedeutet, durch Abspaltung der Schutzgruppe. So können die genannten Gruppen $X_A$ in Resten $X_8$ der Formel –NH–$X_A$ hydrolytisch in die Oxalogruppe überführt werden. Als funktionell abgewandelte Carboxygruppe eine Iminoether-, Orthoester- oder Esterhalogenidgruppierung und/oder als funktionell abgewandelte α-Carbonylgruppe Thioxo- oder Iminomethylen oder eine veresterte oder veretherte Dihydroxymethylengruppe aufweisende Gruppe $X_A$ kann ferner zu veresterten Oxalogruppen –C(=O)–$R_8$ hydrolisiert werden. Ebenso können als funktionell abgewandelte Carboxygruppe die Cyanogruppe, eine Amidino- oder Imid- bzw. Amidhalogenidgruppierung und/oder als funktionell abgewandelte α-Carbonylgruppe Thioxo- oder Iminomethylen oder eine veretherte oder veresterte Dihydroxymethylengruppe aufweisende Gruppen n$X_A$ zu amidierten Oxalogruppen –C(=O)–$R_8$ hydrolysiert werden. Die Hydrolyse kann in üblicher Weise durchgeführt werden, erforderlichenfalls in Gegenwart eines basischen oder vorzugsweise sauren Hydrolysemittels, wie eines Alkalimetallhydroxides, wie Natrium- oder Kaliumhydroxid, oder vorzugsweise einer Protonensäure, vorzugsweise einer Mineralsäure, z.B. einer Halogenwasserstoffsäure, wie Salzsäure oder einer organischen Carbon- oder Sulfonsäure, z.B. von Essigsäure oder p-Toluolsulfonsäure.

Als funktionell abgewandelte Carboxygruppe eine anhydridisierte Carboxygruppe, wie Halogencarbonyl, z.B. Chlorcarbonyl, Cyanocarbonyl oder eine Niederalkyleniminethergruppierung, z.B. 4,4- oder 5,5-Dimethyl-oxa-zolinyl-(2), oder 4,4,6-Trimethyl-dihydro-oxazinyl-(2), aufweisende funktionell abgewandelte Oxalogruppen n$X_A$' können ferner durch übliche Alkoholyse, d.h. Umsetzung mit dem entsprechenden Alkohol, in veresterte Oxalogruppen –C(=O)–$R_8$ überführt werden. Bei der Alkoholyse von anhydridisierten Carboxygruppen arbeitet man vorteilhaft in Gegenwart eines basischen Kondensationsmittels, z.B. von Pyridin oder Triethylamin, während man die Alkoholyse von Carboxy oder einer Niederalkyleniminoethergruppierung vorzugsweise sauer, z.B. in Gegenwart von Chlorwasserstoffsäure, p-Toluolsulfonsäure oder Essigsäure, durchführt. In analoger Weise kann man eine anhydridisierte Carboxygruppe aufweisende funktionell abgewandelte Oxalogruppe auch durch Ammono- bzw. Aminolyse, d.h. Umsetzung mit Ammoniak oder eines entsprechenden primären oder sekundären Amins, vorzugsweise in Gegenwart eines basischen Kondensationsmittels, z.B. von Natriumhydroxid, Pyridin oder Triehtylamin, in eine amidierte Oxalogruppe C(=O)–$R_8$ überführen.

Die Überführung der genannten Gruppen $X_B$ in solche der Formel –NH–C(=O)–$R_8$ erfolgt beispielsweise oxidativ. Die Oxidation kann in üblicher Weise durch Umsetzung mit einem geeigneten Oxydationsmittel erfolgen. Geeignete Oxydationsmittel sind insbesondere oxydierende Schwermetallverbindungen, wie Silberverbindungen, z.B. Silbernitrat oder Silberpicolinat, Sauerstoffsäuren von Schwermetallen, z.B. von Mangan-IV, Mangan-VII, Chrom-VI und Eisen-III, oder von Halogen bzw. deren Anhydride oder Salze, wie Chromsäure, Chromdioxid, Kaliumdichromat, Kaliumpermanganat, Mangandioxid, Kaliumhexacyanoferrat, Natriumchlorit in Gegenwart von Sulfaminsäure, Natriumhypochlorit in Gegenwart von Nickelchlorid oder Natriumjodat, Natriumperjodat oder Bleitetraacetat. Die Umsetzung mit diesen Oxydationsmitteln erfolgt in üblicher Weise, beispielsweise in einem inerten Lösungsmittel, wie Aceton, Essigsäure, Pyridin oder Wasser, oder einem, vorzugsweise wässrigen, inerten Lösungsmittelgemisch, bei Normaltemperatur oder erforderlichenfalls unter Kühlen oder Erwärmen, z.B. bei etwa 0° bis etwa 100°C. Die Oxydation von gegebenenfalls veretherten Glykoloylgruppen zu gegebenenfalls veresterten Oxalogruppen wird z.B. vorteilhaft mit Kaliumpermanganat in wässrigem Pyridin oder Aceton bei Raumtemperatur vorgenommen. Acetalisierte Glyoxylgruppen und Iminoacetylgruppen werden vorzugsweise sauer oxydiert, z.B. mit Kaliumdichromat in Schwefelsäure, Acylgruppen von α,β-dihydroxylierten aliphatischen Carbonsäuren, wie der Acylrest der Weinsäure, werden vorteilhaft mit Perjodsäure oxydiert, während man für die Oxydation der Glycylgruppe vorzugsweise Kaliumferrat in alkalischem Milieu, z.B. bei pH 10–13, z.B. 11,5 oder organische Silbersalze, wie Silberpicolinat, verwendet. Gruppen der Formel –N=CH–$R_8$ werden vorzugsweise mit einer organischen Persäure, z.B. mit Peressigsäure oder m-Chlorperbenzoesäure, in einem inerten Lösungsmittel, z.B. Dichlormethan, Chloroform oder Benzol, oxydiert.

Die Umsetzung von Gruppen $X_8$ der Formel –NH–C(=O)–CN mit Stickstoffwasserstoffsäure erfolgt vorzugsweise unter Bildung derselben in situ durch Behandeln eines Alkalimetallazides mit einer Säure, wie Chlorwasserstoffsäure, vorzugsweise in Toluol oder ähnlichen Lösungsmitteln.

Die Abspaltung der Schutzgruppe aus Gruppen $X_8$ der Formel –NH–C(=O)–$R_8$', worin $R_8$' in 1-Stellung geschütztes 5-Tetrazolyl ist, erfolgt in üblicher Weise, insbesondere durch Acidolyse, d.h. Behandeln mit einer Säure, z.B. mit Trifluoressigsäure in einem Ether, wie Anisol, oder durch katalytische Hydrierung, z.B. in Gegenwart von Palladium.

Die Ausgangsstoffe XI werden beispielsweise hergestellt, indem man eine Verbindung der Formel

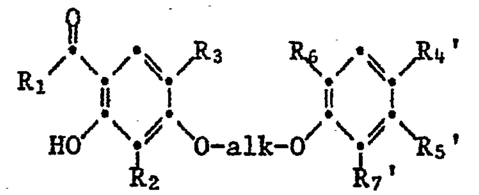

(IX) ,

worin einer der Reste $R_4'$, $R_5'$ und $R_7'$ die Aminogruppe, ein davon verschiedener Rest $R_4'$ bzw. $R_5'$ einen Rest $R_9$ und ein davon verschiedener Rest $R_7'$ einen Rest $R_{10}$ darstellt oder ein Säureadditionssalz davon mit einer entsprechenden Säure, z.B. der Formel $X_A$–OH (XXXIIa) bzw. $X_B$–OH (XXXIIb) oder $R_8$ –COOH (XXXIIc) oder einem funktionellen Derivat davon umsetzt. Funktionelle Derivate von Säuren XXXIIa bis XXXIIc sind vor allem eine veresterte, amidierte oder anhydridisierte Carboxygruppe, wie Niederalkoxycarbonyl, gegebenenfalls substituiertes Carbamyl, z.B. Carbamyl oder Imidazolyl-1-carbonyl, oder Halogencarbonyl, z.B. Chlor oder Bromcarbonyl, oder eine Gruppe der Formel $-CON_3$oder $-CON_2^{\oplus}$ $Hal^{\ominus}$enthaltende Säurederivate. Als Beispiele für Säuren XXXIIa bis XXXIIc und deren funktionelle Derivate seien insbesondere genannt: Als funktionelle Derivate von Säuren XXXIIa Oxalylhalogenide, wie Oxalylchlorid oder Oxalylbromid, Triniederalkoxy- und Dihalogenniederalkoxyessigsäureniederalkylester, wie Oxalsäuretetraethylester oder Dichloroxalsäurediethylester, Oxalsäureiminodialkylester, wie Oxalsäuremono- oder -diiminodiethylester, Oxalsäureamidine, wie N-Niederalkyloxalsäureesteramidine, Oxalsäuredithioniederalkyl-, wie -dimethylester, Cyanoformylchlorid oder Cyanogen und als Säuren XXXIIb und deren funktionelle Derivate Glykolsäuren und ihre Niederalkylester bzw. das entsprechende Lactid, z.B. Ethoxy- oder Diethoxyessigsäureethylester, Halogenacetanhydride, wie Chloracetanhydrid oder Chloracetylchlorid und Weinsäure, bzw. 2,3-Diacetoxybernsteinsäureanhydrid, ferner Cinnamoylchlorid, Acetylchlorid und Glycin. Funktionelle Derivate von Säuren XXXIIc sind insbesondere deren Chloride.

Die Umsetzung von Verbindungen IX und XXXIIa bis XXXIIc oder ihrer Derivate kann in üblicher Weise erfolgen, beispielsweise in Gegenwart eines wasserbindenden Mittels, wie eines Säurenahydrides, z.B. von Phosphorpentoxid, oder von Dicyclohexylcarbodiimid, oder eines z.B. sauren oder basischen Kondensationsmittels, wie einer Mineralsäure, z.B. von Chlorwasserstoffsäure, oder eines Alkalimetallhydroxides oder -carbonates, z.B. von Natrium- oder Kaliumhydroxid, oder einer organischen Stickstoffbase, z.B. von Triethylamin oder Pyridin. Bei der Umsetzung mit einem Säureanhydrid, wie Säurechlorid, verwendet man vorzugsweise eine organische Stickstoffbase als Kondensationsmittel.

Die Umsetzung mit Carbonsäuren führt man vorzugsweise in Gegenwart eines wasserbindenden Mittels durch. Erforderlichenfalls arbeitet man jeweils in einem inerten Lösungsmittel, bei normaler Temperatur oder unter Kühlen oder Erwärmen, z.B. ein einem Temperaturbereich von etwa 0° bis etwa 100°C, in einem geschlossenen Gefäss und/oder unter Inertgas, z.B. Stickstoff.

Analog kann man Verbindungen XI, in denen $X_8$ eine Gruppe $R_8$–CH=N– bedeutet, durch Kondensation von Verbindungen IX mit der gegebenenfalls veresterten oder amidierten Glyoxylsäure herstellen.

Verbindungen XI, in denen $X_8$ für eine Gruppe –NH–$X_B$ und $X_B$ für Glyoxyloyl steht, können ferner hergestellt werden, indem man eine entsprechende Halogen-, wie Bromacetylverbindung mit Hexamethylentetramin, vorzugsweise in einem wässrigen Alkohol, erhitzt oder mit Silbertetrafluoroborat in Dimethylsulfoxid oxydiert. Analog kann man auch eine Chloracetylverbindung mit Kaliumdichromat in Hexamethylphosphorsäuretriamid in Gegenwart von Dicyclohexyl-18-crown-6-ether oxydieren. Verbindungen XI, in denen $X_8$ eine Gruppe –NH–$X_B$ und $X_B$ eine Iminoacetylgruppe, z.B. gegebenenfalls substituiertes Benzyliminoacetyl, bedeutet, können ausgehend von den entsprechenden Glycylverbindungen hergestellt werden, indem man diese mit der entsprechenden Carbonylverbindung, z.B. mit Benzaldehyd, umsetzt und das so erhältliche Zwischenprodukt, z.B. eine N-Benzylidenglycylverbindung, vorzugsweise unter den Reaktionsbedingungen, umlagert.

Als funktionell abgewandelte Carboxygruppe eine Iminoethergruppierung aufweisende funktionell abgewandelte Oxalogruppen können ausgehend von der entsprechenden Cyanocarbonylverbindung durch Umsetzung mit dem entsprechenden Alkohol, z.B. Niederalkan(di)ol oder Aminoniederalkanol, hergestellt werden.

Die Überführung des Restes alk' einer Verbindung I' gemäss Verfahrensvariante h) erfolgt in üblicher Weise, ausgehend von Verbindungen I', worin alk' einen durch Oxo, mit einem α-Aralkanol verähthertes oder einem Kohlensäuremono-α-aralkylester verestertes Hydroxy substituierten Rest alk bedeutet, beispielsweise reduktiv und ausgehend von Verbindungen IX, worin alk' einen durch andere verätherte oder veresterte Hydroxygruppen als die vorstehend genannten substituierten Rest alk bedeutet, solvolytisch.

Als Reduktionsmittel für die Reduktion von durch Oxo substituierten Resten alk' zu den entsprechenden durch Hydroxy substituierten Resten alk kommen beispielsweise Alkalimetallborhydride, wie Lithiumborhydrid, Natriumborhydrid oder Natriumcyanoborhydrid, oder sekundäre Alkohole, wie sekundäre Niederalkanole oder Cycloniederalkanole, z.B. Isopropanol oder Cyclohexanol, in Gegenwart eines Aluminiumalkoholats, insbesondere Isopropanol in Gegenwart von Aluminiumisopropanolat, in Betracht. Die Umsetzung mit den genannten Alkalimetallborhydriden wird vorteilhaft in einem Niederalkanol, einem Diniederalkylether oder Niederalkylenether oder Gemischen dieser Lösungsmittel, z.B. in Äthanol, durchgeführt. Bei der Umsetzung mit Alkoholen in Gegenwart eines Aluminiumalkoholats arbeitet man zweckmässigerweise in einem Überschuss des als Reduktionsmittel verwendeten Alkohols.

Durch Oxo substituierte Reste alk' können aber auch unter Ersatz der Oxogruppe durch 2 Wasserstoffatome zu den entsprechenden unsubstituierten Resten alk' reduziert werden, indem man mit Hydrazin bzw. Semicarbazid und einer Base, z.B. einem Alkalimetallniederalkanolat, wie Natriummethanolat, oder in einem hochsiedenden Alkohol, z.B. Di- oder Triethylenglykol oder Diethylenglykolmonomethylether, mit Hydrazin und einem Alkalimetallhydroxid, wie Kaliumhydroxid umsetzt, vorzugsweise unter Erwärmen, z.B. bei der Siedehitze. Dabei werden intermediär die entsprechenden Hydrazone bzw. Semicarbazone gebildet. Man kann aber auch mit einem Sulfonsäurehydrazid, z.B. mit p-Toluolsulfonsäurehydrazid, zu den entsprechenden N'-Sulfonylhydrazonen, z.B. N'-(p-Toluolsulfonyl)-hydrazonen, umsetzen und diese mit einem Alkalimetallborhydrid, z.B. mit Lithium- oder Natriumborhydrid, reduzieren.

Die reduktive Spaltung von α-Aralkoxy bzw. α-Aralkoxycarbonyloxy zu Hydroxy erfolgt vorzugsweise durch Hydrogenolyse, d.h. Einwirken von Wasserstoff in Gegenwart eines Hydrierungskatalysators, wie eines Palladium-, Platin-, Iridium- oder Nickel-Katalysators, z.B. von Palladium auf Kohle, Platinoxid oder Raney-Nickel, vorteilhaft in einem Niederalkanol, z.B. in methanolischer Lösung, erforderlichenfalls unter Überdruck und/oder Erwärmen, z.B. bei etwa 1 bis 10 bar und 20° bis 60°C.

Die solvolytische Freisetzung von Hydroxy aus anderen als den genannten verätherten oder veresterten Hydroxygruppen, wie aus Silyloxy, Niederalkanoyloxy oder Niederalkoxycarbonyloxy, erfolgt beispielsweise durch Hydrolyse (Umsetzung mit Wasser), Alkoholyse (Umsetzung mit einem Alkohol) oder Ammono- bzw. Aminolyse (Umsetzung mit Ammoniak oder einem mindestens ein freies Wasserstoff aufweisenden Amin), erforderlichenfalls in Gegenwart eines sauren oder basischen Mittels und/oder unter Erwärmen, z.B. bei etwa 20° bis 100°C. Geeignete saure Mittel sind beispielsweise Mineralsäuren, wie Halogenwasserstoffsäuren, z.B. Chlorwasserstoffsäure, oder Sauerstoffsäuren des Schwefels oder Phosphors, z.B. Schwefel- oder Phosphorsäure, oder organische Sulfon- oder Carbonsäuren, wie Niederalkansulfonsäuren oder gegebenenfalls substituierte Benzolsulfonsäuren bzw. Niederalkansäuren, z.B. p-Toluolsulfonsäure oder Essigsäure. Basische Hydrolysemittel sind beispielsweise Hydroxide oder Carbonate von Alkalimetallen, z.B. Kaliumcarbonat, Natriumhydroxid oder Kaliumhydroxid, für die Alkoholyse ferner entsprechende Alkoholate, wie Alkalimetallniederalkanolate, z.B. Natriummethanolat.

Ausgangsstoffe I' können beispielsweise erhalten werden, indem man Verbindungen der Formeln

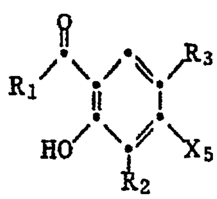 (VII) und 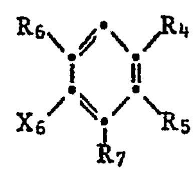 (VIII)

untereinander umsetzt, worin einer der Reste $X_5$ und $X_6$ ggf. in Salzform vorliegendes Hydroxy und der andere einen Rest –O–alk'–X, d.h. einen durch Oxo oder verethertes oder verestertes Hydroxy substituierten Rest –O–alk–X, wie einen durch X substituierten Oxoalkoxy-, Oxo(oxa)alkoxy-, Oxo(dioxa)alkoxy-, oder veretherten oder veresterten Hydroxy(oxa)alkoxy- oder Hydroxy(dioxa)alkoxyrest, z.B. der Formeln

$$-O-[(CH_2)_{l'}-O]_o-CH_2C(=O)CH_2-[O-(CH_2)_{l}]_p-X \quad (Ib^{III}) \quad \text{oder}$$

$$-O-[(CH_2)_{l'}-O]_o-CH_2CH(X'')CH_2-[O-(CH_2)_{l}]_p-X \quad (Ib^{IV})$$

bedeutet, worin X reaktionsfähiges verestertes Hydroxy, z.B. Halogen, bedeutet.

Eine erfindungsgemäss erhältliche Verbindung der allgemeinen Formel I kann in an sich bekannter Weise in eine andere Verbindung der allgemeinen Formel I umgewandelt werden.

So kann man beispielsweise Verbindungen der Formel I, worin eine freie Carboxylgruppe $R_6$, $R_7$ und/oder $R_8$ vorliegt, in üblicher Weise, z.B. durch Behandeln mit einem Diazoniederalkan oder Triniederalkyloxonium-, Triniederalkylcarboxonium- oder Diniederalkylcarboniumsalz, wie -hexachloroantimonat oder -hexafluorophosphat, oder vor allem durch Umsetzung mit dem entsprechenden Alkohol oder einem reaktionsfähigen Derivat, wie einem Carbon-, Phosphorig-, Schweflig- oder Kohlensäureester davon, z.B. einem Niederalkanol, N,N-Diniederalkylaminoniederalkanol, N,N-Niederalkylenaminoniederalkanol, gegebenenfalls substituiertem N,N-(Aza)-niederalkylenaminoniederalkanol N,N-(Oxa)niederalkylenaminoniederalkanol oder N,N-(Thia)Niederalkylenaminoniederalkanol bzw. mit einem Niederalkancarbonsäureester, Triniederalkylphosphit oder Diniederalkylsulfit, zu Verbindungen der all-

gemeinen Formel I worin $R_6$, $R_7$ und/oder $R_8$ verestertes Carboxy ist, umsetzen. Die Umsetzung mit dem entsprechenden Alkohol selbst kann vorteilhaft in Gegenwart eines sauren Katalysators erfolgen, wie einer Protonensäure, z.B. von Chlor- oder Bromwasserstoff-, Schwefel-, Phosphor-, Bor-, Benzolsulfon- und/oder Toluolsulfonsäure, in einem inerten Lösungsmittel, insbesondere einem Überschuss des eingesetzten Alkohols und erforderlichenfalls in Gegenwart eines wasserbindenden Mittels und/oder unter destillativer, z.B. azetroper, Entfernung des Reaktionswassers und/oder bei erhöhter Temperatur. Die Umsetzung mit einem reaktionsfähigen Derivat des entsprechenden Alkohols kann in üblicher Weise durchgeführt werden, ausgehend von einem Carbon-, Phosphorig-, Schweflig- oder Kohlensäureester beispielsweise in Gegenwart eines sauren Katalysators, wie eines der vorstehend genannten, in einem inerten Lösungsmittel, z.B. in Toluol, oder einem Überschuss des eingesetzten Alkoholderivates oder des entsprechenden Alkohols, erforderlichenfalls unter, z.B. azeotroper Abdestillation des Reaktionswassers. Ausgehend von einem Mineralsäure- oder Sulfonsäureester setzt man die zu veresternde Säure vorteilhaft in Form eines Salzes, z.B. des Natrium- oder Kaliumsalzes, ein und arbeitet erforderlichenfalls in Gegenwart eines basischen Kondensationsmittels, wie einer anorganischen Base, z.B. von Natrium-, Kalium-, oder Calciumhydroxid oder -carbonat, oder einer tertiären organischen Stickstoffbase, z.B. von Triethylamin oder Pyridin, und/oder in einem inerten Lösungsmittel, wie einer der vorstehenden tertiären Stickstoffbasen oder eines polaren Lösungsmittels, z.B. in Dimethylformamid und/oder bei erhöhter Temperatur. Die Umsetzung mit einem Olefin kann beispielsweise in Gegenwart eines sauren Katalysators, z.B. einer Lewissäure, z.B. von Bortrifluorid, einer Sulfonsäure, z.B. von p-Toluolsulfonsäure, oder vor allem eines basischen Katalysators, z.B. von Natrium- oder Kaliumhydroxid, vorteilhaft in einem inerten Lösungsmittel, wie einem Ether, z.B. in Diethylether oder Tetrahydrofuran, erfolgen.

Eine freie Carboxylgruppe $R_6$, $R_7$ und/oder $R_8$ kann ferner durch Umsetzung mit Ammoniak oder einem mindestens ein Wasserstoffatom aufweisenden Amin in üblicher Weise, unter Dehydratisieren des intermediär gebildeten Ammoniumsalzes, z.B. durch azeotrope Destillation mit Benzol oder Toluol oder trockenes Erhitzen, in eine amidierte Carboxylgruppe überführt werden.

Die vorstehend beschriebenen Umwandlungen von Carboxy in veresterte oder amidierte Carboxygruppen können aber auch so durchgeführt werden, dass man eine Verbindung der Formel I, worin $R_6$, $R_7$ und/oder $R_8$ Carboxy ist, zunächst in üblicher Weise in ein reaktionsfähiges Derivat, beispielsweise mittels eines Halogenides des Phosphors oder Schwefels, z.B. mittels Phosphortrichlorid oder -bromid, Phosphorpentachlorid oder Thionylchlorid, in ein Säurehalogenid oder durch Umsetzung mit einem entsprechenden Alkohol oder Amin in einen reaktiven Ester, d.h. Ester mit elektronenanziehenden Strukturen, wie den Ester mit Phenol, Thiophenol, p-Nitrophenol oder Cyanomethylalkohol, oder ein reaktives Amid, z.B. das von Imidazol oder 3,5-Dimethylpyrazol abgeleitete Amid, überführt und das erhaltene reaktionsfähige Derivat dann in üblicher Weise, z.B. wie nachstehend für die Umesterung, Umamidierung bzw. gegenseitige Umwandlung veresterter und amidierter Carboxylgruppen beschrieben, mit einem entsprechenden Alkohol, Ammoniak oder dem entsprechenden mindestens ein Wasserstoffatom aufweisenden Amin zu der gewünschten Gruppe $R_4$, $R_6$ und/oder $R_8$ umsetzt.

Eine veresterte Carboxylgruppe $R_6$, $R_7$ und/oder $R_8$ sowie Cyano $R_6$ und/oder $R_7$ kann in üblicher Weise, z.B. durch Hydrolyse in Gegenwart eines Katalysators, beispielsweise eines basischen oder sauren Mittels, wie einer starken Base, z.B. von Natrium- oder Kaliumhydroxid, oder einer Mineralsäure, z.B. von Salzsäure, Schwefelsäure oder Phosphorsäure, zur freien Carboxylgruppe, Cyano $R_6$ und/oder $R_7$ ferner zu Carbamyl hydrolysiert werden. Ebenso kann man verestertes Carboxy $R_6$, $R_7$ und/oder $R_8$, z.B. durch Umsetzung mit Ammoniak oder dem entsprechenden, mindestens ein Wasserstoffatom aufweisenden Amin, in eine amidierte Carboxylgruppe überführt.

Eine veresterte Carboxylgruppe $R_6$, $R_7$ und/oder $R_8$ kann ferner in üblicher Weise, z.B. durch Umsetzung mit einem Metallsalz, wie dem Natrium- oder Kaliumsalz, eines entsprechenden Alkohols oder mit diesem selbst in Gegenwart eines Katalysators, beispielsweise einer starken Base, z.B. von Natrium- oder Kaliumhydroxid, oder einer starken Säure, wie einer Mineralsäure, z.B. von Salzsäure, Schwefelsäure oder Phosphorsäure, oder einer organischen Sulfonsäure, z.B. von p-Toluolsulfonsäure, oder einer Lewissäure, z.B. von Bortrifluorid-Etherat, zu einer anderen veresterten Carboxylgruppe umgeestert werden.

Eine amidierte Carboxylgruppe $R_6$, $R_7$ und/oder $R_8$ kann in üblicher Weise, z.B. durch Hydrolyse in Gegenwart eines Katalysators, beispielsweise einer starken Base, wie eines Alkalimetall- oder Erdalkalimetallyhydroxids oder -carbonats, z.B. von Natrium- oder Kaliumhydroxid oder -carbonat, oder einer starken Säure, wie einer Mineralsäure, z.B. von Salzsäure, Schwefelsäure oder Phosphorsäure, in die freie Carboxylgruppe umgewandelt werden.

In einer erfindungsgemäss erhältlichen Verbindung kann man weiterhin in einen oder beide Phenylringe Substituenten einführen und/oder vorhandene Substituenten in andere Substituenten umwandeln. So kann man durch Umsetzung mit einem Niederalkylhalogenid oder Niederalken bzw. einem Niederalkansäurehalogenid oder -anhydrid, jeweils in Gegenwart einer Lewissäure, wie Aluminiumtrichlorid, Niederalkyl bzw. Niederalkanoyl einführen. Ferner kann man Halogen einführen, beispielsweise durch Behandeln mit einem Halogen in Gegenwart einer Lewissäure, wie Eisen-III-chlorid, oder durch Umsetzung mit N-Chlorsuccinimid. Ferner kann man Halogen, insbesondere Jod, durch Umsetzung mit Trifluorjodmethan in Gegenwart von Kupfer durch Trifluormethyl ersetzen.

Die neuen Verbindungen können, wie erwähnt, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden oder als Isomerengemisch, wie Racemate, Diastereoisomerengemische oder Racematgemische, vorliegen.

Erhaltene Diastereoisomerengemische oder Racematgemische können aufgrund der physikalischchemischen Unterschiede der Bestandteile in bekannter Weise in die reinen Isomeren, Diastereomeren oder Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen, oder durch Umsetzen eines sauren Endstoffes mit einer mit der racemischen Säure Salze bildenden optisch aktiven Base und Trennung der auf diese Weise erhaltenen Salze, z.B. aufgrund ihrer verschiedenen Löslichkeiten, in die Diastereomeren, aus denen die Antipoden durch Einwirkung geeigneter Mittel freigesetzt werden können. Vorteilhaft isoliert man den wirksameren der beiden Antipoden.

Erhaltene freie Verbindungen der Formel I, z.B. solche, worin $R_6$ und/oder $R_7$ für Carboxy und/oder $R_8$ für Carboxy oder 5-Tetrazolyl steht, können in an sich bekannter Weise in Salze überführt werden, z.B. durch Behandeln mit einer Base oder mit einem geeigneten Salz einer Carbonsäure, üblicherweise in Gegenwart eines Lösungs- oder Verdünnungsmittels.

Erhaltene Salze können in an sich bekannter Weise in die freien Verbindungen bzw. erhaltene freie Verbindungen, worin $R_8$ zur Bildung von Säureadditionssalzen befähigt ist, in deren Säureadditionssalze umgewandelt werden, z.B. durch Behandeln mit einem sauren Reagens, wie einer Mineralsäure bzw. einer der genannten salzbildenden Säuren.

Die Verbindungen einschliesslich ihrer Salze können auch in Form ihrer Hydrate erhalten werden oder das zur Kristallisation verwendete Lösungsmittel einschliessen.

Infolge der engen Beziehungen zwischen den neuen Verbindungen in freier Form und in Form ihrer Salze sind im vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes und/oder Racemates, bzw. Antipoden verwendet oder insbesondere unter den Reaktionsbedingungen bildet.

Die bei den erfindungsgemässen Verfahren und ihren Vorstufen auftretenden neuen Ausgangsstoffe und Zwischenprodukte sowie Verfahren zu ihrer Herstellung bilden ebenfalls Gegenstand der Erfindung.

Vorzugsweise werden solche Ausgangsstoffe verwendet und die Reaktionsbedingungen so gewählt, dass man zu den vorstehend als besonders bevorzugt aufgeführten Verbindungen gelangt.

In diesem Zusammenhang sei insbesondere auf Verbindungen der Formeln VII, worin $X_5$ Hydroxy bedeutet, sowie XII und ihre Salze hingewiesen, die speziell als Ausgangsstoffe für Verbindungen der Formel I entwickelt wurden.

Die Erfindung betrifft dementsprechend ebenfalls Verbindungen der Formel

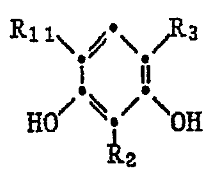 (XXXIII),

worin $R_2$ fluoriertes Niederalkyl bedeutet, $R_3$ Wasserstoff, Niederalkoxy, Trifluormethyl oder Halogen bedeutet und $R_{11}$ Wasserstoff oder eine Gruppe der Formel $R_1$–C(=O)– bedeutet, in der $R_1$ für Niederalkyl steht, wobei Halogen eine Atomnummer bis und mit 53 aufweisen kann und "niedere" Gruppen bis und mit 7 C-Atome haben können, und ihre Salze, ein Verfahren zu ihrer Herstellung und ihre Verwendung als Zwischenprodukte, z.B. zur Herstellung von Arzneimittelwirkstoffen.

Dabei haben $R_1$, $R_2$ und $R_3$ beispielsweise die für Verbindungen I angegebenen Bedeutungen. Ebenso sind Salze von Verbindungen XXXIII vorzugsweise Metallsalze vom für Verbindungen I angegebenen Typ.

Die Erfindung betrifft beispielsweise Verbindungen der Formeln VII, worin $X_5$ Hydroxy ist, und/oder Verbindungen der Formel XII und jeweils ihre Salze, Verfahren zu ihrer Herstellung und ihre Verwendung.

Die Erfindung betrifft vor allem Verbindungen der Formeln VII, XII oder XXXIII, worin $R_1$ für Niederalkyl mit bis und mit 4 C-Atomen, wie Methyl, steht, $R_2$ ω-Fluor, ω,ω-Difluor- oder ω,ω,ω-Trifluorniederalkyl mit bis und mit 4, z.B. 1 oder 3, C-Atomen, wie Trifluormethyl oder 3,3,3-Trifluorpropyl, ist und $R_3$ Wasserstoff bedeutet, und ihre Salze, Verfahren zu ihrer Herstellung und ihre Verwendung.

Die Erfindung betrifft vorzugsweise Verbindungen der Formeln VII, XII oder XXXIII, worin $R_1$ Niederalkyl mit bis und mit 4 C-Atomen, wie Methyl, bedeutet, $R_2$ ω,ω,ω-Trifluorniederalkyl mit bis und mit 4, z.B. 1 oder 3, C-Atomen, wie Trifluormethyl oder 3,3,3-Trifluorpropyl, ist und $R_3$ Wasserstoff bedeutet, und ihre Salze, Verfahren zu ihrer Herstellung und ihre Verwendung.

Die Erfindung betrifft namentlich 2-(3,3,3-Trifluorpropyl)-resorcin der Formel XII und 4-Acetyl-2-(3,3,3-trifluorpropyl)-resorcin der Formel VII in freier Form oder in Salzform, Verfahren zur Herstellung derselben und deren Verwendung.

Die Erfindung betrifft ferner ein Verfahren zur Herstellung von Verbindungen der Formel XXXIII oder ihrer Salze. Dieses ist dadurch gekennzeichnet, dass man

i) in einer Verbindung der Formel

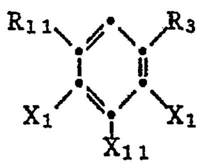

(XXIV),

worin mindestens einer der Reste $X_1$ verethertes Hydroxy R und ein davon verschiedener Rest $X_1$ freies Hydroxy bedeutet und $X_{11}$ eine gegen $R_2$ austauschbare oder in $R_2$ überführbare Gruppe darstellt, $X_{11}$ gegen $R_2$ austauscht oder in $R_2$ überführt, verethertes Hydroxy R zu Hydroxy spaltet und gewünschtenfalls die verfahrensgemäss erhältliche Verbindung der Formel XXXIII in eine andere Verbindung der Formel XXXIII überführt, insbesondere anstelle von Wasserstoff $R_{11}$ eine Gruppe der Formel $R_1$–C(=O)– einführt und/oder eine verfahrensgemäss erhältliche freie Verbindung der Formel XXXIII in ein Salz oder ein verfahrensgemäss erhältliches Salz in die freie Verbindung der Formel XXXIII überführt.

Gegen Reste $R_2$ austauschbare Gruppen $X_{11}$ sind beispielsweise Halogenatome –Hal der Atomnummer 19 bis und mit 53, d.h. Chlor, Brom oder Jod, vor allem Brom oder Jod, oder metallische Reste, beispielsweise eine Gruppe der Formeln $-M^I$, $-M^{II}/2$ oder $-M^{II}-Hal$, worin $M^I$ ein Alkalimetallatom, z.B. Lithium oder Natrium, oder einwertiges Kupfer, $M^{II}$ ein Erdalkalimetallatom, z.B. Magnesium, Zink, Cadmium, oder zweiwertiges Kupfer und –Hal Halogen der Atomnummer 19 bis und mit 53 bedeutet.

Der Austausch der genannten Gruppen $X_{11}$ gegen Reste $R_2$ erfolgt in üblicher Weise, beispielsweise durch Umsetzung mit einer Verbindung der Formel $X_{10}$–$R_2$ (XV), worin einer der Reste $X_{10}$ und $X_{11}$ einen metallischen Rest und der andere Halogen bedeutet, bzw. durch Umsetzen mit einer Verbindung der Formel $X_{10}$–$R_2$ (XV), worin beide Reste $X_{10}$ und $X_{11}$ ein Halogen –Hal bedeuten, in Gegenwart eines Metalles, vorzugsweise eines der genannten, insbesondere von Kupferpulver. Die Umsetzung von Verbindungen XXXIV und XV, worin $X_{10}$ und $X_{11}$ einen metallischen Rest bedeutet, erfolgt vorzugsweise in Gegenwart eines aliphatischen oder cycloaliphatischen Ethers, wie eines Diniederalkylethers, z.B. von Diethylether oder Tertiärbutoxymethan, oder eines 5- bis 7-gliedrigen Oxa- oder Dioxacycloalkens, z.B. von Tetrahydrofuran oder 1,4-Dioxan, erforderlichenfalls in Gegenwart eines Lösungs- oder Verdünnungsmittels, z.B. von Dimethylformamid, vorteilhaft unter Inertgas, wie Argon. Die Umsetzung von Verbindungen XXXIV und XV, worin $X_{10}$ und $X_{11}$ beide Halogen Hal bedeuten, in Gegenwart eines Metalls wird vorzugsweise unter Erwärmen z.B. auf etwa 80° bis 150°C in einem inerten, hochsiedenden Lösungsmittel, wie Dimethylformamid, 1,2-Dimethoxyethan, 1,5-Dimethoxy-(3-oxa)-butan oder Diphenylether, vorteilhaft ebenfalls unter Inertgas, wie Argon, vorgenommen.

Ausgangsstoffe XV bzw. XXXIV, worin $X_{10}$ bzw. $X_{11}$ einen metallischen Rest bedeutet, werden vorzugsweise durch Behandeln der entsprechenden Verbindungen XV bzw. XXXIV, worin $X_{10}$ bzw. $X_{11}$ ein Halogen Hal ist, mit dem entsprechenden Metall <u>in situ</u> gebildet und ohne Isolierung eingesetzt.

Ausgehend von Verbindungen XXXIV, worin $R_3$ ebenfalls ein Halogen Hal bedeutet, werden die genannten Austauschreaktionen durch Nebenreaktionen gestört. Diese können umgangen werden, indem man von einer entsprechenden Nitroverbindung ausgeht und nachfolgend in üblicher Weise die Nitrozur Aminogruppe reduziert, z.B. mit Eisen und Salzsäure, und diese, z.B. mittels Natriumnitrit in einer Halogenwasserstoffsäure H–Hal, durch das gewünschte Halogenatom ersetzt. Die Kondensation von Verbindungen XV und XXXIV, worin $X_{10}$ bzw. $X_{11}$ einen metallischen Rest bedeutet, ist ferner nicht besonders geeignet für die Herstellung von Verbindungen XXXIII, worin $R_{11}$ eine Acylgruppe $R_1$–C(=O)– ist.

In einer bevorzugten Ausführungsform erhält man beispielsweise durch Erwärmen einer Verbindung XXXIV, worin $X_{11}$ für Brom oder Jod, $X_1$ für verethertes Hydroxy R und $R_{11}$ für Wasserstoff stehen, mit Trifluormethyljodid und Kupferpulver in Dimethylformamid auf etwa 120°–180°C und nachfolgend Überführung von verethertem Hydroxy R in Hydroxy Verbindungen der Formel XXXIII, worin $R_{11}$ Wasserstoff und $R_2$ Trifluormethyl bedeutet.

In Reste $R_2$ überführbare Gruppen $X_{11}$ sind beispielsweise fluorierte Niederalkenylreste, wie 3-Fluor-, 3,3-Difluor- oder 3,3,3-Trifluorprop-1-enyl bzw. 3-Fluor- oder 3,3-Difluorprop-2-enyl, fluorierte Niederalkinylreste, wie 3-Fluor-, 3,3-Difluor- oder 3,3,3-Trifluorprop-1-inyl, ferner fluorierte Hydroxyniederalkylreste, wie 3-Fluor-, 3,3-Difluor- oder 3,3,3-Trifluor-1-hydroxy-propyl.

Die Überführung der genannten Gruppen $X_{11}$ in Reste $R_2$ erfolgt beispielsweise durch Reduktion. Als Reduktionsmittel kommt beispielsweise Wasserstoff in Gegenwart eines Hydrierungskatalysators, wie eines Platin-, Palladium- oder Rhodium-Katalysators, z.B. von Platinoxid, in Betracht. Die Behandlung mit katalytisch aktiviertem Wasserstoff (Hydrierung) erfolgt unter normalen oder höchstens mässig erhöhten Druck- und/oder Temperaturbedingungen, z.B. unter etwa 0 bis 5 bar Überdruck und/oder im Temperaturbereich von etwa 20° bis etwa 80°C.

Ausgangsstoffe XXXIV, worin $X_{11}$ einen fluorierten Niederalk-1-enyl- oder 1-Hydroxyniederalkylrest, $R_{11}$ Wasserstoff ist, die Reste $X_1$ für veretherte Hydroxygruppen R stehen und $R_3$ von Halogen Hal verschieden ist, werden beispielsweise erhalten, indem man eine entsprechende Verbindung XXXIV, worin $X_{11}$ ein Halogen Hal ist, mit einem Metall $M^I$ oder $M^{II}$ behandelt, die erhaltene Verbindung XXXIV, worin $X_{11}$ eine Gruppe $-M^I$, $-M^{II}$ oder –MHal ist, mit einem fluorierten Oxoniederalkan umsetzt und zur Herstellung von Verbindungen XXXIV, worin $X_{11}$ einen fluorierten Niederalk-1-enylrest bedeutet, aus der primär erhaltenen fluorierten 1-Hydroxyalkylverbindung Wasser abspaltet, z.B. durch Acetylieren und anschliessende Säurebehandlung.

Ausgangsstoffe XXXIV, worin $X_{11}$ einen mono- oder difluorierten Niederalk-2-enylrest bedeutet und mindestens einer der Reste $X_1$ freies Hydroxy bedeutet, werden beispielsweise erhalten, indem man eine Verbindung der Formel

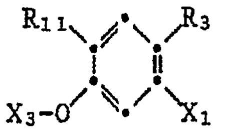

(XXXV)

der Allylumlagerung unterwirft, beispielsweise durch Erwärmen auf etwa 150° bis 250°C, vorzugsweise auf etwa 190° bis 220°C, vorteilhaft in einem Lösungsmittel, wie Diphenylether oder N,N-Dimethyl- oder N,N-Diethylanilin. Dabei wird auch der Rest $X_3$ umgelagert, so dass aus einem 1-Fluor- bzw. 1,1-Difluorniederalk-2-enylrest der entsprechende 3-Fluor- bzw. 3,3-Difluorniederalk-2-enylrest entsteht.

Verbindungen XXXIV, worin $X_{11}$ einen fluorierten Niederalk-1-inylrest bedeutet und $R_{11}$ Wasserstoff ist, werden beispielsweise erhalten, indem man in einer entsprechenden Verbindung XXXIV, worin $X_{11}$ einen fluorierten, an den doppeltgebundenen C-Atomen mindestens 2 Wasserstoffatome aufweisenden Niederalkenylrest bedeutet, an $X_{11}$ Halogen anlagert und in üblicher Weise 2 Mol Halogenwasserstoff abspaltet.

Verbindungen der Formel XXXIII, worin $R_2$ 3,3,3-Trifluorpropyl bedeutet und $R_3$ und $R_{11}$ die angegebenen Bedeutungen haben, oder ihre Salze werden gemäss einer insgesamt und/oder in wichtigen Einzelschritten neuartigen Verfahrensweise besonders leicht erhalten, indem man eine Verbindung der Formel

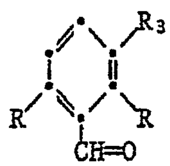

(XX),

worin R verethertes Hydroxy, wie Niederalkoxy mit bis und mit 4 C-Atomen, z.B. Methoxy, bedeutet, in einem inerten Lösungsmittel, z.B. in Dimethylformamid, mit einem Etherat von 1,1-Dichlor-2,2,2-trifluorethyl-zinkchlorid, z.B. der Formel $CF_3CCl_2ZnCl(C_2H_5)_2O$ (XXI), zu der entsprechenden Verbindung der Formel

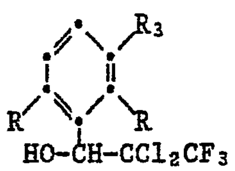

(XXII)

umsetzt, diese in der Seitenkette acyliert, z.B. acetyliert, beispielsweise mittels Acetanhydrid in Pyridin, das Reaktionsprodukt der Formel

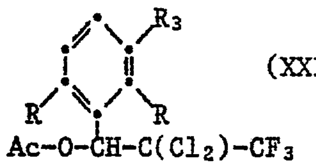

(XXIIa),

worin der Rest Ac für die eingeführte Acylgruppe, z.B. Acetyl, steht, durch Behandeln mit einem metallischen Reduktionsmittel, beispielsweise einem unedlen Metall, vorzugsweise mit aktiviertem Zinkstaub in Gegenwart von Ammoniumchlorid, in die entsprechende Verbindung der Formel

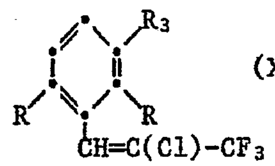

(XXIII)

überführt, aus dieser durch Behandeln mit einer Metallbase, wie einem Alkali- oder Erdalkalimetallalkoholat, z.B. mit Kaliumtertiärbutanolat, Chlorwasserstoff abspaltet, in der erhaltenen Verbindung der Formel

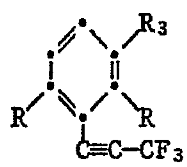

(XXIV)

die Seitenkette, z.B. in Gegenwart von Palladiumkohle, hydriert und die veretherten Hydroxygruppen R zu Hydroxy spaltet, beispielsweise durch Behandeln mit Bromwasserstoffsäure in Dichlormethan, gewünschtenfalls einen Rest $R_{11}$ der Formel $R_1$–C(=O)– einführt und gewünschtenfalls eine erhaltene freie Verbindung der Formel XXXIII in ein Salz oder ein erhaltenens Salz in die freie Verbindung der Formel XXXIII umwandelt.

Die ggf. nachträglich vorzunehmende Einführung eines Restes $R_{11}$ der Formel $R_1$–C(=O)– erfolgt beispielsweise wie unter der Verfahrensvariante a) oder b) angegeben, beispielsweise durch Umsetzung mit einer Verbindung der Formel $R_1$–$X_2$ (IV), worin $X_2$ ggf. funktionell abgewandeltes Carboxy, wie Carboxy, Halogencarbonyl der Formel –C(=O)–Hal oder anhydridisiertes Carboxy beispielsweise der Formel –C(=O)–O–C(=O)–$R_1$, in Gegenwart einer Lewissäure – wie unter der Verfahrensvariante b) definiert –, z.B. durch Umsetzung mit einer Säure der Formel (IV; $X_2$ = Carboxy) in Gegenwart von Zinkchlorid. Die gegenseitige Umwandlung erhaltener Säuren und Salze erfolgt ebenfalls wie für Verbindungen der Formel I angegeben.

Die vorliegende Erfindung betrifft ebenfalls pharmazeutische Präparate, welche eine der erfindungsgemässen Verbindungen der Formel I oder ein pharmazeutisch verwendbares Salz davon enthalten. Bei den erfindungsgemässen pharmazeutischen Präparaten handelt es sich um solche, die zur topischen und lokalen sowie enteralen, wie oralen oder rektalen, sowie parenteralen Verabreichung an und zur Inhalation durch Warmblüter bestimmt sind und den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab.

Die neuen pharmazeutischen Präparate enthalten z.B. von etwa 10% bis etwa 95%, vorzugsweise von etwa 20% bis etwa 90% des Wirkstoffs. Erfindungsgemässe pharmazeutische Präparate sind z.B. solche in Aerosol- oder Sprayform oder in Dosiseinheitsformen, wie Dragées, Tabletten, Kapseln oder Suppositorien, ferner Ampullen.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, z.B. Mais-, Weizen-, Reis- oder Kartoffelstärkekleister, Gelatine, Tragaganth, Methylcellulose und/oder Polyvinylpyrrolidon, und/oder, wenn erwünscht, Sprengmittel, wie die obengenannten Stärken, ferner Carboxymethylstärke, quervernetztes

Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Überzügen versehen, wobei man u.a. konzentrierte Zuckerlösungen, welche gegebenenfalls arabischen Gummi, Talk, Polyvinylpyrrolidone, Polyethylenglykol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Präparate Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet, oder wässrige Injektionssuspensionen, welche viskositätserhöhende Stoffe, z.B. Natrium-carboxymethylcellulose, Sorbit und/oder Dextran und gegebenenfalls auch Stabilisatoren, enthalten.

Inhalationspräparate für die Behandlung der Atemwege durch nasale oder buccale Verabreichung sind z.B. Aerosole oder Sprays, welche den pharmakologischen Wirkstoff in Form eines Puders oder in Form von Tropfen einer Lösung oder Suspension verteilen können. Präparate mit Puder-verteilenden Eigenschaften enthalten ausser dem Wirkstoff üblicherweise ein flüssiges Treibgas mit einem Siedepunkt unter der Raumtemperatur, sowie, wenn erwünscht, Trägerstoffe wie flüssige oder feste nicht-ionische oder anionische oberflächenaktive Mittel und/oder Verdünnungsmittel. Präparate, in welchen der pharmakologische Wirkstoff in Lösung vorliegt, enthalten ausser diesem ein geeignetes Treibmittel, ferner, falls notwendig, ein zusätzliches Lösungsmittel und/oder einen Stabilisator. Anstelle des Treibgases kann auch Druckluft verwendet werden, wobei diese mittels einer geeigneten Verdichtungs- und Entspannungsvorrichtung nach Bedarf erzeugt werden kann.

Pharmazeutische Präparate für topische und lokale Verwendung sind z.B. für die Behandlung der Haut Lotionen und Cremen, die eine flüssige oder semifeste Öl-in-Wasser- oder Wasser-in-Öl-Emulsion enthalten, und Salben (wobei solche vorzugsweise ein Konservierungsmittel enthalten), für die Behandlung der Augen Augentropfen, welche die aktive Verbindung in wässriger oder öliger Lösung enthalten und Augensalben, die vorzugsweise in steriler Form hergestellt werden, für die Behandlung der Nase Puder, Aerosole und Sprays (ähnlich den oben beschriebenen für die Behandlung der Atemwege), sowie grobe Puder, die durch schnelles Inhalieren durch die Nasenlöcher verabreicht werden, und Nasentropfen, welche die aktive Verbindung in wässriger oder öliger Lösung enthalten, oder für die lokale Behandlung des Mundes Lutschbonbons, welche die aktive Verbindung in einer im allgemeinen aus Zucker und Gummiarabikum oder Tragaganth gebildeten Masse enthalten, welcher Geschmacksstoffe beigegeben sein können, sowie Pastillen, die den Aktivstoff in einer inerten Masse, z.B. aus Gelatine und Glycerin oder Zucker und Gummiarabikum, enthalten.

Die Erfindung betrifft ebenfalls die Verwendung der neuen Verbindungen der Formel I und ihrer Salze als pharmakologisch aktive Verbindungen, beispielsweise als Antiallergika oder insbesondere Antiinflammatorika vorzugsweise in der Form von pharmazeutischen Präparaten. Die tägliche Dosis, die einem Warmblüter von etwa 70 kg verabreicht wird, beträgt von etwa 200 mg bis etwa 1200 mg.

Die nachfolgenden Beispiele illustrieren die oben beschriebene Erfindung. Temperaturen sind in Celsiusgraden angegeben.

Beispiel 1: Eine Lösung von 3 g (4,5 mMol) N-{{3-{3-[4-Acetyl-3-hydroxy-2-(3,3,3-trifluorpropyl)-phenoxy]-propyloxy}-4-chlor-6-methyl-phenyl}}1-(4-methoxybenzyl)-tetrazol-5-carboxamid in 90 ml Trifluoressigsäure und 9 ml Anisol wird 60 Minuten zum Rückfluss erhitzt. Das Reaktionsgemisch wird unter vermindertem Druck eingeengt, mit etwa 150 ml Ether sowie 300 ml Petrolether versetzt und die Kristalle abfiltriert. Das so erhaltene N-{{3-{3-[4-Acetyl-3-hydroxy-2-(3,3,3-trifluorpropyl)-phenoxy]-propoxy}-4-chlor-6-methyl-phenyl}}-1H-tetrazol-5-carboxamid vom Smp. 236–238° (Zersetzung) wird in 50 ml Tetrahydrofuran gelöst und mit einem Äquivalent Natrium, gelöst in 5 ml Methanol versetzt.

Nach Zugabe von Ether setzt Kristallisation ein. Man erhält so das Natriumsalz von N-{{3-{3-[4-Acetyl-3-hydroxy-2-(3,3,3-trifluorpropyl)-phenoxy]-propyloxy}-4-chlor-6-methyl-phenyl}}-1H-tetrazol-5-carboxamid vom Smp. 90° (Zersetzung).

In analoger Weise erhält man, ausgehend von N-{{3-{3-[4-Acetyl-3-hydroxy-2-(3,3,3-trifluorpropyl)-phenoxy]-propyloxy}-4-brom-6-methyl-phenyl}}-1-(4-methoxybenzyl)-tetrazol-5-carboxamid, das N-{{3-{3-[4-Acetyl-3-hydroxy-2-(3,3,3-trifluorpropyl)-phenoxy]-propyloxy}-4-brom-6-methylphenyl}}-1H-tetrazol-5-carboxamid vom Smp. 245–248° (Zersetzung) und dessen Natriumsalz vom Smp. 120–130°C.

Das Ausgangsmaterial kann z.B. folgendermassen hergestellt werden:

Stufe A: 1,1-Dichlor-2,2,2-trifluor-ethylzinkchlorid-diethyletherat, $CF_3CCl_2ZnCl(C_2H_5)O$

In einem 1 l Dreihalsrundkolben werden unter Argon 65,4 g (1 Mol) Zinkstaub (aktiviert nach Fieser & Fieser) in 800 ml Diethylether suspendiert und langsam mit 188 g (1 Mol) $CF_3CCl_3$ versetzt. Man lässt 20 Stunden bei Raumtemperatur nachrühren. Anschliessend wird die Reaktionsmischung über Filterflockenmasse "Selecta" filtriert, wobei nach Erkalten oder Kühlen des Filtrats der Komplex in kristalliner Form ausfällt. Der Komplex wird zur weiteren Reinigung aus Diethylether umkristallisiert. Der überschüssige Diethylether wird abdekantiert und der farblos kristalline Rückstand im Vakuum getrocknet. Man erhält 1,1-Dichlor-2,2,2-trifluor-ethylzinkchlorid-diethyletherat vom Smp. 105°, Ausbeute 80%.

Stufe B: 1,3-Dimethoxy-2-(1-hydroxy-2,2-dichlor-3,3,3-trifluor-propyl)-benzol

In einem Dreihalsrundkolben (1000 ml) mit Thermometer, Blasenzähler und Argon-Anschluss werden nach 3maligem Vakuum/Argon-Spülen 130,1 g (0,40 Mol) 1,1-Dichlor-2,2,2-trifluor-ethylzinkchlorid-diethyletherat in 700 ml absolutem Dimethylformamid vorgelegt. Anschliessend werden 51,0 g (0,32 Mol) 2,6-Dimethoxybenzaldehyd auf einmal zugegeben. Die gelbe, klare Reaktionslösung wird über 72 Stunden bei Raumtemperatur gerührt und dann auf eine Mischung von 600 g Eis und 600 ml 10%ige Salzsäure gegossen. Nach Ausethern, Waschen mit 2%iger Salzsäure, Sole, Trocknen über Magnesiumsulfat und Einengen erhält man ein dunkelrotes Öl, aus welchem rotbraune Kristalle von 1,3-Dimethoxy-2-(1-hydroxy-2,2-dichlor-3,3,3-trifluor-propyl)-benzol ausfallen, Ausbeute 99%. Dieses braucht für die Weiterarbeit nicht nachgereinigt zu werden.

Stufe C: 1,3-Dimethoxy-2-(1-acetoxy-2,2-dichlor-3,3,3-trifluorpropyl)-benzol

In einem 500 ml Birnenkolben werden 97,6 g (0,30 Mol) 1,3-Dimethoxy-2-(1-hydroxy-2,2-dichlor-3,3,3-trifluor-propyl)-benzol bei Raumtemperatur mit 31 ml (0,33 Mol) Essigsäureanhydrid versetzt. Anschliessend werden zur dunkelroten Lösung 30 ml (0,36 Mol) Pyridin zugegeben (schwach exotherm). Die Reaktionslösung wird 3 Tage bei Raumtemperatur gerührt. Dann wird das Reaktionsgemisch auf Eis gegossen und mit 10%iger Salzsäure angesäuert. Es wird mit Ether extrahiert, mit Wasser und Sole gewaschen, über Magnesiumsulfat getrocknet und zur Trockne eingedampft. Das zurückbleibende 1,3-Dimethoxy-2-(1-acetoxy-2,2-dichlor-3,3,3-trifluorpropyl)-benzol (Ausbeute 87%) wird ohne weitere Reinigung für Stufe D eingesetzt.

Stufe D: 1,3-Dimethoxy-2-(2-chlor-3,3,3-trifluorprop-1-enyl)-benzol

In einem 1,5 l Sulfierkolben mit Thermometer, Kühler und Blasenzähler werden 96,5 g (0,266 Mol) 1,3-Dimethoxy-2-(1-hydroxy-2,2-dichlor-3,3,3-trifluor-propyl)-benzol in 1000 ml Ethanol gelöst und mit 30 g (0,56 Mol) Ammonchlorid versetzt. Zur erhaltenen bräunlichen Suspension wird portionsweise das nach Fieser & Fieser aktivierte Zink (36,9 g = 0,564 Mol) zugegeben, wobei die Reaktionstemperatur bis auf 42° ansteigt. Es wird über Nacht weitergerührt und dann das grau-gelbe Reaktionsgemisch auf Eiswasser gegossen, mit Ether extrahiert, mit Wasser (3x) und Sole gewaschen, über Magnesiumsulfat getrocknet und eingedampft. Man erhält 1,3-Dimethoxy-2-(2-chlor-3,3,3-trifluorprop-1-enyl)-benzol (Ausbeute: 94%) als rötliches Öl.

Zinkstaub wird wie folgt aktiviert: 2x Aufschlämmen in 5% HCl und dekantieren (exotherm), 3x Aufschlämmen mit $H_2O$ und dekantieren, 2x Aufschlämmen mit Methanol und Dekantieren, 3x Aufschlämmen mit Ether und Dekantieren; Trocknen unter stark vermindertem Druck und Aufbewahren unter Argon.

Stufe E: 1,3-Dimethoxy-2-(3,3,3-trifluorprop-1-inyl)-benzol

In einem 1,5 l Sulfierkolben mit Thermometer, Kühler, Blasenzähler und Tropftrichter werden 30,7 g Kaliumtertiärbutanolat in 900 ml Tertiärbutanol vorgelegt. Zu dieser klaren, farblosen Lösung werden 63,7 g (0,25 Mol) 1,3-Dimethoxy-2-(2-chlor-3,3,3-trifluorprop-1-enyl)-benzol in 100 ml Tertiärbutanol zugetropft (schwach exotherm) und 24 Stunden bei Raumtemperatur ausgerührt. Die orange Suspension wird auf Eis gegossen, mit Ether extrahiert, mit Wasser, n-Bicarbonatlösung und Sole gewaschen, über

Magnesiumsulfat getrocknet und eingeengt, bis Kristalle ausfallen. Die Kristallmasse wird gekühlt, in der Kälte filtriert und mit wenig eiskaltem Petrolether nachgewaschen. Man erhält das 1,3-Dimethoxy-2-(3,3,3-trifluorprop-1-inyl)-benzol in Form von hellbeigen, feinen Kristallen vom Smp 110–111°, (Ausbeute 73%).

Stufe F: 1,3-Dimethoxy-2-(3,3,3-trifluorpropyl)-benzol

41,5 g (0,18 Mol) 1,3-Dimethoxy-2-(3,3,3-trifluorprop-1-inyl)-benzol werden in Tetrahydrofuran gelöst und in Gegenwart von Palladium auf Kohle bei Raumtemperatur und Normaldruck hydriert. Der Katalysator wird über Diatomerenerde abfiltriert, mit Tetrahydrofuran nachgewaschen und das Filtrat eingeengt. Nach Trocknen unter vermindertem Druck erhält man kristallines 1,3-Dimethoxy-2-(3,3,3-trifluorpropyl)-benzol (Ausbeute 87%).

Stufe G: 2-(3,3,3-Trifluorpropyl)-resorcin

In einem 1,5 l Sulfierkolben mit Thermometer, Blasenzähler, Tropftrichter mit Kühlmantel, Argonanschluss, werden 30,5 g (0,13 Mol) 1,3-Dimethoxy-2-(3,3,3-trifluorpropyl)-benzol in 275 ml Dichlormethan vorgelegt und auf –75° gekühlt. Nun werden 340 ml einer vorgekühlten 1-molaren Lösung von Bortribromid in Dichlormethan so zugetropft, dass die Reaktionstemperatur –70° nicht übersteigt (ca. 90 Min.). Nach 6 Stunden Nachrühren bei Raumtemperatur wird die rote Reaktionslösung auf 800 ml Eiswasser gegossen. Die organische Phase wird abgetrennt, die Wasserphase nochmals ausgeethert, und die vereinigten organischen Phasen mit Sole gewaschen, über Magnesiumsulfat getrocknet und eingedampft. Man erhält 2-(3,3,3-trifluorpropyl)-resorcin in Form roter Kristalle vom Smp. 82–84° (Ausbeute 100%).

Stufe H: 2,4-Dihydroxy-3-(3,3,3-trifluorpropyl)-acetophenon

In einem 100 ml Dreihalsrundkolben mit Kühler, Blasenzähler, Thermometer werden 21,0 g (0,154 Mol) Zinkchlorid in 27 ml Eisessig vorgelegt und auf 120° erhitzt, wobei eine klare, farblose Lösung entsteht. Bei dieser Temperatur werden 27,0 g (0,13 Mol) 2-(3,3,3-Trifluorpropyl)-resorcin zugegeben, wobei die Temperatur auf 82° absinkt. Die rote, klare Lösung wird noch 4 Stunden zum Rückfluss erhitzt (132°) und erkalten gelassen. Man erhält eine rote Suspension, diese wird auf 440 ml halbkonzentrierte Salzsäure gegossen und 3x mit Ether extrahiert. Die organische Phase wird mit Sole gewaschen, über Magnesiumsulfat getrocknet und stark eingeengt. Die erhaltenen rötlichen Kristalle werden in Pentan aufgeschlammt, abgenutscht und mit Pentan nachgewaschen. Man erhält 2,4-Dihydroxy-3-(3,3,3-trifluorpropyl)-acetophenon, Ausbeute: 26,5 g gelbe Kristalle vom Smp. 180–184°; 82%

Stufe I: 4-(3-Brompropyloxy)-2-hydroxy-3-(3,3,3-trifluorpropyl)-acetophenon

Zu einer Lösung von 2,45 ml 1,3-Dibrompropan in 30 ml Aceton gibt man 3,32 g Kaliumcarbonat und 0,5 g Kaliumjodid und erhitzt zum Rückfluss.
Innert 2 Stunden tropft man die Lösung von 1,98 g 2,4-Dihydroxy-3-(3,3,3-trifluorpropyl)-acetophenon in 10 ml Aceton zu und erhitzt noch weitere 3 Stunden zum Rückfluss. Das Reaktionsgemisch wird filtriert und im Vakuum eingedampft. Der Rückstand wird in 50 ml Dichlormethan gelöst und mit 10 ml Wasser gewaschen. Die organische Phase wird über Natriumsulfat getrocknet, im Vakuum eingedampft und der Rückstand mit an 100 g Kieselgel mit Dichlormethan als Laufmittel chromatographiert. In der ersten Fraktion eluiert man 4-(3-Brompropyloxy)-2-hydroxy-3-(3,3,3-trifluorpropyl)-acetophenon, das nach Eindampfen in Form farbloser Kristalle vom Smp. 70–72°C anfällt.

Stufe J: 3-{3-[4-Acetyl-3-hydroxy-2-(3,3,3-trifluorpropyl)-phenoxy]propyloxy}-4-chlor bzw. brom-6-methyl-nitrobenzol

Eine Suspension von 1,87 g (10 mMol) 2-Chlor-4-methyl-5-nitro-phenol und 1,5 g (10,5 mMol) geglühten Kaliumcarbonates in 30 ml Ethylmethylketon wird mit einer Spatelspitze Kaliumjodid und 3,5 g (9,5 mMol) 4-(3-Brompropyloxy)-2-hydroxy-3-(3,3,3-trifluorpropyl)-acetophenon versetzt und 10 Stunden zum Rückfluss erhitzt. Das Reaktionsgemisch wird abgekühlt, auf Wasser gegossen und dreimal mit Dichlormethan extrahiert. Die organischen Phasen werden vereinigt, mit Wasser gewaschen, über Natriumsulfat getrocknet und unter vermindertem Druck zur Trockene eingeengt. Kristallisation des Rückstandes aus Ether/Petrolether liefert das 3-{3[4-Acetyl-3-hydroxy-2-(3,3,3-trifluorpropyl)-phenoxy]-propyloxy}-4chlor-6-methyl-nitrobenzol vom Smp. 118–120°.
In analoger Weise erhält man ausgehend von 2-Brom-4-methyl-5-nitrophenol das 3-{3-[4-Acetyl-3-hydroxy-2-(3,3,3-trifluorpropyl)-phenoxy]-propyloxy}-4-brom-6-methyl-nitrobenzol vom Smp. 106–109°.

Stufe K: 3-{3-[4-Acetyl-3-hydroxy-2-(3,3,3-trifluorpropyl)-phenoxy]-propyloxy}-4-chlor bzw. brom-6-methyl-anilin

Eine Lösung von 3,7 g 3-[3-(4-Acetyl-3-hydroxy-2-(3,3,3-trifluorpropyl)-phenoxy)-propyloxy]-4-chlor-6-methyl-nitrobenzol in 40 ml Tetrahydrofuran wird mit 1,0 g Raney-Nickel versetzt und bei Raumtemperatur hydriert. Der Katalysator wird abfiltriert und mit Tetrahydrofuran nachgewaschen. Das Filtrat wird unter vermindertem Druck zur Trockne eingedampft und der Rückstand aus Ether/Petrolether kristallisiert. Man erhält so das 3-{3-[4-Acetyl-3-hydroxy-2-(3,3,3-tri-fluorpropyl)-phenoxy]-propyloxy}-4-chlor-6-methyl-anilin vom Smp. 111–113°.

In analoger Weise erhält man ausgehend von 3-{3-[4-Acetyl-3-hydroxy-2-(3,3,3-trifluorpropyl)-phenoxy]-propyloxy}-4-brom-6-methyl-nitrobenzol das 3-{3-[4-Acetyl-3-hydroxy-2-(3,3,3-trifluorpropyl)-phenoxy]-propyloxy}-4-brom-6-methyl-anilin vom Smp. 124–126°.

Stufe L: N-{{3-{3-[4-Acetyl-3-hydroxy-2-(3,3,3-trifluorpropyl)-phenoxy]-propyloxy}-4-chlor bzw. brom-6-methyl-phenyl}}-1-(p-methoxy-benzyl)-tetrazol-5-carboxamid

Zu einer Suspension von 2,07 g (7,7 mMol) Kalium [1-(4-methoxybenzyl)-tetrazol]-5-carboxylat in 30 ml Benzol und 0,5 ml Pyridin gibt man bei 0–5° 0,66 ml (7,7 mMol) Oxalylchlorid und lässt 30 Minuten bei Raumtemperatur rühren. Das Reaktionsgemisch wird unter vermindertem Druck eingeengt, der Rückstand in Benzol aufgenommen und erneut unter vermindertem Druck eingedampft. Der Rückstand wird in 200 ml Dichlormethan suspendiert und bei 0–5° innerhalb von 10 Minuten zu einer Lösung von 2,6 g (5,8 mMol) 3-{3-[4-Acetyl-3-hydroxy-2-(3,3,3-trifluorpropyl)-phenoxy]-propyloxy}-4-chlor-6-methyl-anilin und 0,61 ml Pyridin in 30 ml Dichlormethan zugetropft. Anschliessend wird 3 Stunden bei Raumtemperatur gerührt. Das Reaktionsgemisch wird mit Dichlormethan verdünnt und zweimal mit Wasser gewaschen. Die organische Phase wird über Natriumsulfat getrocknet und unter vermindertem Druck eingedampft. Kristallisation des Rückstandes aus Ether liefert N-{{3-{3-[4-Acetyl-3-hydroxy-2-(3,3,3-trifluorpropyl)-phenoxy]-propyloxy}-4-chlor-6-methyl-phenyl}}1-(4-methoxybenzyl)-tetrazol-5-carboxamid vom Smp. 148–150°.

In analoger Weise erhält man ausgehend von 3-{3-[4-Acetyl-3-hydroxy-2-(3,3,3-trifluorpropyl)-phenoxy]-propyloxy}-4-brom-6-methyl-anilin das N-{{3-{3-[4-Acetyl-3-hydroxy-2-(3,3,3-trifluorpropyl)-phenoxy]-propyloxy}-4-brom-6-methyl-phenyl}}-1-(4-methoxy-benzyl)-tetrazol-5-carboxamid vom Smp. 140–142°C.

Beispiel 2: Zu einer auf 0° gekühlten Lösung von 1,75 g (3,57 mMol) 3-{3-[4-Acetyl-3-hydroxy-2-(3,3,3-trifluorpropyl)-phenoxy]-propyloxy}-4-brom-6-methyl-anilin (Beispiel 1) und 0,55 ml (3,57 mMol) Triethylamin in 15 ml Dichlormethan wird innerhalb von 5 Minuten eine Lösung von 0,36 ml (3,57 mMol) Chloroxalsäuremethylester in 5 ml Dichlormethan zugetropft. Man lässt 90 Minuten bei Raumtemperatur nachrühren, giesst das Reaktionsgemisch auf Eiswasser und trennt die organische Phase ab.

Die organische Phase wird mit Wasser gewaschen, über Natriumsulfat getrocknet und unter vermindertem Druck eingeengt. Kristallisation des Rückstandes aus Dichlormethan/Ether ergibt den N-{{3-{3-[4-Acetyl-3hydroxy-2-(3,3,3-trifluorpropyl)-phenoxy]-propyloxy}-4-brom-6-methyl-phenyl}}-oxaminsäuremethylester vom Smp. 139–140°.

Beispiel 3: Eine Suspension von 1,75 g (3 mMol) N-{{3-{3-[4-Acetyl-3-hydroxy-2-(3,3,3-trifluorpropyl)-phenoxy]-propyloxy}-4-brom-6-methyl-phenyl}}-oxaminsäuremethylester (Beispiel 2) in 30 ml Methanol und 3,2 ml n-Natronlauge wird 60 Minuten zum Rückfluss erhitzt. Das Reaktionsgemisch wird unter vermindertem Druck eingeengt, in Aceton und verdünnter Natronlauge gelöst und mit verdünnter Salzsäure angesäuert. Das ausgefallene Produkt wird abfiltriert und mit Wasser neutral gewaschen. Man erhält so die N-{{3-{3-[4-Acetyl-3-hydroxy-2-(3,3,3-trifluorpropyl)-phenoxy]-propyloxy}-4-brom-6-methyl-phenyl}}-oxaminsäure vom Smp. 207–209°.

Beispiel 4: 1,55 g (2,76 mMol) N-{{3-{3-[4-Acetyl-3-hydroxy-2-(3,3,3-trifluorpropyl)-phenoxy]-propyloxy}-4-brom-6-methyl-phenyl}}-oxaminsäure (Beispiel 3) werden in 300 ml Aceton heiss gelöst und mit einer Lösung von 411 mg (2,8 mMol) Triethanolamin in 10 ml Aceton versetzt. Die Reaktionslösung wird unter vermindertem Druck auf 50 ml aufkonzentriert.

Nach Zugabe von Ether beginnt die Kristallisation. Das ausgefallene Produkt wird abfiltriert und mit Ether gewaschen. Man erhält so das Triethanolammoniumsalz der N-{{3-{3-[4-Acetyl-3-hydroxy-2-(3,3,3-trifluorpropyl)-phenoxy]-propyloxy}-4-brom-6-methyl-phenyl}}-oxaminsäure; Smp. 133–135°.

Beispiel 5: Zu einer auf 0° gekühlten Lösung von 7,5 g 3-{3-[4-Acetyl-3-hydroxy-2-(3,3,3-trifluorpropyl)-phenoxy]-propyloxy}-6-methyl-anilin und 3,0 g Triethylamin in 60 ml Dichlormethan wird innerhalb von etwa 5 Minuten eine Lösung von 1,9 ml (21 mMol) Chloroxalsäuremethylester in 8 ml Dichlormethan zugetropft. Man lässt 90 Minuten bei Raumtemperatur nachrühren, giesst das Reaktionsgemisch auf Eiswasser und trennt die organische Phase ab. Die Dichlormethan-Phase wird mit Wasser gewaschen, über Natriumsulfat getrocknet und unter vermindertem Druck eingeengt. Kristallisation des Rückstandes aus Dichlormethan/Ether ergibt den N-{{3-{3-[4-Acetyl-3-hydroxy-2-(3,3,3-trifluorpropyl)-phenoxy]-propyloxy}-6-methyl-phenyl}}-oxaminsäuremethylester.

In analoger Weise erhält man ausgehend von 3-{3-[4-Acetyl-3-hydroxy-2-(3,3,3-trifluorpropyl)-phenoxy]-propyloxy}-anilin N-{{3-{3-[4-Acetyl-3-hydroxy-2-(3,3,3-trifluorpropyl)-phenoxy]-propyloxy}-phenyl}}-oxaminsäuremethylester.

Das Ausgangsmaterial kann man z.B. folgendermassen herstellen.

Eine Suspension von 5,1 g 4-Methyl-3-nitro-phenol und 4,6 g geglühten Kaliumcarbonates in 100 ml Ethylmethylketon wird mit einer Spatelspitze Kaliumjodid und 9,5 g 4-(3-Brompropoxy)-2-hydroxy-3-(3,3,3-trifluorpropyl)-acetophenon versetzt und 14 Stunden zum Rückfluss erhitzt. Das Reaktionsgemisch wird abgekühlt, auf Wasser gegossen und dreimal mit Ether extrahiert. Die organischen Phasen werden mit Wasser gewaschen, vereinigt, über Natriumsulfat getrocknet und unter vermindertem Druck zur Trockne eingeengt. Kristallisation des Rückstandes liefert das 3-{3-[4-Acetyl-3-hydroxy-2-(3,3,3-trifluorpropyl)-phenoxy]-propyloxy}-6-methyl-nitrobenzol.

In analoger Weise erhält man ausgehend von m-Nitrophenol 3-{3-[4-Acetyl-3-hydroxy-2-(3,3,3-trifluorpropyl)-phenoxy]-propyloxy}-nitrobenzol.

Eine Lösung von 9,1 g (23,5 mMol) 3-[3-(4-Acetyl-3-hydroxy-2-n-propylphenoxy)-propyloxy]-6-methyl-nitrobenzol in 90 ml Tetrahydrofuran wird mit 1,0 g Raney-Nickel versetzt und bei Raumtemperatur hydriert. Der Katalysator wird abfiltriert und mit Tetrahydrofuran gewaschen. Das Filtrat wird unter vermindertem Druck zur Trockne eingedampft. Man erhält so das 3-{3-[4-Acetyl-3-hydroxy-2-(3,3,3-trifluorpropyl)-phenoxy]-propyloxy}-6-methyl-anilin.

In analoger Weise erhält man ausgehend von 3-{3-[4-Acetyl-3-hydroxy-2-(3,3,3-trifluorpropyl)-phenoxy]-propyloxy}-nitrobenzol 3-{3-[4-Acetyl-3-hydroxy-2-(3,3,3-trifluorpropyl)-phenoxy]-propyloxy}-anilin (Ether/Petrolether).

Beispiel 6: Eine Suspension von 8,60 g (19,4 mMol) N-{{3-{3-[4-Acetyl-3-hydroxy-2-(3,3,3-trifluorpropyl)-phenoxy]-propyloxy}-6-methyl-phenyl}}-oxaminsäuremethylester in 60 ml Methanol und 20 ml Wasser wird mit 20 ml 1n-Natronlauge versetzt und 10 Minuten zum Rückfluss erhitzt. Das Reaktionsgemisch wird abgekühlt und das ausgefallene Produkt abfiltriert. Man erhält so das Natriumsalz der N-{{3-{3-[4-Acetyl-3-hydroxy-2-(3,3,3-trifluorpropyl)-phenoxy]-propyloxy}-6-methyl-phenyl}}-oxaminsäure.

In analoger Weise kann das Natriumsalz der N-{{3-{3-[4-Acetyl-3-hydroxy-2-(3,3,3-trifluorpropyl)-phenoxy]-propyloxy}-phenyl}}-oxaminsäure hergestellt werden.

Beispiel 7: In analoger Weise wie in Beispiel 2 beschrieben erhält man:

N-{{3-{3-[4-Acetyl-3-hydroxy-2-(3,3,3-trifluorpropyl)-phenoxy]-propyloxy}-2-methyl-phenyl}}-oxaminsäuremethylester;

N-{{3-{5-[4-Acetyl-3-hydroxy-2-(3,3,3-trifluorpropyl)-phenoxy]-pentyloxy}-phenyl}}-oxaminsäuremethylester;

N-{{4-{3-[4-Acetyl-3-hydroxy-2-(3,3,3-trifluorpropyl)-phenoxy]-propyloxy}-3-methoxy-phenyl}}-oxaminsäuremethylester;

N-{{4-{3-[4-Acetyl-3-hydroxy-2-(3,3,3-trifluorpropyl)-phenoxy]-propyloxy}-2-methyl-phenyl}}-oxaminsäuremethylester;

N-{{2-{3-[4-Acetyl-3-hydroxy-2-(3,3,3-trifluorpropyl)-phenoxy]-1-propyloxy}-phenyl}}-oxaminsäuremethylester;

N-{{4-{3-[4-Acetyl-3-hydroxy-2-(3,3,3-trifluorpropyl)-phenoxy]-propyloxy}-phenyl}}-oxaminsäuremethylester;

N-{{3-{3-[4-Acetyl-3-hydroxy-2-(3,3,3-trifluorpropyl)-phenoxy]-propyloxy}-4-chlor-6-trifluormethyl-phenyl}}-oxaminsäuremethylester;

N-{{3-{3-[4-Acetyl-3-hydroxy-2-(3,3,3-trifluorpropyl)-phenoxy]-propyloxy}-2,4,6-trichlorphenyl}}-oxaminsäuremethylester als Öl und

N-{{3-{3-[4-Acetyl-3-hydroxy-2-(3,3,3-trifluorpropyl)-phenoxy]-propyloxy}-4,6-dimethyl-phenyl}}-oxaminsäuremethylester .

Die Ausgangsmaterialien können z.B. in analoger Weise wie in Beispiel 1 beschrieben hergestellt werden.

Beispiel 8: In analoger Weise wie in Beispiel 3 beschrieben erhält man N-{{3-{3-[4-Acetyl-3-hydroxy-2-(3,3,3-trifluorpropyl)-phenoxy]-propyloxy}-2-methyl-phenyl}}-oxaminsäure und deren Triethanolammoniumsalz sowie N-{{3-{5-[4-Acetyl-3-hydroxy-2-(3,3,3-trifluorpropyl)-phenoxy]-pen-tyloxy}-phenyl}}-oxaminsäure sowie deren Triethanolammoniumsalz;

N-{{3-{3-[4-Acetyl-3-hydroxy-2-(3,3,3-trifluorpropyl)-phenoxy]-propyloxy}-4-chlor-6-trifluormethyl-phenyl}}-oxaminsäure und deren Triethanolammoniumsalz;

N-{{3-{3-[4-Acetyl-3-hydroxy-2-(3,3,3-trifluorpropyl)-phenoxy]-propyloxy}-2,4,6-trichlor-phenyl}}-oxaminsäure und deren Triethanolammoniumsalz und

N-{{3-{3-[4-Acetyl-3-hydroxy-3-(3,3,3-trifluorpropyl)-phenoxy]-propyloxy}-4,6-dimethyl-phenyl}}-oxaminsäure und deren Triethanolammoniumsalz.

Beispiel 9: Eine Lösung von 7,95 g N-{{4-{3-[4-Acetyl-3-hydroxy-2-(3,3,3-trifluorpropyl)-phenoxy]-propyloxy}-3-methoxy-phenyl}}-oxaminsäuremethylester in 100 ml Methanol wird mit 18 ml einer n-Natronlauge versetzt und 1 Stunde zum Rückfluss erhitzt. Die heisse Lösung wird anschliessend in 200 ml 0,1n-Salzsäure gegossen. Das ausgefallene Produkt wird abfiltriert, mit Wasser gewaschen und im Trockenschrank über Phosphorpentoxid getrocknet. Die so erhaltene N-{{4-{3-[4-Acetyl-3-hydroxy-2-(3,3,3-trifluorpropyl)-phenoxy]-propyloxy}-3-methoxy-phenyl}}-oxaminsäure wird erneut in 100 ml Methanol heiss gelöst und mit einem Äquivalent Triethanolamin in 10 ml Methanol versetzt. Nach Zugabe von 400 ml Ether setzt Kristallisation ein. Man erhält so das Triethanolammoniumsalz der N-{{4-{3-[4-Acetyl-3-hydroxy-2-(3,3,3-trifluorpropyl)-phenoxy]-propyloxy}-3-methoxy-phenyl}}-oxaminsäure.

In analoger Weise erhält man:

N-{{4-{3-[4-Acetyl-3-hydroxy-2-(3,3,3-trifluorpropyl)-phenoxy]-propyloxy}-2-methyl-phenyl}}-oxaminsäure und deren Triethanolammoniumsalz;

N-{{2-{3-[4-Acetyl-3-hydroxy-2-(3,3,3-trifluorpropyl)-phenoxy]-propyloxy}-phenyl}}-oxaminsäure und deren Triethanolammoniumsalz;

N-{{4-{3-[4-Acetyl-3-hydroxy-2-(3,3,3-trifluorpropyl)-phenoxy]-propyloxy}-phenyl}}-oxaminsäure und deren Triethanolammoniumsalz.

Beispiel 10: In analoger Weise wie in Beispiel 1 beschrieben erhält man:

N-{{3-{3-[4-Acetyl-3-hydroxy-2-(3,3,3-trifluorpropyl)-phenoxy)-propyloxy}-phenyl}}-1H-tetrazol-5-carboxamid und dessen Triethanolammoniumsalz.

Beispiel 11: Zu einer auf 0°C gekühlten Lösung von 43,6 g 3-{3-[4-Acetyl-3-hydroxy-(3,3,3-trifluorpropyl)-phenoxy]-propyloxy}-4-brom-6-methyl-anilin und 15,3 ml Triethylamin in 400 ml Dichlormethan wird innerhalb von etwa 5 Minuten eine Lösung von 12,3 ml Chloroxalsäureethylester in 30 ml Dichlormethan zugetropft und anschliessend noch 90 Minuten bei Raumtemperatur gerührt. Das Reaktionsgemisch wird auf Eiswasser gegossen und die organische Phase abgetrennt, mit Wasser gewaschen, über Natriumsulfat getrocknet und unter vermindertem Druck eingeengt. Kristallisation aus Essigester/Ether/Petrolether liefert den N-{{3-{3-[4-Acetyl-3-hydroxy-2-(3,3,3-trifluorpropyl)-phenoxy]-propyloxy}-4-brom-6-methyl-phenyl}}-oxaminsäureethylester.

Beispiel 12: Eine Suspension von 4,0 g N-{{3-{3-[4-Acetyl-3-hydroxy-2-(3,3,3-trifluorpropyl)-phenoxy]-propyloxy}-4-brom-6-methyl-phenyl}}-oxaminsäureethylester in 400 ml Ethanol werden mit 448 mg (7,5 mMol) Kaliumhydroxid versetzt und 4 Stunden am Rückfluss erhitzt. Das Reaktionsgemisch wird abgekühlt und das ausgefallene Produkt abfiltriert. Man erhält so das Kaliumsalz der N-{{3-{3-[4-Acetyl-3-hydroxy-2-(3,3,3-trifluorpropyl)-phenoxy]-propyloxy}-4-brom-6-methyl-phenyl}}-oxaminsäure.

In analoger Weise kann man auch das Natriumsalz der N-{{3-{3-[4-Acetyl-3-hydroxy-2-(3,3,3-trifluorpropyl)-phenoxy]-propyloxy}-4-brom-6-methyl-phenyl}}-oxaminsäure herstellen.

Beispiel 13: 4,06 g N-{{3-[3-(4-Acetyl-3-hydroxy-2-(3,3,3-trifluorpropyl)-phenoxy]-propyloxy}-4-brom-6-methyl-phenyl}}-oxaminsäure werden in 40 ml Aceton gelöst und mit einer Lösung von 840 mg Diethanolamin in 5 ml Aceton versetzt. Nach Zugabe von Ether beginnt die Kristallisation. Das ausgefallene Produkt wird abfiltriert und mit Ether gewaschen. Man erhält so das Diethanolammoniumsalz der N-{{3-{3-[4-Acetyl-3-hydroxy-2-(3,3,-trifluorpropyl)-phenoxy]-propyloxy}-4-brom-6-methyl-phenyl}}-oxaminsäure.

In analoger Weise kann man auch das Tris(hydroxymethyl)-methylammoniumsalz der N-{{3-{3-[4-Acetyl-3-hydroxy-2-n-(3,3,3-trifluorpropyl)phenoxy]-propylox}}-4-brom-6-methyl-phenyl}}-oxaminsäure herstellen.

Beispiel 14: Eine Lösung von 7,05 g N-{{3-{3-[4-Acetyl-3-hydroxy-2-(3,3,3-trifluorpropyl)-phenoxy]-propyloxy}-2-cyano-phenyl}}-1-(4-methoxybenzyl)-tetrazol-5-carboxamid in 150 ml Trifluoressigsäure und 15 ml Anisol wird 30 Minuten zum Rückfluss erhitzt. Das Reaktionsgemisch wird unter vermindertem Druck eingeengt, mit etwa 200 ml Ether sowie 300 ml Petrolether versetzt und die Kristalle abfiltriert. Das so erhaltene N-{{3-{3-[4-Acetyl-3-hydroxy-2-(3,3,3-trifluorpropyl)-phenoxy]-propyloxy}-2-cyano-phenyl}}-1H-tetrazol-5-carboxamid vom Smp. 206–208° wird in 50 ml Aceton heiss gelöst und mit der berechneten Menge Triethanolamin in 30 ml Aceton versetzt. Nach Zugabe von Ether setzt Kristallisation ein.

Man erhält so das Triethanolammoniumsalz von N-{{3-{3-[4-Acetyl-3-hydroxy-2-(3,3,3-trifluorpropyl)-phenoxy]-propyloxy}-2-cyano-phenyl}}1H-tetrazol-5-carboxamid.

In analoger Weise kann man herstellen:

N-{{3-{3-[4-Acetyl-3-hydroxy-2-(3,3,3-trifluorpropyl)-phenoxy]-propyloxy}-4-cyano-6-methyl-phenyl}}-1-H-tetrazol-5-carboxamid,

N-{{3-{3-[4-Acetyl-3-hydroxy-2-(3,3,3-trifluorpropyl)-phenoxy]-propyloxy}}-4-fluor-6-methyl-phenyl}}-1-H-tetrazol-5-carboxamid.

Das Ausgangsmaterial kann man z.B. folgendermassen herstellen:

Eine Suspension von 13,1 g 2-Cyano-3-nitro-phenol und 13,8 g geglühten Kaliumcarbonates in 100 ml Ethylmethylketon wird mit einer Spatelspitze Kaliumjodid und 31,5 g 4-(3-Brompropoxy)-2-hydroxy-3-(3,3,3-trifluorpropyl)-acetophenon versetzt und 20 Stunden zum Rückfluss erhitzt. Das Reaktionsgemisch wird abgekühlt, auf Wasser gegossen und dreimal mit Dichlormethan extrahiert. Die organischen Phasen werden mit Wasser gewaschen, vereinigt, über Natriumsulfat getrocknet und unter vermindertem Druck zur Trockne eingeengt. Kristallisation des Rückstandes aus Ether/Hexan liefert das 2-{3-[4-Acetyl-3-hydroxy-2-(3,3,3-trifluorpropyl)-phenoxy]-propyloxy}-6-nitro-benzonitril.

Zur Lösung von 10 g 2-{3-[4-Acetyl-3-hydroxy-2-(3,3,3-trifluorpropyl)-phenoxy]-propyloxy}-6-nitro-benzonitril und 10 g Cyclohexen in 500 ml Ethanol gibt man 2,5 g 10% Pd auf Kohle und erhitzt 30 Minuten zum Rückfluss. Nach Abkühlen auf Raumtemperatur wird filtriert und vom Lösungsmittel befreit. Der Rückstand wird mit Ether versetzt und die ausgeschiedenen Kristalle werden abfiltriert. Man erhält 2-Amino-6-{3-[4-Acetyl-3-hydroxy-2-(3,3,3-trifluorpropyl)-phenoxy]-propyloxy}-benzonitril.

Zu einer Suspension von 5,8 g Kalium-{1-(4-methoxybenzyl)-tetrazol}-5-carboxylat in 110 ml Benzol und 1,0 ml Pyridin gibt man bei 0–5° 1,84 ml (21,5 mMol) Oxalylchlorid und lässt 30 Minuten bei Raumtemperatur rühren. Das Reaktionsgemisch wird unter vermindertem Druck eingeengt, der Rückstand in Benzol aufgenommen und erneut unter vermindertem Druck eingedampft. Der Rückstand wird in 80 ml Dichlormethan gelöst und bei 0–5° innerhalb von etwa 10 Minuten zu einer Lösung von 6,3 g 2-Amino-6-{3-[4-Acetyl-3-hydroxy-2-(3,3,3-trifluorpropyl)-phenoxy]-propyloxy}-benzonitril und 1,72 ml Pyridin in 40 ml Dichlormethan zugetropft. Anschliessend wird 3 Stunden bei Raumtemperatur gerührt. Das Reaktionsgemisch wird mit Dichlormethan verdünnt und dreimal mit Wasser gewaschen. Die organischen Phasen werden vereinigt, über Natriumsulfat getrocknet und unter vermindertem Druck eingedampft. Kristallisation des Rückstandes aus Ethylacetat/Hexan liefert das N-{{3-{3-[4-Acetyl-3-hydroxy-2-(3,3,3-trifluorpropyl)-phenoxy]-propyloxy}-2-cyano-phenyl}}-1-(4-methoxybenzyl)-tetrazol-5-carboxamid.

<u>Beispiel 15:</u> In analoger Weise wie in den Beispielen 1–14 beschrieben erhält man ferner:
N-{{3-{3-[4-Acetyl-3-hydroxy-2-(3,3,3-trifluorpropyl)-phenoxy]-propyloxy}-4-methyl-6-chlor-phenyl}}-oxaminsäuremethylester;
N-{{3-{3-[4-Acetyl-3-hydroxy-2-(3,3,3-trifluorpropyl)-phenoxy]-propyloxy}-4-methyl-6-bromphenyl}}-oxaminsäuremethylester;
N-{{3-{3-[4-Acetyl-3-hydroxy-2-(3,3,3-trifluorpropyl)-phenoxy]-propyloxy}-4-methyl-6-chlorphenyl}}-oxaminsäure und deren Triethanolammoniumsalz;
N-{{3-{3-[4-Acetyl-3-hydroxy-2-(3,3,3-trifluorpropyl)-phenoxy]-propyloxy}-4-methyl-6-chlor-phenyl}}-1H-tetrazol-5-carboxamid und dessen Triethanolammoniumsalz;
N-{{3-{3-[4-Acetyl-3-hydroxy-2-(3,3,3-trifluorpropyl)-phenoxy]-propyloxy}-4-methyl-6-brom-phenyl}}-1H-tetrazol-5-carboxamid und dessen Triethanolammoniumsalz;
N-{{3-{3-[4-Acetyl-3-hydroxy-2-(3,3,3-trifluorpropyl)-phenoxy]-propyloxy}-2-cyano-phenyl}}-oxaminsäureethylester;
N-{{3-{3-[4-Acetyl-3-hydroxy-2-(3,3,3-trifluorpropyl)-phenoxy]-propyloxy}-2-cyano-phenyl}}-oxaminsäure und deren Triethanolammoniumsalz.

<u>Beispiel 16</u>: Zu einer auf –78°C gekühlten Lösung von 2,2 g N-{{3-{3-[4-Acetyl-3-methoxy-2-(3,3,3-trifluorpropyl)-phenoxy)-propoxy}-phenyl}}-oxaminsäuremethylester in 20 ml Dichlormethan tropft man innerhalb von 5 Minuten 3,0 g Bortribromid hinzu. Dann wird 6 Stunden bei Raumtemperatur gerührt. Unter Kühlung gibt man 5 ml Wasser zu, trennt die organische Phase ab und dampft unter vermindertem Druck ein. Nach Umkristallisieren aus Dichlormethan/Ether erhält man den N-{{3-{3-[4-Acetyl-3-hydroxy-2-(3,3,3-trifluor-propyl)-phenoxy]-propoxy}-phenyl}}-oxaminsäuremethylester.

Das Ausgangsmaterial kann man z.B. wie folgt herstellen:

Zu einer Lösung von 7,46 g 3-{3-[4-Acetyl-3-hydroxy-2-(3,3,3-trifluorpropyl)-phenoxy]-propyloxy}-nitrobenzol in 75 ml Dimethylformamid gibt man 5530 mg Natriumhydrid und erwärmt das Reaktionsgemisch auf 40°. Innert 15 Minuten werden 5,7 g Methyljodid zugetropft. Danach hält man die Reaktionsmischung noch 1 Stunde bei 40°C. Nach Abkühlen giesst man auf verdünnte Salzsäure und extrahiert mit Methylenchlorid und dampft ein. Nach Umkristallisieren aus Ether/Hexan erhält man das 3-{3-[4Acetyl-3-methoxy-2-(3,3,3-trifluorpropyl)-phenoxy]-propyloxy}-nitrobenzol.

Eine Lösung von 10 g 3-{3-[4-Acetyl-3-methoxy-2-(3,3,3-trifluorpropyl)-phenoxy]-propyloxy}-nitrobenzol in 100 ml Tetrahydrofuran wird mit 2,0 g Raney-Nickel versetzt und bei Raumtemperatur hydriert. Der Katalysator wird abfiltriert und mit Tetrahydrofuran gewaschen. Nach Eindampfen des Filtrats unter vermindertem Druck erhält man das 3-{3-[4-Acetyl-3-methoxy-2-(3,3,3-trifluorpropyl)-phenoxy]-propyloxy}-anilin als farbloses Öl.

Zu einer Lösung von 9,2 g 3-{3-[4-Acetyl-3-methoxy-2-(3,3,3-trifluorpropyl)-phenoxy]-propyloxy}-anilin und 4,3 ml Triethanolamin in 90 ml Dichlormethan tropft man innert 10 min die Lösung von 3,45 ml Chloroxalsäuremethylester in 10 ml Dichlormethan. Nach 5-stündigem Rühren bei Raumtemperatur giesst man auf Wasser und extrahiert mit Dichlormethan.

Eindampfen der Extrakte und Umkristallisieren des Rückstandes aus Ether liefert den N-{{3-{3-[4-Acetyl-3-methoxy-2-(3,3,3-trifluorpropyl)-phenoxy]-propoxy}-phenyl}}-oxaminsäuremethylester.

Beispiel 17: Eine Suspension von 2,3 g 2,4-Dihydroxy-3-(3,3,3-trifluorpropyl)-acetophenon und 1,6 g geglühten Kaliumcarbonates in 40 ml Ethylmethylketon wird mit einer Spatelspitze Kaliumjodid und 3,2 g N-[3-(3-Brompropoxy)-phenyl]-oxaminsäuremethylester versetzt und 8 Stunden zum Rückfluss erhitzt. Das Reaktionsgemisch wird abgekühlt, auf Wasser gegossen und dreimal mit Dichlormethan extrahiert. Die vereinigten Extrakte werden mit Wasser gewaschen, über Natriumsulfat getrocknet und unter vermindertem Druck zur Trockne eingeengt. Kristallisation des Rückstandes aus Ether liefert den N-{{3-{3-[4-Acetyl-3-hydroxy-2-(3,3,3-trifluorpropyl)-phenoxy)-propoxy}-phenyl}}-oxaminsäuremethylester.

Das Ausgangsmaterial kann man z.B. wie folgt herstellen:

Zu einer Suspension von 29 g Kaliumcarbonat und 0,5 g Kaliumjodid in 170 ml Aceton gibt man 43 ml 1,3-Dibrompropan und erwärmt zum Rückfluss. Dann tropft man innert 2 Stunden eine Lösung von 19,4 g 3-Nitrophenol zu und erhitzt noch 15 Stunden am Rückfluss. Das Reaktionsgemisch wird heiss filtriert und eingedampft. Nach Chromatographie des Rückstandes an Kieselgel mit Toluol erhält man das 3-(3-Brompropoxy)-nitrobenzol als hellgelbes Öl.

Eine Lösung von 4 g 3-(3-Brompropoxy)-nitrobenzol in 40 ml Tetrahydrofuran wird mit 1 g Raney-Nickel versetzt und bei Raumtemperatur hydriert. Der Katalysator wird abfiltriert und mit Tetrahydrofuran gewaschen. Nach Eindampfen des Filtrats erhält man das 3-(3-Brompropoxy)-anilin als farbloses Öl.

Zu einer Lösung von 3,5 g 3-(3-Brompropoxy)-anilin und 1,3 ml Pyridin in 40 ml Dichlormethan tropft man innert 10 Minuten die Lösung von 1,5 ml Oxalsäuremonomethylesterchlorid in 10 ml Methylen-chlorid. Nach 2-stündigem Rühren bei Raumtemperatur giesst man auf Wasser und extrahiert mit Dichlormethan. Die vereinigten Extrakte werden eingedampft und der Rückstand mit Dichlormethan/Ethylacetat (10:1) an Kieselgel chromatographiert. Das Eluat wird eingedampft und der Rückstand aus Ether/Hexan kristallisiert. Man erhält den N-[3-(3-Brom-propoxy)-phenyl]-oxaminsäuremethylester vom Smp. 90–91°.

Beispiel 18: Eine Suspension von 1,8 g N-(3-Hydroxyphenyl)-oxaminsäuremethylester und 1,38 g geglühtem Kaliumcarbonat in 20 ml Ethylmethylketon wird mit einer Spatelspitze Kaliumjodid und 3,15 g 4-(3-Brompropoxy)-2-hydroxy-3-(3,3,3-trifluorpropyl)-acetophenon versetzt und 12 Stunden zum Rückfluss erhitzt. Das Reaktionsgemisch wird abgekühlt, auf Wasser gegossen und dreimal mit Dichlormethan extrahiert. Die vereinigten Extrakte werden mit Wasser gewaschen, über Natriumsulfat getrocknet und unter vermindertem Druck zur Trockne eingeengt. Kristallisation des Rückstandes aus Methylenchlorid/Ether liefert den N-{{3-{3-[4-Acetyl-3-hydroxy-2-(3,3,3-trifluorpropyl)-phenoxy]-propoxy}-phenyl}}-oxaminsäuremethylester.

Das Ausgangsmaterial erhält man z.B. wie folgt:

Zu einer Lösung von 2,5 g 3-Aminoanisol und 1,5 ml Pyridin in 40 ml Dichlormethan tropft man innert 10 Minuten die Lösung von 1,9 ml Chloroxalsäuremethylester in 10 ml Dichlormethan. Nach 3-stündigem Rühren bei Raumtemperatur giesst man auf Wasser und extrahiert mit Dichlormethan.

Die Extrakte werden über Natriumsulfat getrocknet, eingedampft und aus Ether/Hexan kristallisiert. Man erhält den N-(3-Methoxyphenyl)-oxaminsäuremethylester.

Zu einer auf –78°C gekühlten Lösung von 2 g N-(3-Methoxyphenyl)oxaminsäuremethylester in 20 ml Dichlormethan tropft man innert 5 Minuten 5 g Bortribromid. Man rührt 5 Stunden bei Raumtemperatur, gibt unter Kühlung 5 ml Wasser zu und trennt die organischen Phase ab. Nach Trocknen über Natriumsulfat und Eindampfen erhält man N-(3-Hydroxyphenyl)-oxaminsäuremethylester.

Beispiel 19: In analoger Weise wie in den Beispielen 1 bis 14 und 16 bis 18 beschrieben erhält man ferner: N-{{3-{3-[4-Acetyl-3-hydroxy-2-(3,3,3-trifluorpropyl)-phenoxy]-propyloxy}-4-methoxycarbonyl-phenyl}}-oxaminsäure und deren Triethanolammoniumsalz sowie

N-{{3-{3-[4-Acetyl-3-hydroxy-2-(3,3,3-trifluorpropyl)-phenoxy]-propyloxy}-4-carboxy-phenyl}}-oxaminsäure und deren Mono- und Dinatriumsalz.

Beispiel 20: Eine Suspension von 10,0 g N-{{3-{3-[4-Acetyl-3-hydroxy-2-(3,3,3-trifluorpropyl)-phenoxy]-2-acetoxy-propyloxy}-6-methyl-phenyl}}-oxaminsäuremethylester in 60 ml Methanol und 100 ml n-Natronlauge wird 1 Stunde zum Rückfluss erhitzt. Das Reaktionsgemisch wird abgekühlt, mit verdünnter Salzsäure angesäuert und die ausgefallene N-{{3-{3-[4-Acetyl-3-hydroxy-2-(3,3,3-trifluorpropyl)-phenoxy]-2-hydroxy-propyloxy]-6methyl-phenyl}}-oxaminsäure abfiltriert und mit Wasser gewaschen. Zur Reinigung wird das Produkt in 200 ml siedendem Methanol gelöst, mit 22 ml n-Natronlauge versetzt und unter vermindertem Druck auf etwa 100 ml eingeengt. Nach Zugabe von Aceton und Ether setzt Kristallisation ein. Das Produkt wird abfiltriert und mit Ether gewaschen. Man erhält so das Natriumsalz der N-{{3-{3-[4-Acetyl-3-hydroxy-2-(3,3,3-trifluorpropyl)-phenoxy]-2-hydroxy-propyloxy}-6-methyl-phenyl}}-oxaminsäure.

In analoger Weise kann ausgehend von N-{{4-{3-[4-Acetyl-3-hydroxy-2-(3,3,3-trifluorpropyl)-phenoxy]-2-acetoxy-propoxy}-3-methoxy-phenyl}}-oxaminsäuremethylester N-{{4-{3-[4-Acetyl-3-hydroxy-2-(3,3,3-trifluorpropyl)-phenoxy]-2-hydroxy-propyloxy}-3-methoxy-phenyl}}-oxaminsäure hergestellt werden.

Die Ausgangsmaterialien erhält man beispielsweise wie folgt:

Eine Lösung von 18,4 g 4-Methyl-3-nitro-phenol in 180 ml Ethanol wird mit 440 mg einer 55%igen Natriumhydrid-Suspension in Mineralöl versetzt.Unter Rückfluss wird dann innerhalb von 1 Stunde eine Lösung von 25,0 g 4-(2,3-Epoxypropoxy)-2-hydroxy-3-(3,3,3-trifluorpropyl)-acetophenon, erhältlich durch Umsetzung von 2,4-Dihydroxy-3-(3,3,3-trifluorpropyl)-acetophenon und Epichlorhydrin, in 150 ml Ethanol zugetropft und 6 Stunden unter Rückfluss nachgerührt. Das Reaktionsgemisch wird abgekühlt, unter vermindertem Druck auf etwa 150 ml eingeengt und auf Eis gegossen. Die wässrige Phase wird mit verdünnter Salzsäure angesäuert und dreimal mit Methylenchlorid extrahiert. Die organischen Phasen werden mit Wasser gewaschen, vereinigt, über Natriumsulfat getrocknet und unter vermindertem Druck eingeengt. Kristallisation des Rückstands aus Ether ergibt das 3-{3-[4-Acetyl-3-hydroxy-2-(3,3,3-trifluorpropyl-phenoxy]-2-hydroxy-propyloxy}-6-methyl-nitrobenzol.

In analoger Weise erhält man ausgehend von 4-Hydroxy-3-methoxy-nitrobenzol und 4-(2,3-Epoxypropoxy)-2-hydroxy-3-(3,3,3-trifluorpropyl)acetophenon 4-{3-[4-Acetyl-3-hydroxy-2-(3,3,3-trifluorpropyl)-phenoxy]-2-hydroxy-propyloxy}-3-methoxy-nitrobenzol.

Eine Suspension von 10,0 g 3-{3-[4-Acetyl-3-hydroxy-2-(3,3,3-trifluorpropyl)-phenoxy]-2-hydroxy-propyloxy}-6-methyl-nitrobenzol in 100 ml Essigsäureanhydrid und 1 ml Pyridin wird 1 Stunde bei 60° gerührt.

Anschliessend wird das Reaktionsgemisch unter vermindertem Druck zur Trockne eingedampft und das so erhaltene rohe 3-{3-[4-Acetyl-3-hydroxy-2-(3,3,3-trifluorpropyl-phenoxy]-2-acetoxy-propyloxy}-6-methyl-nitrobenzol mit Raney-Nickel in Tetrahydrofuran hydriert. Man erhält das 3-{3-[4-Acetyl-3-hydroxy-2-(3,3,3-trifluorpropyl)-phenoxy]-2-acetoxy-propyloxy}-6-methyl-anilin.

In analoger Weise erhält man ausgehend von 4-{3-[4-Acetyl-3-hydroxy-2-(3,3,3-trifluorpropyl)-phenoxy]-2-hydroxy-propyloxy}-3-methoxy-nitrobenzol das 4-{3-[4-Acetyl-3-hydroxy-2-(3,3,3-trifluorpropyl)-phenoxy]-2-acetoxy-propyloxy}-3-methoxy-anilin als Öl vom Rf-Wert = 0,10 (Kieselgel; Toluol/Essigester = 6:1).

Zu einer auf 0° gekühlten Lösung von 8,3 g 3-{3-[4-Acetyl-3-hydroxy-2-(3,3,3-trifluorpropyl)-phenoxy]-2-acetoxy-propyloxy}-6-methyl-anilin und 2,9 ml Triethylamin in 70 ml Methylenchlorid wird innerhalb von etwa 5 Minuten eine Lösung von 1,9 ml Chloroxalsäuremethylester in 8 ml Methylenchlorid zugetropft und anschliessend noch 2 Stunden bei Raumtemperatur gerührt. Das Reaktionsgemisch wird auf Eiswasser gegossen und die organische Phase abgetrennt. Die Methylenchloridphase wird mit Wasser gewaschen, über Natriumsulfat getrocknet und unter vermindertem Druck eingedampft. Man erhält so den N-{{3-{3-[4-Acetyl-3-hydroxy-2-(3,3-trifluorpropyl)-phenoxy]-2-acetoxy-propyloxy}-6-methyl-phenyl}}-oxaminsäuremethylester als Öl mit dem Rf-Wert = 0,13 (Kieselgel; Toluol/Essigester = 6,1).

In analoger Weise erhält man ausgehend von 4-{3-[4-Acetyl-3-hydroxy-2-(3,3,3-trifluorpropyl)-phenoxy]-2-acetoxy-propyloxy}-3-methoxy-anilin den N-{4-[3-(4-Acetyl-3-hydroxy-2-(3,3,3-trifluorpropyl)-phenoxy)-2-acetoxy-propyloxy}-3-methoxy-phenyl}}-oxaminsäuremethylester.

<u>Beispiel 21</u>: Zu einer auf 0° gekühlten Lösung von 5,5 g 3-{3-[4-Acetyl-3-hydroxy-2-(3,3,3-trifluorpropyl)-phenoxy]-2-hydroxy-propyloxy}}-4-brom-6-methyl-anilin in 60 ml Dichlormethan wird unter schnellem Rühren 1,45 ml Chloroxalsäureethylester zugetropft. Man lässt 40 Minuten bei 0° und 5 Stunden bei Raumtemperatur nachrühren, giesst das Reaktionsgemisch auf Eiswasser und trennt die organische Phase ab. Die Dichlormethanphase wird dreimal mit je 10 ml Wasser gewaschen, über Natriumsulfat getrocknet und unter vermindertem Druck eingedampft. Der Rückstand wird aus Ether-Petrolether kristallisiert. Der N-{{3-{3-[4-Acetyl-3-hydroxy-2-(3,3,3-trifluorpropyl)-phenoxy]-2-hydroxy-propyloxy}-4-brom-6-methyl-phenyl}}-oxaminsäuremethylester schmilzt bei 113–115°. In ähnlicher Weise kann man auch den N-{{3-{3-[4-Acetyl-3-hydroxy-2-(3,3,3-trifluorpropyl)-phenoxy]-2-hydroxy-propyloxy}-6-methyl-phenyl}}-oxaminsäuremethylester herstellen.

Das Ausgangsmaterial kann wie folgt hergestellt werden:

Eine Mischung von 10,0 g 2,4-Dihydroxy-3-(3,3,3-trifluorpropyl)-acetophenon und 16,4 g Epibromhydrin in 20 ml Ethanol wird zum Rückfluss erhitzt und tropfenweise mit einer Lösung von 2,46 g Kaliumhydroxid in 20 ml Ethanol und 0,5 ml Wasser versetzt. Die Suspension wird 2 Stunden unter Rückfluss erhitzt, abgekühlt, mit 150 ml Wasser verdünnt und mit 200 ml Dichlormethan extrahiert. Die Dichlormethanphase wird mit 40 ml Wasser gewaschen, über Natriumsulfat getrocknet und unter vermindertem Druck zur Trockne eingeengt. Man erhält 4-(2,3-Epoxypropoxy)-2-hydroxy-3-(3,3,3-trifluorpropyl)-acetophenon als gelbes Öl.

Eine Lösung von 3,8 g 2-Brom-4-methyl-5-nitro-phenol in 50 ml wasserfreiem Ethanol wird mit 0,1 g Natriumhydrid-Mineralöl-Dispersion (55%) versetzt. Die rote Lösung wird zum Rückfluss erhitzt und unter Einleiten von Argon tropfenweise innerhalb zwei Stunden mit einer Lösung von 5,0 g 4-(2,3-Epoxypropyloxy}-2-hydroxy-3-(3,3,3-trifluorpropyl)-acetophenon in 50 ml wasserfreiem Ethanol versetzt. Die Mischung wird 7 Stunden unter Rückfluss erhitzt, abgekühlt, mit 200 ml Wasser und 200 ml Dichlormethan versetzt und mit 2-n Salzsäure angesäuert. Die Dichlormethanphase wird abgetrennt und die wässrige Suspension nochmals mit 100 ml Dichlormethan extrahiert. Die vereinigten Dichlormethanphasen werden mit 50 ml Wasser gewaschen, über Natriumsulfat getrocknet und unter vermindertem Druck zur Trockne eingedampft. Kristallisation des Rückstandes aus Ether/Petrolether ergibt das 3-{3-[4-Acetyl-3-hydroxy-2-(3,3,3-trifluorpropyl)-phenoxy]-2-hydroxy-propyloxy}-4-brom-6-methyl-nitrobenzol als blassgelbe Kristalle vom Smp. 127–129°.

Eine Lösung von 6,5 g 3-{3-[4-Acetyl-3-hydroxy-2-(3,3,3-trifluorpropyl)-phenoxy]-2-hydroxy-propyloxy}-4-brom-6-methyl-nitrobenzol in 65 ml Tetrahydrofuran wird mit 1,0 g Raney-Nickel versetzt

und bei Raumtemperatur hydriert. Der Katalysator wird abfiltriert und mit Tetrahydrofuran nachgewaschen. Das Filtrat wird unter vermindertem Druck zur Trockne eingedampft und der Rückstand aus Ether/Petrolether kristallisiert. Das 3-{3-[4-Acetyl-3-hydroxy-2-(3,3,3-trifluorpropyl)-phenoxy]-2-hydroxy-propyloxy}-4-brom-6-methyl-anilin schmilzt bei 118–120°.

Beispiel 22: Eine Mischung von 3,3 g N-{{3-{3-[4-Acetyl-3-hydroxy-2-(3,3,3-trifluorpropyl)-phenoxy]-2-hydroxy-propyloxy}-4-brom-6-methylphenyl}}-oxaminsäureethylester in 40 ml Ethanol und 12,5 ml n-Natronlauge wird 2 Stunden zum Rückfluss erhitzt. Das Reaktionsgemisch wird unter vermindertem Druck eingeengt, mit 50 ml Wasser versetzt und mit 2–n Salzsäure angesäuert. Die Suspension wird mit 100 ml Ethylacetat extrahiert. Die Ethylacetatlösung wird abgetrennt, mit 20 ml Wasser gewaschen, mit Natriumsulfat getrocknet und unter vermindertem Druck eingeengt. Den Rückstand kristallisiert man aus Ethylacetat-Petrolether. Die N-{{3-{3-[4-Acetyl-3-hydroxy-2-(3,3,3-trifluorpropyl)-phenoxy]-2-hydroxy-propyloxy}-4-brom-6-methyl-phenyl}}-oxaminsäure schmilzt bei 195–196°.

Beispiel 23: 2,6 g N-{{3-{3-[4-Acetyl-3-hydroxy-2-(3,3,3-trifluorpropyl)-phenoxy]-2-hydroxy-propyloxy}-4-brom-6-methyl-phenyl}}-oxaminsäure werden in 250 ml Aceton gelöst und mit einer Lösung von 1 ml Diethylamin in 10 ml Aceton versetzt. Die Reaktionslösung wird unter vermindertem Druck auf 30 ml eingeengt und mit Ether versetzt, wobei das Diethylammoniumsalz der N-{{3-{3-[4-Acetyl-3-hydroxy-2-(3,3,3-trifluorpropyl)-phenoxy]-2-hydroxy-propyloxy}-4-brom-6-methyl-phenyl}}-oxaminsäure auskristallisiert. Smp. 160–162°.

Beispiel 24: Eine Lösung von 6,2 g N-{{3-{3-[4-Acetyl-3-hydroxy-2-(3,3,3-trifluorpropyl)-phenoxy]-2-hydroxy-propyloxy}-4-brom-6-methylphenyl}}-1-(4-methoxybenzyl)-tetrazol-5-carboxamid in 130 ml Trifluoressigsäure und 14 ml Anisol wird 30 Minuten zum Rückfluss erhitzt. Das Reaktionsgemisch wird unter vermindertem Druck eingeengt, mit etwa 200 ml Ether und 300 ml Petrolether versetzt und die Kristalle abfiltriert. Man erhält das N-{{3-{3-[4-Acetyl-3-hydroxy-2-(3,3,3-trifluorpropyl)-phenoxy]-2-hydroxy-propyloxy}-4-brom-6-methyl-phenyl}}-1H-tetrazol-5-carboxamid.

Das Ausgangsmaterial kann z.B. wie folgt hergestellt werden:

Zu einer Suspension von 3,0 g Kalium-[1-(4-methoxy-benzyl)-tetrazol]-5carboxylat in 40 ml Benzol und 0,7 ml Pyridin gibt man bei 0–5° 1,0 ml Oxalylchlorid und lässt 30 Minuten bei Raumtemperatur rühren. Das Reaktionsgemisch wird unter vermindertem Druck eingeengt, der Rückstand in Benzol aufgenommen und erneut unter vermindertem Druck eingedampft. Der Rückstand wird in 300 ml Dichlormethan suspendiert und bei 0–5° innerhalb von 10 Minuten zu einer Lösung von 4,2 g 3-{3-[4-Acetyl-3-hydroxy-2-3,3,3-trifluorpropyl)-phenoxy]-2-hydroxy-propyloxy}-4-brom-6-methyl-anilin und 0,92 ml Pyridin in 40 ml Dichlormethan zugetropft. Anschliessend wird 3 Stunden bei Raumtemperatur gerührt. Das Reaktionsgemisch wird mit Dichlormethan verdünnt und zweimal mit Wasser gewaschen. Die organische Phase wird über Natriumsulfat getrocknet und unter vermindertem Druck eingedampft. Das N-{{3-{3-[4-Acetyl-3-hydroxy-2-(3,3,3-trifluorpropyl)-phenoxy]-2-hydroxy-propyloxy}-4-brom-6-methylphenyl}}-1-(4-methoxybenzyl)-tetrazol-5-carboxamid liegt als gelbes Öl vor.

In analoger Weise kann man herstellen:

N-{{3-{3-[4-Acetyl-3-hydroxy-2-(3,3,3-trifluorpropyl)-phenoxy]-2hydroxy-propyloxy}-4-chlor-6-methyl-phenyl}}-1H-tetrazol-5-carboxamid,

N-{{3-{3-[4-Acetyl-3-hydroxy-2-(3,3,3-trifluorpropyl)-phenoxy]-2-hydroxy-propyloxy}-4-cyano-6-methyl-phenyl}}-1H-tetrazol-5-carboxamid,

N-{{3-{3-[4-Acetyl-3-hydroxy-2-(3,3,3-trifluorpropyl)-phenoxy]-2-hydroxy-propyloxy}-4-fluor-6-methyl-phenyl}}-1H-tetrazol-5-carboxamid.

Beispiel 25: In analoger Weise wie in Beispiel 2 beschrieben erhält man durch Umsetzung von 3-{3-[4-Acetyl-3-hydroxy-2-(3,3,3-trifluorpropyl)-phenoxy]-propyloxy}-4-chlor-6-methyl-anilin, 3-{3-[4-Acetyl-3-hydroxy-2-(3,3,3-trifluorpropyl)-phenoxy]-propyloxy}-4-fluor-6-methyl-anilin bzw. 2-{3-[4-Acetyl-3-hydroxy-2-(3,3,3-trifluorpropyl)-phenoxy]-propyloxy}-4-amino-5-methyl-benzonitril mit Chloroxalsäuremethylester bzw. Chloroxalsäureethylester:

N-{{3-{3-[4-Acetyl-3-hydroxy-2-(3,3,3-trifluorpropyl)-phenoxy]-propyloxy}-4-chlor-6-methyl-phenyl}}-oxaminsäuremethylester,

N-{{3-{3-[4-Acetyl-3-hydroxy-2-(3,3,3-trifluorpropyl)-phenoxy]-propyloxy}-4-chlor-6-methyl-phenyl}}-oxaminsäureethylester,

N-{{3-{3-[4-Acetyl-3-hydroxy-2-(3,3,3-trifluorpropyl)-phenoxy]-propyloxy}-4-fluor-6-methyl-phenyl}}-oxaminsäureethylester,

N-{{3-{3-[4-Acetyl-3-hydroxy-2-(3,3,3-trifluorpropyl)-phenoxy]-propyloxy}-4-fluor-6-methyl-phenyl}}-oxaminsäureethylester,

N-{{3-{3-[4-Acetyl-3-hydroxy-2-(3,3,3-trifluorpropyl)-phenoxy]-propyloxy}-4-cyano-6-methyl-phenyl}}-oxaminsäuremethylester und

N-{{3-{3-[4-Acetyl-3-hydroxy-2-(3,3,3-trifluorpropyl)-phenoxy]-propyloxy}-4-cyano-6-methyl-phenyl}}-oxaminsäureethylester.

Beispiel 26: In analoger Weise wie in Beispiel 3, 6 bzw. 12 beschrieben erhält man durch Hydrolyse von N-{{3-{3-[4-Acetyl-3-hydroxy-2-(3,3,3-trifluorpropyl)-phenoxy]-propyloxy}-4-chlor-6-methyl-phenyl}}-oxaminsäuremethylester bzw. -ethylester, N-{{3-{3-[4-Acetyl-3-hydroxy-2-(3,3,3-trifluorpropyl)-phenoxy]-propyloxy}-4-fluor-6-methyl-phenyl}}oxaminsäuremethylester bzw. -ethylester bzw. N-{{3-{3-[4-Acetyl-3-hydroxy-2-(3,3,3-trifluorpropyl)-phenoxy]-propyloxy}-4-cyano-6-methyl-phenyl}}-oxaminsäure-methylester bzw. -ethylester:

N-{{3-{3-[4-Acetyl-3-hydroxy-2-(3,3,3-trifluorpropyl)-phenoxy]-propyloxy}-4-chlor-6-methyl-phenyl}}-oxaminsäure und deren Natrium- und Kaliumsalz, N-{{3-{3-[4-Acetyl-3-hydroxy-2-(3,3,3-trifluorpropyl)phenoxy]-propyloxy}-4-fluor-6-methyl-phenyl}}-oxaminsäure und deren Natrium- und Kaliumsalz sowie N-{{3-{3-[4-Acetyl-3-hydroxy-2-(3,3,3-trifluorpropyl)-phenoxyl]-propyloxy}-4-cyano-6-methyl-phenyl}}-oxaminsäure und deren Natrium- und Kaliumsalz.

Beispiel 27: In analoger Weise wie in den Beispielen 13 und 23 beschrieben kann man auch eine andere der in den vorstehenden Beispielen genannten Oxaminsäure- bzw. 5-Tetrazolcarboxamidverbindungen in ihr Diethanolammonium-, Tris(hydroxymethyl)methylammonium- bzw. Diethylammoniumsalz überführen.

Beispiel 28: Tabletten, enthaltend 25 mg Wirkstoff, z.B. Triethanolammoniumsalz von N-{{3-{3-[4-Acetyl-3-hydroxy-2-(3,3,3-trifluorpropyl)-phenoxy]-propyloxy}-4-chlor-6-methyl-phenyl}}-oxaminsäure, können folgendermassen hergestellt werden:

| Bestandteile (für 1000 Tabletten): | |
|---|---|
| Wirkstoff | 25,0 g |
| Lactose | 100,7 g |
| Weizenstärke | 7,5 g |
| Polyethylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzen werden zunächst durch ein Sieb mit 0,6 mm Maschenweite getrieben. Dann werden der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben und das Gemisch, wenn nötig unter Hinzufügen von Wasser, granuliert. Das Granulat wird über Nacht bei 35° getrocknet durch ein Sieb mit 1,2 mm Maschenweite getrieben und zu beidseitig konkaven Tabletten von etwa 6 mm Durchmesser verpresst.

In analoger Weise können auch Tabletten, enthaltend jeweils 25 mg einer anderen der in den Beispielen 1 bis 27 genannten Verbindungen der Formel I, hergestellt werden, wobei Verbindungen, worin $R_8$ Carboxy oder 5-Tetrazolyl ist, in Form von Salzen mit Basen, z.B. als Natriumsalz oder Triethanolammoniumsalz, oder in freier Form vorliegen können.

Beispiel 29: Kautabletten, enthaltend 30 mg Wirkstoff, z.B. Triethanolammoniumsalz von N-{{3-{3-[4-Acetyl-3-hydroxy-2-(3,3,3-trifluorpropyl)-phenoxy]-propyloxy}-4-chlor-6-methyl-phenyl}}-oxaminsäure, können z.B. folgendermassen hergestellt werden:

| Zusammensetzung (für 1000 Tabletten): | |
|---|---|
| Wirkstoff | 30,0 g |
| Mannit | 267,0 g |
| Lactose | 179,5 g |
| Talkum | 20,0 g |
| Glycin | 12,5 g |
| Stearinsäure | 10,0 g |
| Saccharin | 1,0 g |
| 5%ige Gelatinelösung | q.s. |

Herstellung: Alle festen Ingredienzen werden zunächst durch ein Sieb mit 0,25 mm Maschenweite getrieben. Der Mannit und die Lactose werden gemischt, unter Hinzufügen von Gelatinelösung granuliert, durch ein Sieb mit 2 mm Maschenweite getrieben, bei 50° getrocknet und nochmals durch ein Sieb mit 1,7 mm Maschenweite getrieben. Der Wirkstoff, das Glycin und das Saccharin werden sorgfältig vermischt, der Mannit, das Lactosegranulat, die Stearinsäure und das Talkum hinzugegeben, das Ganze

gründlich vermischt und zu beidseitig konkaven Tabletten von etwa 10 mm Durchmesser mit Bruchrille auf der Oberseite verpresst.

In analoger Weise können auch Tabletten, enthaltend jeweils 30 mg einer anderen der in den Beispielen 1 bis 27 genannten Verbindungen der Formel I, hergestellt werden, wobei Verbindungen, worin $R_8$ Carboxy oder 5-Tetrazolyl ist, in freier Form oder in Form von Salzen mit Basen, z.B. als Natriumsalz oder Triethanolammoniumsalz, vorliegen können.

Beispiel 30: Tabletten, enthaltend 100 mg Wirkstoff, z.B. Triethanolammoniumsalz von N-{{3-{3-[4-Acetyl-3-hydroxy-2-(3,3,3-trifluorpropyl)-phenoxy]-propyloxy}-4-chlor-6-methyl-phenyl}}-oxaminsäure, können folgendermassen hergestellt werden:

| Zusammensetzung (für 1000 Tabletten): | |
|---|---|
| Wirkstoff | 100,0 g |
| Lactose | 248,5 g |
| Maisstärke | 17,5 g |
| Polyethylenglykol 6000 | 5,0 g |
| Talkum | 15,0 g |
| Magnesiumstearat | 4,0 g |
| entmineralisiertes Wasser | q.s. |

Herstellung: Die festen Ingredienzen werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyethylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

In analoger Weise können auch Tabletten, enthaltend 100 mg einer anderen Verbindung der Formel I gemäss den Beispielen 1 bis 27, hergestellt werden, wobei Verbindungen, worin $R_8$ Carboxy oder 5-Tetrazolyl ist, in freier Form oder in Form von Salzen mit Basen, z.B. als Natriumsalz oder Triethanolammoniumsalz, vorliegen können.

Beispiel 31: Eine treibmittelhaltige, feststoffaerosolbildende Inhalationssuspension, enthaltend 0,1 Gew.-% N-{{3-{3-[4-Acetyl-3-hydroxy-2-(3,3,3-trifluorpropyl)-phenoxy]-propyloxy}-4-chlor-6-methyl-phenyl}}-oxaminsäuremethylester (Wirkstoff), kann z.B. folgendermassen hergestellt werden:

| Zusammensetzung: | |
|---|---|
| Wirkstoff, mikronisiert | 0,1 Gew.-% |
| «Sorbitantrioleate» | 0,5 Gew.-% |
| Treibmittel A (Trichlortrifluoräthan) | 4,4 Gew.-% |
| Treibmittel B (Gemisch aus 15 Teilen Dichlordifluormethan und 80 Teilen symm. Dichlortetrafluoräthan) | q.s. |

Herstellung: Der Wirkstoff wird unter Feuchtigkeitsausschluss mit Hilfe eines üblichen Homogenisators unter Zusatz des Sorbitantrioleats in dem Trichlortrifluoräthan suspendiert, die Suspension in einen Dosieraerosolbehälter abgefüllt, dieser verschlossen und unter Druck mit dem Dichlordifluormethan-Dichlortetrafluoräthan-Gemisch ausgefüllt.

In analoger Weise können auch Inhalationssuspensionen, enthaltend eine andere Verbindung der Formel I gemäss den Beispielen 1 bis 27, hergestellt werden, wobei Verbindungen, worin $R_8$ Carboxy oder 5-Tetrazolyl ist, in freier Form oder in Form von Salzen mit Basen, z.B. als Natriumsalz oder Triethanolammoniumsalz, vorliegen können.

Beispiel 32: Eine zur Inhalation geeignete, etwa 2%-ige wässrige Lösung des Triethanolammoniumsalzes von N-{{3-{3-[4-Acetyl-3-hydroxy-2-(3,3,3-trifluorpropyl)-phenoxy]-propyloxy}-4-chlor-6-methylphenyl}}-oxaminsäure (Wirkstoff) kann z.B. in folgender Zusammensetzung hergestellt werden:

| Zusammensetzung: | |
|---|---|
| Wirkstoff | 2000 mg |
| Stabilisator, z. B. Ethylendiamintetraessigsäure-dinatriumsalz | 10 mg |
| Konservierungsmittel, z. B. Benzalkoniumchlorid | 10 mg |
| Wasser, frisch destilliert | ad 100 ml |

Herstellung: Der Wirkstoff wird in frisch destilliertem Wasser gelöst. Dann wird der Stabilisator und das Konservierungsmittel hinzugegeben. Nach vollständiger aller Komponenten wird die erhaltene Lösung auf 100 ml aufgefüllt, in Fläschchen abgefüllt und diese gasdicht verschlossen.

In analoger Weise können auch 2%-ige Inhalationslösungen, enthaltend einen anderen Wirkstoff eines der Beispiele 1 bis 27, hergestellt werden.

Beispiel 33: Zur Insufflation geeignete, etwa 25 mg von N-{{3-{3-[4-Acetyl-3-hydroxy-2-(3,3,3-trifluorpropyl)-phenoxy]-propyloxy}-4-chlor-6-methyl-phenyl}}-oxaminsäuremethylester (Wirkstoff) enthaltende Kapseln können z.B. in folgender Zusammensetzung hergestellt werden:

| Zusammensetzung: | |
|---|---|
| Wirkstoff | 25 g |
| Lactose, fein gemahlen | 25 g |

Herstellung: Der Wirkstoff und die Lactose werden innig vermischt. Das erhaltene Pulver wird sodann gesiebt und in Portionen zu je 50 mg in 1000 Gelatinekapseln abgefüllt.

In analoger Weise können auch Insufflationskapseln, enthaltend jeweils einen anderen Wirkstoff gemäss einem der Beispiele 1 bis 27, hergestellt werden.

**Patentansprüche für die Vertragsstaaten: BE, CH, DE, FR, GB, IT, LI, LU, NL, SE**

1. 2-Fluoralkylierter 4-Acylresorcinether der Formel

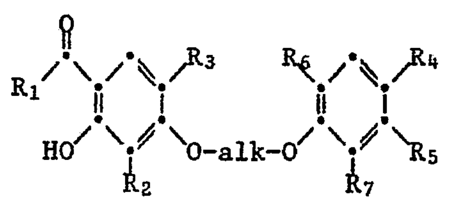

(I),

worin $R_1$ Niederalkyl bedeutet, $R_2$ fluoriertes Niederalkyl darstellt, $R_3$ Wasserstoff, Niederalkoxy, Trifluormethyl oder Halogen bedeutet, alk einen Alkylenrest mit 2 bis und mit 9 Kettengliedern und mit 2 bis und mit 9 C-Atomen, einen Hydroxyalkylenrest mit 3 bis und mit 7 Kettengliedern und mit 3 bis und mit 7 C-Atomen, einen durch Sauerstoff unterbrochenen Alkylenrest mit 5 bis und mit 19 Kettengliedern und mit 4 bis und mit 16 C-Atomen oder einen durch Sauerstoff unterbrochenen Hydroxyalkylenrest mit 6 bis und mit 11 Kettengliedern und mit 5 bis und mit 9 C-Atomen darstellt, einer der Reste $R_4$, $R_5$ und $R_7$ für eine Gruppe der Formel $-NH-C(=O)-R_8$, ein davon verschiedener Rest $R_4$ oder $R_5$ für einen Rest $R_9$ und ein davon verschiedener Rest $R_7$ für einen Rest $R_{10}$ steht, $R_6$ Wasserstoff, Niederalkyl, Trifluormethyl, Halogen, gegebenenfalls verestertes oder amidiertes Carboxy, Cyano oder Niederalkanoyl darstellt, $R_8$ gegebenenfalls verestertes oder amidiertes Carboxy oder 5-Tetrazolyl bedeutet, $R_9$ Wasserstoff, Niederalkyl, Niederalkoxy, Halogen oder Trifluormethyl bedeutet und $R_{10}$ für Wasserstoff, Niederalkyl, Niederalkoxy, Halogen, Trifluormethyl, Cyano oder gegebenenfalls verestertes oder amidiertes Carboxy steht, wobei Halogen eine Atomnummer bis und mit 53 aufweisen kann und "niedere" Gruppen bis und mit 7 C-Atome haben können, oder ein Salz davon.

2. Eine Verbindung gemäss Anspruch 1, worin $R_1$ Niederalkyl bedeutet, $R_2$ fluoriertes Niederalkyl mit bis und mit 3 Fluoratomen bedeutet, $R_3$ Wasserstoff, Niederalkoxy, Trifluormethyl oder Halogen darstellt, alk für gegebenenfalls durch Sauerstoff unterbrochenes Alkylen bzw. Hydroxyalkylen der Formeln $-(CH_2)_m-$ (Ia),

$$-(CH_2)_m-\ (Ia),\ -(CH_2)_{n'}-[O-(CH_2)_n]_k\ (Ib)$$

oder $-[(CH_2)_{l}-O]_o-CH_2-CH(OH)-CH_2-[O-(CH_2)_{l'}]_p$ (Ic)

steht, worin k für 1, 2 oder 3 steht, 1 und 1' unabhängig voneinander 2 oder 3 bedeuten, m eine ganze Zahl von 2 bis und mit 9 bedeutet, n und n' unabhängig voneinander 2, 3 oder 4 bedeuten und o und p unabhängig voneinander für 0 oder 1 stehen, einer der Reste $R_4$, $R_5$ und $R_7$ für eine Gruppe der Formel $-NH-C(=O)-R_8$, ein davon verschiedener Rest $R_4$ oder $R_5$ für einen Rest $R_9$ und ein davon verschiedener Rest $R_7$ für einen Rest $R_{10}$ steht, $R_6$ Wasserstoff, Niederalkyl, Trifluormethyl, Halogen, Niederalkanoyl, Carboxy, Niederalkoxycarbonyl, Cyano, Carbamyl, N-Mono- oder N,N-Diniederalkylcarbamyl bedeutet, $R_8$ einerseits Carboxy, Niederalkoxycarbonyl, N,N-Diniederalkylaminoniederalkoxycarbonyl, N,N-Niederalkylenaminoniederalkoxycarbonyl, Carbamyl, N-Mono- oder N,N-Diniederalkylcarbamyl, N,N-Niederalkylen- oder N,N-(Aza)niederalkylen-, N,N-(Oxa)niederalkylen- bzw. N,N-(Thia)niederalkylencarbamyl oder andererseits 5-Tetrazolyl bedeutet, $R_9$ Wasserstoff, Niederalkyl, Niederalkoxy, Halogen oder Trifluormethyl bedeutet und $R_{10}$ für Wasserstoff, Niederalkyl, Niederalkoxy, Halogen, Trifluormethyl, Carboxy, Niederalkoxycarbonyl, Cyano, Carbamyl, N-Mono- oder N,N-Diniederalkylcarbamyl steht, wobei Halogen eine Atomnummer bis und mit 53 aufweisen kann und "niedere" Gruppen bis und mit 7 C-Atome haben können, oder ein Salz davon.

3. Eine Verbindung gemäss Anspruch 1, worin $R_1$ Niederalkyl mit bis und mit 4 C-Atomen ist, $R_2$ ω-Fluor-, ω,ω-Difluor- oder ω,ω,ω-Trifluorniederalkyl mit bis und mit 4 C-Atomen bedeutet, $R_3$ für Wasserstoff steht, $R_4$ Wasserstoff, Niederalkyl mit bis und mit 4 C-Atomen, Trifluormethyl oder Halogen mit einer Atomnummer von 17 bis und mit 53 bedeutet, $R_5$ eine Gruppe der Formel $-NH-C(=O)-R_8$ darstellt, $R_6$ für Wasserstoff, Niederalkyl mit bis und mit 4 C-Atomen, Halogen mit einer Atomnummer von 17 bis und mit 53, Trifluormethyl, Niederalkoxycarbonyl mit bis und mit 5 C-Atomen, Cyano oder Carboxy steht, $R_7$ Wasserstoff, Niederalkyl mit bis und mit 4 C-Atomen, Halogen mit einer Atomnummer von 17 bis und mit 53, Carbamyl oder Cyano bedeutet, $R_8$ für Carboxy, Niederalkoxycarbonyl mit bis und mit 5 C-Atomen oder 5-Tetrazolyl steht und alk geradkettiges Niederalkylen mit 2 bis und mit 7 C-Atomen oder Hydroxyniederalkylen mit 3 bis und mit 7 C-Atomen, in dem die Hydroxygruppe in höherer als der α-Stellung und in niedrigerer als der ω-Stellung gebunden ist, bedeutet, oder ein Salz davon.

4. Eine Verbindung gemäss Anspruch 1, worin $R_1$ Niederalkyl mit bis und mit 4 C-Atomen ist, $R_2$ ω-Fluor-, ω,ω-Difluor- oder ω,ω,ω-Trifluorniederalkyl mit bis und mit 4 C-Atomen bedeutet, $R_3$ für Wasserstoff steht, $R_4$ Wasserstoff, Niederalkyl mit bis und mit 4 C-Atomen, Trifluormethyl oder Halogen mit einer Atomnummer von 17 bis und mit 53 bedeutet, $R_5$ eine Gruppe der Formel $-NH-C(=O)-R_8$ ist, $R_6$ für Wasserstoff, Niederalkyl mit bis und mit 4 C-Atomen, Halogen mit einer Atomnummer von 17 bis und mit 53, Trifluormethyl oder Cyano steht, $R_7$ Wasserstoff, Niederalkyl mit bis und mit 4 C-Atomen, Cyano oder Halogen mit einer Atomnummer von 17 bis und mit 53 darstellt, $R_8$ für Carboxy, Niederalkoxycarbonyl mit bis und mit 5 C-Atomen oder 5-Tetrazolyl steht und alk geradkettiges Niederalkylen mit 2 bis und mit 7 C-Atomen oder Hydroxyniederalkylen mit 3 bis und mit 7 C-Atomen, in dem die Hydroxygruppe in höherer als der α-Stellung und in niedrigerer als der ω-Stellung gebunden ist, bedeutet, oder ein Salz davon.

5. Eine Verbindung gemäss Anspruch 1, worin $R_1$ Niederalkyl mit bis und mit 4 C-Atomen bedeutet, $R_2$ ω,ω,ω-Trifluorniederalkyl mit bis und mit 3 C-Atomen bedeutet, $R_3$ Wasserstoff bedeutet, $R_5$ eine Gruppe der Formel $-NH-C(=O)-R_8$ darstellt, $R_4$ für Niederalkyl mit bis und mit 4 C-Atomen steht, $R_6$ Halogen mit einer Atomnummer von 17 bis und mit 53 oder Cyano darstellt, $R_7$ Wasserstoff ist, $R_8$ für Carboxy, Niederalkoxycarbonyl mit bis und mit 5 C-Atomen oder 5-Tetrazolyl steht und alk geradkettiges Niederalkylen mit 2 bis und mit 7 C-Atomen oder Hydroxyniederalkylen mit 3 bis und mit 7 C-Atomen, in dem die Hydroxygruppe in höherer als der α-Stellung und in niedrigerer als der ω-Stellung gebunden ist, darstellt, oder ein Salz davon.

6. Eine Verbindung gemäss Anspruch 1 worin $R_1$ Niederalkyl mit bis und mit 4 C-Atomen bedeutet, $R_2$ ω-Fluor-, ω,ω-Difluor- oder ω,ω,ω-Trifluorniederalkyl mit bis und mit 3 C-Atomen bedeutet, $R_3$ und $R_7$ für Wasserstoff stehen, einer der Reste $R_4$ und $R_6$ Niederalkyl mit bis und mit 4 C-Atomen und der andere Halogen mit einer Atomnummer von 17 bis und mit 53 bedeutet, $R_5$ eine Gruppe der Formel $-NH-C(=O)-R_8$ ist, $R_8$ für Carboxy oder 5-Tetrazolyl steht und alk geradkettiges Niederalkylen mit 2 bis und mit 7 C-Atomen oder Hydroxyniederalkylen mit 3 bis und mit 7 C-Atomen, in dem die Hydroxygruppe in höherer als der α-Stellung und in niedrigerer als der ω-Stellung gebunden ist, darstellt, oder ein Salz davon.

7. N-{{3-{3-[4-Acetyl-3-hydroxy-2-(3,3,3-trifluorpropyl)-phenoxy]-propyloxy}-4-chlor-6-methyl-phenyl}}-1H-tetrazol-5-carboxamid oder ein Salz davon gemäss Anspruch 1.

8. N-{{3-{3-[4-Acetyl-3-hydroxy-2-(3,3,3-trifluorpropyl)-phenoxy]-propyloxy}-4-brom-6-methyl-phenyl}}-1H-tetrazol-5-carboxamid oder ein Salz davon gemäss Anspruch 1.

9. N-{{3-{3-[4-Acetyl-3-hydroxy-2-(3,3,3-trifluorpropyl)-phenoxy]-propyloxy}-4-brom-6-methyl-phenyl}}-oxaminsäure oder ein Salz davon gemäss Anspruch 1.

10. N-{{3-{3-[4-Acetyl-3-hydroxy-2-(3,3,3-trifluorpropyl)-phenoxy]-2-hydroxy-propoxy}-4-brom-6-methyl-phenyl}}-oxaminsäure oder ein Salz davon gemäss Anspruch 1.

11. N-{{3-{3-[4-Acetyl-3-hydroxy-2-(3,3,3-trifluorpropyl)-phenoxy]-2-hydroxy-propoxy}-4-brom-6-methyl-phenyl}}-1H-tetrazol-5-carboxamoxid oder ein Salz davon gemäss Anspruch 1.

12. N-{{3-{3-[4-Acetyl-3-hydroxy-2-(3,3,3-trifluorpropyl)-phenoxy]-2-hydroxy-propoxy}-4-brom-6-methyl-phenyl}}-1H-tetrazol-5-carboxamid oder ein Salz davon gemäss Anspruch 1.

13. N-{{3-{3-[4-Acetyl-3-hydroxy-2-(3,3,3-trifluorpropyl)-phenoxy]-2-hydroxy-propoxy}-4-cyano-6-methyl-phenyl}}-1H-tetrazol-5-carboxamid oder ein Salz davon gemäss Anspruch 1.

14. N-{{3-{3-[4-Acetyl-3-hydroxy-2-(3,3,3-trifluorpropyl)-phenoxy]propyloxy}-4-brom-6-methyl-phenyl}}-oxaminsäuremethylester, N-{{3-{3-[4-Acetyl-3-hydroxy-2-(3,3,3-trifluorpropyl)-phenoxy]propyloxy}-4-fluor-6-methyl-phenyl}}-1-H-tetrazol-5-carboxamid oder ein Salz davon, N-{{3-{-3-[4-Acetyl-3-hydroxy-2-(3,3,3-trifluorpropyl)-phenoxy]-2-hydroxypropoxy}-4-fluor-6-methyl-phenyl}}-1H-tetrazol-5-carboxamid oder ein Salz davon, N-{{3-[3-[4-Acetyl-3-hydroxy-2-(3,3,3-trifluorpropyl)-phenoxy]propyloxy}-4-brom-6-methyl-phenyl}}-oxaminsäureethylester oder N-{{3-{3[4-Acetyl-3-hydroxy-2-(3,3,3-trifluorpropyl)-phenoxy]-2-hydroxy-propoxy}-4-brom-6-methyl-phenyl}}-oxaminsäureethylester gemäss Anspruch 1.

15. N-{{3-{3-[4-Acetyl-3-hydroxy-2-(3,3,3-trifluorpropyl)-phenoxy]propoxy}-4-chlor-6-methyl-phenyl}}-oxaminsäure oder ein Salz davon, N-{{3-{3-[4-Acetyl-3-hydroxy-2-(3,3,3-trifluorpropyl)-phenoxy]propoxy}-4-fluor-6-methyl-phenyl}}-oxaminsäure oder ein Salz davon, N-{{3-{3-[4-Acetyl-3-hydroxy-2-(3,3,3-trifluorpropyl)-phenoxy]propoxy}-4-cyano-6-methyl-phenyl}}-oxaminsäure oder ein Salz davon, N-{{3-{3-[4-Acetyl-3-hydroxy-2-(3,3,3-trifluorpropyl)-phenoxy]propoxy}-4-chlor-6-methyl-phenyl}}-oxaminsäuremethylester, N-{{3-{3-[4-Acetyl-3-hydroxy-2-(3,3,3-trifluorpropyl)-phenoxy]propoxy}-4-fluor-6-methyl-phenyl}}-oxaminsäuremethylester, N-{{3-{3-[4-Acetyl-3-hydroxy-2-(3,3,3-trifluorpropyl)-phenoxy]propoxy}-4-chlor-6-methyl-phenyl}}-oxaminsäureethylester oder N-{{3-{3-[4-Acetyl-3-hydroxy-2-(3,3,3-trifluorpropyl)-phenoxy]propoxy}-4-fluor-6-methyl-phenyl}}-oxaminsäureethylester gemäss Anspruch 1.

16. Eine salzbildende Verbindung gemäss einem der Ansprüche 1, 2 und 6 bis 14 in Form des Natrium-, Kalium-, Triethanolammonium-, Diethanolammonium-, Tris(hydroxymethyl)methylammonium- oder Diethylammoniumsalzes.

17. Eine salzbildende Verbindung gemäss einem der Ansprüche 3 bis 5 und 15 in Form des Natrium-, Kalium-, Triethanolammonium-, Diethanolammonium- Tris(hydroxymethyl)methylammonium- oder Diethylammoniumsalzes.

18. Eine Verbindung gemäss einem der Ansprüche 1 bis 17 zur Anwendung in einem Verfahren zur therapeutischen Behandlung des menschlichen oder tierischen Körpers.

19. Ein pharmazeutisches Präparat, enthaltend eine Verbindung gemäss einem der Ansprüche 1, 2, 6 bis 14 und 16 neben üblichen pharmazeutischen Hilfs- und/oder Trägerstoffen.

20. Ein pharmazeutisches Präparat, enthaltend eine Verbindung gemäss einem der Ansprüche 3 bis 5, 15 und 17 neben üblichen pharmazeutischen Hilfs- und/oder Trägerstoffen.

21. Verfahren zur Herstellung eines 2-fluoralkylierten 4-Acylresorcinethers der Formel I oder eines Salzes davon gemäss einem der Ansprüche 1 bis 17, dadurch gekennzeichnet, dass man

a) eine Verbindung der Formel

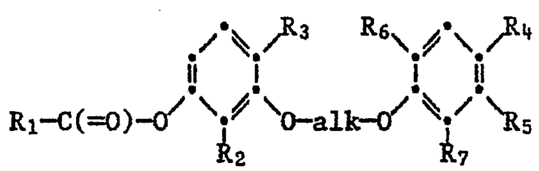

(II)

umlagert oder

b) eine Verbindung der Formel

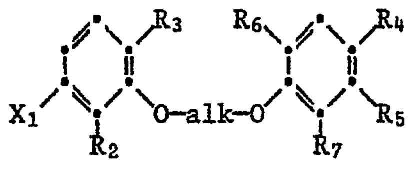

(III),

worin $X_1$ ggf. verethertes Hydroxy bedeutet, mit einer Verbindung der Formel $R_1–X_2$ (IV) umsetzt, worin $X_2$ ggf. funktionell abgewandeltes Carboxy bedeutet, oder

c) eine Verbindung der Formel

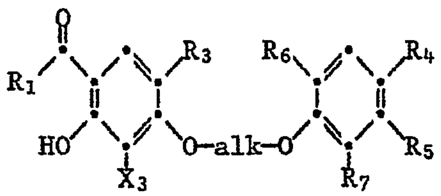

(V),

worin $X_3$ einen in $R_2$ überführbaren Rest bedeutet, oder in einem Salz davon $X_3$ in $R_2$ überführt oder
d) in einer Verbindung der Formel

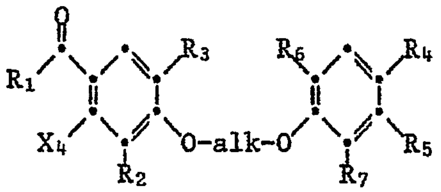

(VI),

worin $X_4$ einen in Hydroxy überführbaren Rest bedeutet, $X_4$ in Hydroxy überführt oder
e) Verbindungen der Formel

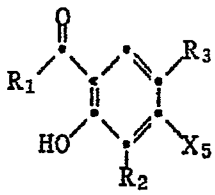

(VII) und

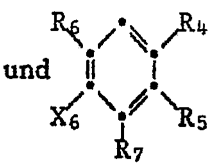

(VIII)

miteinander umsetzt, worin einer der Reste $X_5$ und $X_6$ gegebenenfalls in Salzform vorliegendes Hydroxy und der andere einen durch reaktionsfähiges verestertes Hydroxy oder Epoxy substituierten Rest –O–alkH, d.h. einen durch reaktionsfähiges verestertes Hydroxy substituierten Alkoxy- oder Mono-, Di- oder Trioxaalkoxyrest oder einen durch Epoxy substituierten Alkoxy- oder Mono- oder Dioxaalkoxyrest, darstellt, oder
f) eine Verbindung der Formel

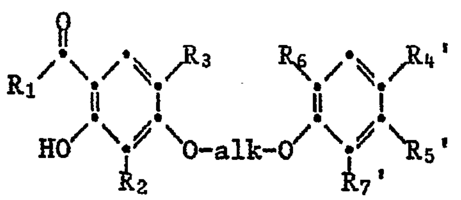

(IX) ,

worin einer der Reste $R_4'$, $R_5'$ und $R_7'$, die Aminogruppe, ein davon verschiedener Rest $R_4'$ bzw. $R_5'$ einen Rest $R_9$ und ein davon verschiedener Rest $R_7'$ einen Rest $R_{10}$ darstellt, oder ein Salz davon mit einer Verbindung der Formel

$$X_7 - R_8' \qquad (X)$$

umsetzt, worin $R_8'$, eine gegebenenfalls veresterte oder amidierte Carboxygruppe oder gegebenenfalls in 1-Stellung geschütztes 5-Tetrazolyl und $X_7$, eine gegebenenfalls veresterte, amidierte, anhydridisierte oder, sofern $R_8'$, für in 1-Stellung geschütztes 5-Tetrazolyl steht, in Salzform vorliegende Carboxygruppe bedeutet, und gegebenenfalls die Schutzgruppe in 1-Stellung einer 5-Tetrazolylgruppe $R_8$ abspaltet oder
g) in einer Verbindung der Formel

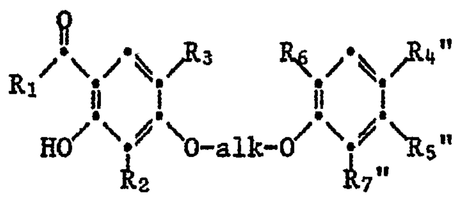

(XI),

worin einer der Reste $R_4$", $R_5$"und $R_7$" einen Rest $X_8$, ein davon verschiedener Rest $R_4$" bzw. $R_5$" einen Rest $R_9$ und ein davon verschiedener Rest $R_7$" einen Rest $R_{10}$ darstellt und $X_8$ einen in die Gruppe der Formel $-NH-C(=O)-R_8$ überführbaren Rest bedeutet, $X_8$ in diese Gruppe überführt oder

h) in einer Verbindung der Formel

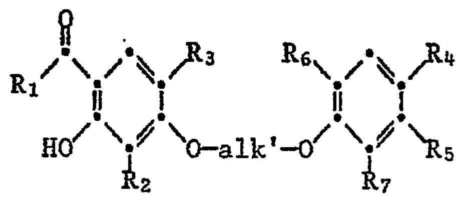

(I'),

worin alk' einen in die Gruppe alk überführbaren Rest bedeutet, alk' in alk überführt und gewünschtenfalls jeweils eine verfahrensgemäss erhältliche Verbindung der Formel I in eine andere Verbindung der Formel I überführt, ein verfahrensgemäss erhältliches Isomerengemisch auftrennt und das oder die gewünschte(n) Isomere(n) der Formel I isoliert und/oder eine verfahrensgemäss erhältliche freie Verbindung der Formel I in ein Salz oder ein verfahrensgemäss erhältliches Salz in die freie Verbindung der Formel I oder in ein anderes Salz umwandelt.

22. Eine Verbindung der Formel

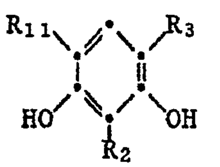

(XXXIII),

worin $R_2$ fluoriertes Niederalkyl bedeutet, $R_3$ Wasserstoff, Niederalkoxy, Trifluormethyl oder Halogen bedeutet und $R_{11}$ Wasserstoff oder eine Gruppe der Formel $R_1-C(=O)-$ bedeutet, in der $R_1$ für Niederalkyl steht, wobei Halogen eine Atomnummer bis und mit 53 aufweisen kann und "niedere" Gruppen bis und mit 7 C-Atome haben können, oder ein Salz davon.

23. 2-(3,3,3-Trifluorpropyl)-resorcin oder ein Salz davon oder 4-Acetyl-2-(3,3,3-trifluorpropyl)-resorcin oder ein Salz davon gemäss Anspruch 22.

24. Verfahren zur Herstellung einer Verbindung der Formel XXXIII oder eines Salzes davon gemäss Anspruch 22 oder 23, dadurch gekennzeichnet, dass man in einer Verbindung der Formel

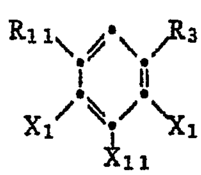

(XXIV),

worin mindestens einer der Reste $X_1$ verethertes Hydroxy R und ein davon verschiedener Rest $X_1$ freies Hydroxy bedeutet und $X_{11}$ eine gegen $R_2$ austauschbare oder in $R_2$ überführbare Gruppe darstellt, $X_{11}$ gegen $R_2$ austauscht oder in $R_2$ überführt, verethertes Hydroxy R zu Hydroxy spaltet und gewünschtenfalls die verfahrensgemäss erhältliche Verbindung der Formel XXXIII in eine andere Verbindung der Formel XXXIII überführt, insbesondere anstelle von Wasserstoff $R_{11}$ eine Gruppe der Formel $R_1-C(=O)-$ einführt und/oder eine verfahrensgemäss erhältliche freie Verbindung der Formel XXXIII in ein Salz oder ein verfahrensgemäss erhältliches Salz in die freie Verbindung der Formel XXXIII überführt.

25. Verfahren zur Herstellung einer Verbindung der Formel XXXIII oder eines Salzes davon gemäss Anspruch 22 oder 23, worin $R_2$ 3,3,3-Trifluorpropyl bedeutet, dadurch gekennzeichnet, dass man eine Verbindung der Formel

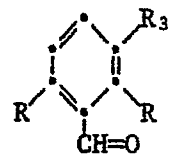

(XX),

worin R verethertes Hydroxy, wie Niederalkoxy mit bis und mit 4 C-Atomen, z.B. Methoxy, bedeutet, in einem inerten Lösungsmittel mit einem Etherat von 2,2,2-Trifluor-1,1-dichlor-ethylzinkchlorid der Formel $CF_3CCl_2ZnClx2(C_2H_5)_2O$ (XXI) zu der entsprechenden Verbindung der Formel

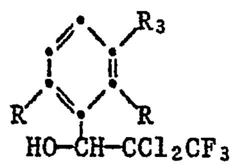

(XXII)

umsetzt, diese in der Seitenkette acyliert, das Reaktionsprodukt der Formel

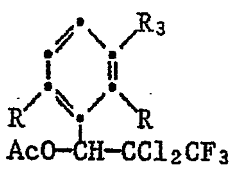

(XXIIa),

worin der Rest Ac für die eingeführte Acylgruppe steht, durch Behandeln mit einem metallischen Reduktionsmittel in die entsprechende Verbindung der Formel

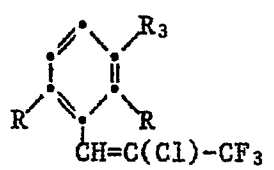

(XXIII)

überführt, aus dieser durch Behandeln mit einer Metallbase Chlorwasserstoff abspaltet, in der erhaltenen Verbindung der Formel

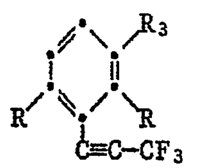

(XXIV)

die Seitenkette hydriert und die veretherten Hydroxygruppen R zu Hydroxy spaltet, gewünschtenfalls einen Rest $R_{11}$ der Formel $R_1$–C(=O)– einführt und gewünschtenfalls eine erhaltene freie Verbindung der Formel XXXIII in ein Salz oder ein erhaltenes Salz in die freie Verbindung der Formel XXXIII umwandelt.

26. Verfahren gemäss Anspruch 24 oder 25 zur Herstellung von 2-(3,3,3-Trifluorpropyl)-resorcin oder 4-Acetyl-2-(3,3,3-trifluorpropyl)-resorcin oder jeweils eines Salzes davon.

27. Verwendung einer Verbindung gemäss einem der Ansprüche 1 bis 18 zur Herstellung allergischer und/oder entzündungshemmender Arzneimittel.

**Patentansprüche für den Vertragsstaat AT**

1. Verfahren zur Herstellung eines 2-fluoralkylierten 4-Acylresorcinethers der Formel

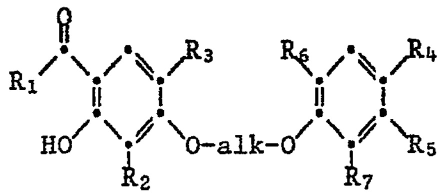

(I),

worin $R_1$ Niederalkyl bedeutet, $R_2$ fluoriertes Niederalkyl darstellt, $R_3$ Wasserstoff, Niederalkoxy, Trifluormethyl oder Halogen bedeutet, alk einen Alkylenrest mit 2 bis und mit 9 Kettengliedern und mit 2 bis und mit 9 C-Atomen, einen Hydroxyalkylenrest mit 3 bis und mit 7 Kettengliedern und mit 3 bis und mit 7 C-Atomen, einen durch Sauerstoff unterbrochenen Alkylenrest mit 5 bis und mit 19 Kettengliedern und mit 4 bis und mit 16 C-Atomen oder einen durch Sauerstoff unterbrochenen Hydroxyalkylenrest mit 6 bis und mit 11 Kettengliedern und mit 5 bis und mit 9 C-Atomen darstellt, einen der Reste $R_4$, $R_5$ und $R_7$ für eine Gruppe der Formel $-NH-C(=O)-R_8$, ein davon verschiedener Rest $R_4$ oder $R_5$ für einen Rest $R_9$ und ein davon verschiedener Rest $R_7$ für einen Rest $R_{10}$ steht, $R_6$ Wasserstoff, Niederalkyl, Trifluormethyl, Halogen, gegebenenfalls verestertes oder amidiertes Carboxy, Cyano oder Niederalkanoyl darstellt, $R_8$ gegebenenfalls verestertes oder amidiertes Carboxy oder 5-Tetrazolyl bedeutet, $R_9$ Wasserstoff, Niederalkyl, Niederalkoxy, Halogen oder Trifluormethyl bedeutet und $R_{10}$ für Wasserstoff, Niederalkyl, Niederalkoxy, Halogen, Trifluormethyl, Cyano oder gegebenenfalls verestertes oder amidiertes Carboxy steht, wobei Halogen eine Atomnummer bis und mit 53 aufweisen kann und "niedere" Gruppen bis und mit 7 C-Atome haben können, oder eines Salzes davon, dadurch gekennzeichnet, dass man

a) eine Verbindung der Formel

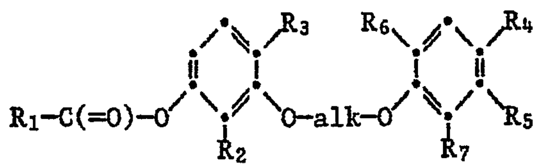

(II)

umlagert oder

b) eine Verbindung der Formel

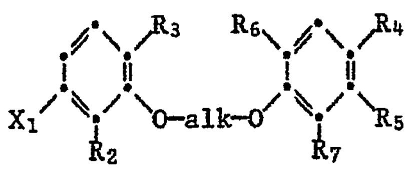

(III),

worin $X_1$ ggf. verethertes Hydroxy bedeutet, mit einer Verbindung der Formel $R_1-X_2$ (IV) umsetzt, worin $X_2$ ggf. funktionell abgewandeltes Carboxy bedeutet, oder

c) eine Verbindung der Formel

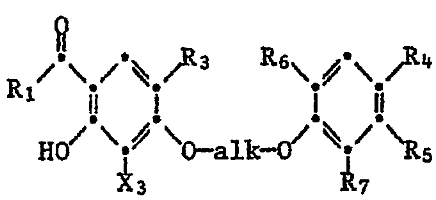

(V),

worin $X_3$ einen $R_2$ überführbaren Rest bedeutet, oder in einem Salz davon $X_3$ in $R_2$ überführt oder

d) eine Verbindung der Formel

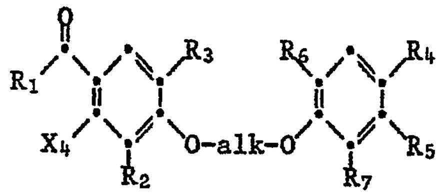

(VI),

worin $X_4$ einen in Hydroxy überführbaren Rest bedeutet, $X_4$ in Hydroxy überführt oder
e) Verbindungen der Formeln

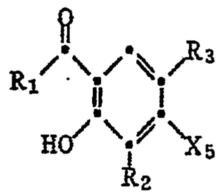

(VII) und

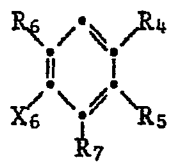

(VIII)

miteinander umsetzt, worin einer der Reste $X_5$ und $X_6$ gegebenenfalls in Salzform vorliegendes Hydroxy und der andere einen durch reaktionsfähiges verestertes Hydroxy oder Epoxy substituierten Rest –O–alkH, d.h. eine durch reaktionsfähiges verestertes Hydroxy substituierten Alkoxy- oder Mono-, Di- oder Trioxaalkoxyrest oder einen durch Epoxy substituierten Alkoxy- oder Mono- oder Dioxaalkoxyrest, darstellt, oder
f) eine Verbindung der Formel

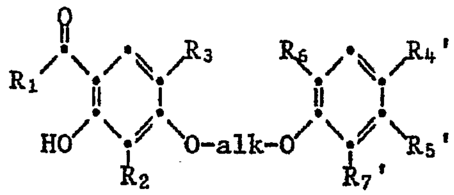

(IX) ,

worin einer der Reste $R_4'$, $R_5'$ und $R_7'$ die Aminogruppe, ein davon verschiedener Rest $R_4'$ bzw. $R_5'$ einen Rest $R_9$ und ein davon verschiedener Rest $R_7'$ einen Rest $R_{10}$ darstellt, oder ein Salz davon mit einer Verbindung der Formel

$$X_7 - R_8' \quad (X)$$

umsetzt, worin $R_8'$ eine gegebenenfalls veresterte oder amidierte Carboxygruppe oder gegebenenfalls in 1-Stellung geschütztes 5-Tetrazolyl und $X_7$ eine gegebenenfalls veresterte, amidierte, anhydrisierte oder, sofern $R_8'$ für in 1-Stellung geschütztes 5-Tetrazolyl steht, in Salzform vorliegende Carboxygruppe bedeutet, und gegebenenfalls die Schutzgruppe in 1-Stellung einer 5-Tetrazolylgruppe $R_8$ abspaltet oder
g) in einer Verbindung der Formel

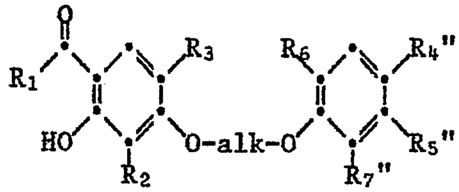

(XI),

worin einer der Reste $R_4''$, $R_5''$ und $R_7''$ einen Rest $X_8$, ein davon verschiedener Rest $R_4''$ bzw. $R_5''$ einen Rest $R_9$ und ein davon verschiedener Rest $R_7''$ einen Rest $R_{10}$ darstellt und $X_8$ einen in die Gruppe der Formel –NH–C(=O)–$R_8$ überführbaren Rest bedeutet, $X_8$ in diese Gruppe überführt oder
h) in einer Verbindung der Formel

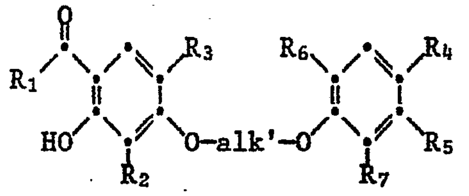

(I'),

worin alk' einen in die Gruppe alk überführbaren Rest bedeutet, alk' in alk überführt und gewünschtenfalls jeweils eine verfahrensgemäss erhältliche Verbindung der Formel I in eine andere Verbindung der Formel I überführt, ein verfahrensgemäss erhältliches Isomerengemisch auftrennt und das oder die gewünschte(n) Isomere(n) der Formel I isoliert und/oder eine verfahrensgemäss erhältliche freie Verbindung der Formel I in ein Salz oder ein verfahrensgemäss erhältliches Salz in die freie Verbindung der Formel I oder in ein anderes Salz umwandelt.

2. Verfahren gemäss Anspruch 1 zur Herstellung einer Verbindung der Formel I, worin $R_1$ Niederalkyl bedeutet, $R_2$ fluoriertes Niederalkyl mit bis und mit 3 Fluoratomen bedeutet, $R_3$ Wasserstoff, Niederalkoxy, Trifluormethyl oder Halogen darstellt, alk für gegebenenfalls durch Sauerstoff unterbrochenes Alkylen bzw. Hydroxyalkylen der Formeln

$$-(CH_2)_m-\ (Ia),\quad -(CH_2)_{n'}-[O-(CH_2)_n]_k\ (Ib)$$

oder

$$-[(CH_2)_{l'}-O]_o-CH_2-CH(OH)-CH_2-[O-(CH_2)_l]_p\ (Ic)$$

steht, worin k für 1, 2 oder 3 steht, l und l' unabhängig voneinander 2 oder 3 bedeuten, m eine ganze Zahl von 2 bis und mit 9 bedeutet, n und n' unabhängig voneinander 2, 3 oder 4 bedeuten und o und p unabhängig voneinander für 0 oder 1 stehen, einer der Reste $R_4$, $R_5$ und $R_7$ für eine Gruppe der Formel $-NH-C-(=O)-R_8$, ein davon verschiedener Rest $R_4$ oder $R_5$ für einen Rest $R_9$ und ein davon verschiedener Rest $R_7$ für einen Rest $R_{10}$ steht, $R_6$ Wasserstoff, Niederalkyl, Trifluormethyl, Halogen, Niederalkanoyl, Carboxy, Niederalkoxycarbonyl, Cyano, Carbamyl, N-Mono- oder N,N-Diniederalkylcarbamyl bedeutet, $R_8$ einerseits Carboxy, Niederalkoxycarbonyl, N,N-Diniederalkylaminoniederalkoxycarbonyl, N,N-Niederalkylenaminoniederalkoxycarbonyl, Carbamyl, N-Mono- oder N,N-Diniederalkylcarbamyl, N,N-Niederalkylen- oder N,N-(Aza)niederalkylen-, N,N-(Oxa)niederalkylen- bzw. N,N-(Thia)niederalkylencarbamyl oder andererseits 5-Tetrazolyl bedeutet, $R_9$ Wasserstoff, Niederalkyl, Niederalkoxy, Halogen oder Trifluormethyl bedeutet und $R_{10}$ für Wasserstoff, Niederalkyl, Niederalkoxy, Halogen Trifluormethyl, Carboxy, Niederalkoxycarbonyl, Cyano, Carbamyl, N-Mono- oder N,N-Diniederalkylcarbamyl steht, wobei Halogen eine Atomnummer bis und mit 53 aufweisen kann und "niedere" Gruppen bis und mit 7 C-Atomen haben können, oder eines Salzes davon.

3. Verfahren gemäss Anspruch 1 zur Herstellung einer Verbindung der Formel I, worin $R_1$ Niederalkyl mit bis und mit 4 C-Atomen ist, $R_2$ ω-Fluor, ω,ω-Difluor- oder ω,ω,ω-Trifluorniederalkyl mit bis und mit 4 C-Atomen bedeutet, $R_3$ für Wasserstoff steht, $R_4$ Wasserstoff, Niederalkyl mit bis und mit 4 C-Atomen, Trifluormethyl oder Halogen mit einer Atomnummer von 17 bis und mit 53 bedeutet, $R_5$ eine Gruppe der Formel $-NH-C(=O)-R_8$ darstellt, $R_6$ Wasserstoff, Niederalkyl mit bis und mit 4 C-Atomen, Halogen mit einer Atomnummer von 17 bis und mit 53, Trifluormethyl, Niederalkoxycarbonyl mit bis und mit 5 C-Atomen, Cyano oder Carboxy steht, $R_7$ Wasserstoff, Niederalkyl mit bis und mit 4 C-Atomen, Halogen mit einer Atomnummer von 17 bis und mit 53, Carbamyl oder Cyano bedeutet, $R_8$ für Carboxy, Niederalkoxycarbonyl mit bis und mit 5 C-Atomen oder 5-Tetrazolyl steht und alk geradkettiges Niederalkylen mit 2 bis und mit 7 C-Atomen oder Hydroxyniederalkylen mit 3 bis und mit 7 C-Atomen, in dem die Hydroxygruppe in höherer als der α-Stellung und in niederigerere als der ω-Stellung gebunden ist, bedeutet, oder eines Salzes davon.

4. Verfahren gemäss Anspruch 1 zur Herstellung einer Verbindung der Formel I, worin $R_1$ Niederalkyl mit bis und mit 4 C-Atomen ist, $R_2$ ω-Fluor, ω,ω-Difluor- oder ω,ω,ω-Trifluorniederalkyl mit bis und mit 4 C-Atomen bedeutet, $R_3$ für Wasserstoff steht, $R_4$ Wasserstoff, Niederalkyl mit bis und mit 4 C-Atomen, Trifluormethyl oder Halogen mit einer Atomnummer von 17 bis und mit 53 bedeutet, $R_5$ eine Gruppe der Formel $-NH-C(=O)-R_8$ ist, $R_6$ für Wasserstoff, Niederalkyl mit bis und mit 4 C-Atomen, Halogen mit einer Atomnummer von 17 bis und mit 53, Trifluormethyl oder Cyano steht, $R_7$ Wasserstoff, Niederalkyl mit bis und mit 4 C-Atomen, Cyano oder Halogen mit einer Atomnummer von 17 bis und mit 53 darstellt, $R_8$ für Carboxy, Niederalkoxycarbonyl mit bis und mit 5 C-Atomen oder 5-Tetrazolyl steht und alk geradkettiges Niederalkylen mit 2 bis und mit 7 C-Atomen oder Hydroxyniederalkylen mit 3 bis und mit 7 C-Atomen, in dem die Hydroxygruppe in höherer als der α-Stellung und in niedrigerer als der ω-Stellung gebunden ist, bedeutet, oder eines Salzes davon.

5. Verfahren gemäss Anspruch 1 zur Herstellung einer Verbindung der Formel I, worin $R_1$ Niederalkyl mit bis und mit 4 C-Atomen bedeutet, $R_2$ ω,ω,ω-Trifluorniederalkyl mit bis und mit 3 C-Atomen bedeutet,

$R_3$ Wasserstoff bedeutet, $R_5$ eine Gruppe der Formel –NH–C(=O)–$R_8$ darstellt, $R_4$ für Niederalkyl mit bis und mit 4 C-Atomen steht, $R_6$ Halogen mit einer Atomnummer von 17 bis und mit 53 oder Cyano darstellt, $R_7$ Wasserstoff ist, $R_8$ für Carboxy, Niederalkoxycarbonyl mit bis und mit 5 C-Atomen oder 5-Tetrazolyl steht und alk geradkettiges Niederalkylen mit 2 bis und mit 7 C-Atomen oder Hydroxyniederalkylen mit 3 bis und mit 7 C-Atomen, in dem die Hydroxygruppe in höherer als der α-Stellung und in niedrigerer als der ω-Stellung gebunden ist, darstellt, oder eines Salzes davon.

6. Verfahren gemäss Anspruch 1 zur Herstellung einer Verbindung der Formel I, worin $R_1$ Niederalkyl mit bis und mit 4 C-Atomen bedeutet, $R_2$ ω-Fluor, ω,ω-Difluor- oder ω,ω,ω-Trifluorniederalkyl mit bis und mit 3 C-Atomen bedeutet, $R_3$ und $R_7$ für Wasserstoff stehen, einer der Reste $R_4$ und $R_6$ Niederalkyl mit bis und mit 4 C-Atomen und der andere Halogen mit einer Atomnummer von 17 bis und mit 53 bedeutet, $R_5$ eine Gruppe der Formel –NH–C(=O)–$R_8$ ist, $R_8$ für Carboxy oder 5-Tetrazolyl steht und alk geradkettiges Niederalkylen mit 2 bis und mit 7 C-Atomen oder Hydroxyniederalkylen mit 3 bis und mit 7 C-Atomen, in dem die Hydroxygruppe in höherer als der α-Stellung und in niedrigerer als der ω-Stellung gebunden ist, darstellt, oder eines Salzes davon.

7. Verfahren gemäss Anspruch 1 zur Herstellung von N-{{3-{3-[4-Acetyl-3-hydroxy-2-(3,3,3-trifluorpropyl)-phenoxy]propyloxy}-4-chlor-6-methyl-phenyl}}-1H-tetrazol-5-carboxamid oder eines Salzes davon.

8. Verfahren gemäss Anspruch 1 zur Herstellung von N-{{3-{3-[4-Acetyl-3-hydroxy-2-(3,3,3-trifluorpropyl)-phenoxy]propyloxy}-4-brom-6-methyl-phenyl}}-1H-tetrazol-5-carboxamid oder eines Salzes davon.

9. Verfahren gemäss Anspruch 1 zur Herstellung von N-{{3-{3-[4-Acetyl-3-hydroxy-2-(3,3,3-trifluorpropyl)-phenoxy]propyloxy}-4-brom-6-methyl-phenyl}}-oxaminsäure oder eines Salzes davon.

10. Verfahren gemäss Anspruch 1 zur Herstellung von N-{{3-{3-[4-Acetyl-3-hydroxy-2-(3,3,3-trifluorpropyl)-phenoxy]-2-hydroxy-propoxy}-4-brom-6-methyl-phenyl}}-oxaminsäure oder eines Salzes davon.

11. Verfahren gemäss Anspruch 1 zur Herstellung von N-{{3-{3-[4-Acetyl-3-hydroxy-2-(3,3,3-trifluorpropyl)-phenoxy]-2-hydroxy-propoxy}-4-brom-6-methyl-phenyl}}-1H-tetrazol-5-carboxamid oder eines Salzes davon.

12. Verfahren gemäss Anspruch 1 zur Herstellung von N-{{3-{3-[4-Acetyl-3-hydroxy-2-(3,3,3-trifluorpropyl)-phenoxy]-2-hydroxy-propoxy}-4-chlor-6-methyl-phenyl}}-1H-tetrazol-5-carboxamid oder eines Salzes davon.

13. Verfahren gemäss Anspruch 1 zur Herstellung von N-{{3-{3-[4-Acetyl-3-hydroxy-2-(3,3,3-trifluorpropyl)-phenoxy]-2-hydroxy-propoxy}-4-cyano-6-methyl-phenyl}}-1H-tetrazol-5-carboxamid oder eines Salzes davon.

14. Verfahren gemäss Anspruch 1 zur Herstellung von N-{{3-{3-[4-Acetyl-3-hydroxy-2-(3,3,3-trifluorpropyl)-phenoxy]propyloxy}-4-brom-6-methyl-phenyl}}-oxaminsäuremethylester,
N-{{3-{3-[4-Acetyl-3-hydroxy-2-(3,3,3-trifluorpropyl)-phenoxy]propyloxy}-4-fluor-6-methyl-phenyl}}-1H-tetrazol-5-carboxamid oder eines Salzes davon,
N-{{3-{3-[4-Acetyl-3-hydroxy-2-(3,3,3-trifluorpropyl)-phenoxy]-2-hydroxy-propoxy}-4-fluor-6-methyl-phenyl}}-1H-tetrazol-5-carboxamid oder eines Salzes davon,
N-{{3-{3-[4-Acetyl-3-hydroxy-2-(3,3,3-trifluorpropyl)-phenoxy]propyloxy}-4-brom-6-methyl-phenyl}}-oxaminsäureethylester,
N-{{3-{3-[4-Acetyl-3-hydroxy-2-(3,3,3-trifluorpropyl)-phenoxy]-2-hydroxy-propoxy}-4-brom-6-methyl-phenyl}}-oxaminsäureethylester,
N-{{3-{3-[4-Acetyl-3-hydroxy-2-(3,3,3-trifluorpropyl)-phenoxy]propoxy}-4-chlor-6-methyl-phenyl}}-oxaminsäure oder eines Salzes davon,
N-{{3-{3-[4-Acetyl-3-hydroxy-2-(3,3,3-trifluorpropyl)-phenoxy]propoxy}-4-fluor-6-methyl-phenyl}}-oxaminsäure oder eines Salzes davon,
N-{{3-{3-[4-Acetyl-3-hydroxy-2-(3,3,3-trifluorpropyl)-phenoxy]propoxy}-4-cyano-6-methyl-phenyl}}-oxaminsäure oder eines Salzes davon,
N-{{3-{3-[4-Acetyl-3-hydroxy-2-(3,3,3-trifluorpropyl)-phenoxy]propoxy}-4-chlor-6-methyl-phenyl}}-oxaminsäuremethylester,
N-{{3-{3-[4-Acetyl-3-hydroxy-2-(3,3,3-trifluorpropyl)-phenoxy]propoxy}-4-fluor-6-methyl-phenyl}}-oxaminsäuremethylester,
N-{{3-{3-[4-Acetyl-3-hydroxy-2-(3,3,3-trifluorpropyl)-phenoxy]-propoxy}-4-chlor-6-methyl-phenyl}}-oxaminsäureethylester oder
N-{{3-{3-[4-Acetyl-3-hydroxy-2-(3,3,3-trifluorpropyl)-phenoxy]propoxy}-4-fluor-6-methyl-phenyl}}-oxaminsäureethylester.

15. Eine salzbildende Verbindung gemäss einem der Ansprüche 1 bis 14 in Form des Natrium-, Kalium-, Triethanolammonium-, Diethanolammonium-, Tris(hydroxymethyl)methylammonium-oder Diethylammoniumsalzes.

16. Verfahren zur Herstellung eines pharmazeutischen Präparates, dadurch gekennzeichnet, dass man eine Verbindung, erhältlich gemäss einem der Ansprüche 1 bis 15, mit üblichen pharmazeutischen Hilfs- und/oder Trägerstoffen vermischt.

17. Verfahren zur Herstellung einer Verbindung der Formel

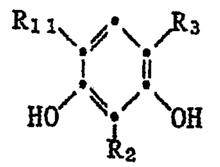 (XXXIII),

worin $R_2$ fluoriertes Niederalkyl bedeutet, $R_3$ Wasserstoff, Niederalkoxy, Trifluormethyl oder Halogen bedeutet und $R_{11}$ Wasserstoff oder eine Gruppe der Formel $R_1$–C(=O)– bedeutet, in der $R_1$ für Niederalkyl steht, wobei Halogen eine Atomnummer bis und mit 53 aufweisen kann und "niedere" Gruppen bis und mit 7 C-Atome haben können, oder eines Salzes davon, dadurch gekennzeichnet, dass man in einer Verbindung der Formel

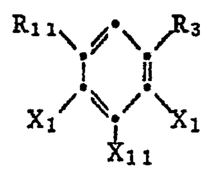 (XXIV),

worin mindestens einer der Reste $X_1$ verethertes Hydroxy R und ein davon verschiedener Rest $X_1$ freies Hydroxy bedeutet und $X_{11}$ eine gegen $R_2$ austauschbare oder in $R_2$ überführbare Gruppe darstellt, $X_{11}$ gegen $R_2$ austauscht oder in $R_2$ überführt, verethertes Hydroxy R zu Hydroxy spaltet und gewünschtenfalls die verfahrengemäss erhältliche Verbindung der Formel XXXIII in eine andere Verbindung der Formel XXXIII überführt, insbesondere anstelle von Wasserstoff $R_{11}$ eine Gruppe der Formel $R_1$–C(=O)– einführt und/oder eine verfahrensgemäss erhältliche freie Verbindung der Formel XXXIII in eine Salz oder ein verfahrensgemäss erhältliches Salz in die freie Verbindung der Formel XXXIII überführt.

18. Verfahren zur Herstellung einer Verbindung der Formel

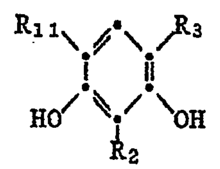 (XXXIII),

worin $R_2$ 3,3,3-Trifluorpropyl bedeutet, $R_3$ Wasserstoff, Niederalkoxy, Trifluormethyl oder Halogen bedeutet und $R_{11}$ Wasserstoff oder eine Gruppe der Formel $R_1$–C(=O)– bedeutet, in der $R_1$ für Niederalkyl steht, wobei Halogen eine Atomnummer bis und mit 53 aufweisen kann und "niedere" Gruppen bis und mit 7 C-Atome haben können, oder eines Salzes davon, dadurch gekennzeichnet, dass man eine Verbindung der Formel

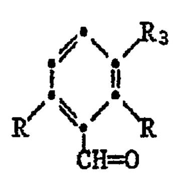 (XX),

worin R verethertes Hydroxy, wie Niederalkoxy mit bis und mit 4 C-Atomen, z.B. Methoxy, bedeutet, in einem inerten Lösungsmittel mit einem Etherat von 2,2,2-Trifluor-1,1-dichlor-ethylzinkchlorid der Formel $CF_3CCl_2ZnClx2(C_2H_5)_2O$ (XXI) zu der entsprechenden Verbindung der Formel

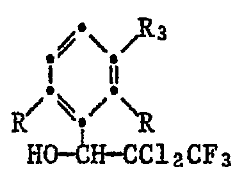 (XXII)

umsetzt, diese in der Seitenkette acyliert, das Reaktionsprodukt der Formel

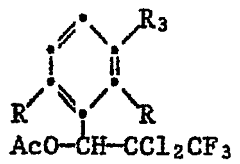

(XXIIa),

worin der Rest Ac für die eingeführte Acylgruppe steht, durch Behandeln mit einem metallischen Reduktionsmittel in die entsprechende Verbindung der Formel

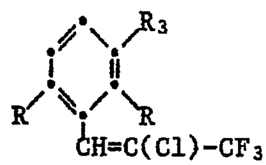

(XXIII)

überführt, aus dieser durch Behandeln mit einer Metallbase Chlorwasserstoff abspaltet, in der erhaltenen Verbindung der Formel

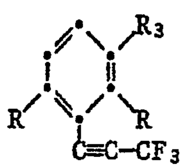

(XXIV)

die Seitenkette hydriert und die veretherten Hydroxygruppen R zu Hydroxy spaltet, gewünschtenfalls einen Rest $R_{11}$ der Formel $R_1$–C(=O)– einführt und gewünschtenfalls eine erhaltene freie Verbindung der Formel XXXIII in ein Salz oder ein erhaltenes Salz in die freie Verbindung der Formel XXXIII umwandelt.

19. Verfahren gemäss Anspruch 17 zur Herstellung einer Verbindung der Formel XXXIII, worin $R_1$, sofern vorhanden, für Niederalkyl mit bis und mit 4 C-Atomen steht, $R_2$ ω-Fluor-, ω,ω-Difluor- oder ω,ω,ω-Trifluorniederalkyl mit bis und mit 4, z.B. 1 oder 3, C-Atomen, ist und $R_3$ Wasserstoff bedeutet, oder eines Salzes davon.

20. Verfahren gemäss Anspruch 17 oder 18 zur Herstellung einer Verbindung der Formel XXXIII, worin $R_1$, sofern vorhanden, Niederalkyl mit bis und mit 4 C-Atomen bedeutet, $R_2$ ω,ω,ω-Trifluorniederalkyl mit bis und mit 4 C-Atomen darstellt und $R_3$ Wasserstoff bedeutet, oder eines Salzes davon.

21. Verfahren gemäss Anspruch 17 oder 18 zur Herstellung von 2-(3,3,3-trifluorpropyl)-resorcin oder eines Salzes davon.

22. Verfahren gemäss Anspruch 17 oder 18 zur Herstellung von 4-Acetyl-2-(3,3,3-trifluorpropyl)-resorcin oder eines Salzes davon.

**Claims for the Contracting States BE, CH, DE, FR, GB, IT, LI, LU, NL, SE**

1. A 2-fluoroalkylated 4-acylresorcinol ether of the formula

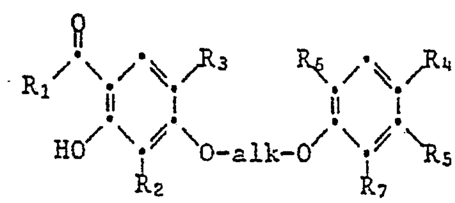

(I)

in which $R_1$ is lower alkyl, $R_2$ is fluorinated lower alkyl, $R_3$ is hydrogen, lower alkoxy, trifluoromethyl or halogen, alk is an alkylene radical having from 2 to not more than 9 chain members and from 2 to not more than 9 C atoms, a hydroxyalkylene radical having from 3 to not more than 7 chain members and from 3 to not more than 7 C atoms, an alkylene radical which is interrupted by oxygen and has from 5 to not more than 19 chain members and from 4 to not more than 16 C atoms, or a hydroxyalkylene radical which is interrupted by oxygen and has from 6 to not more than 11 chain members and from 5 to not more than 9 C atoms, one of the radicals $R_4$, $R_5$ and $R_7$ is a group of the formula –NH–C(=O)–$R_8$, a radical $R_4$ or $R_5$ which differs from this is a radical $R_9$ and a radical $R_7$ which differs from this is a radical $R_{10}$, $R_6$ is hydrogen, lower alkyl, trifluoromethyl, halogen, carboxyl which is free, esterified or amidated, cyano or lower al-

kanoyl, $R_8$ is carboxyl which is free, esterified or amidated or 5-tetrazolyl, $R_9$ is hydrogen, lower alkyl, lower alkoxy, halogen or trifluoromethyl and $R_{10}$ is hydrogen, lower alkyl, lower alkoxy, halogen, trifluoromethyl, cyano or carboxyl which is free, esterified or amidated, it being possible for halogen to have an atomic number of not more than 53 and for "lower" groups to have not more than 7 C atoms, or a salt thereof.

2. A compound according to claim 1, in which $R_1$ is lower alkyl, $R_2$ is fluorinated lower alkyl having not more than 3 fluorine atoms, $R_3$ is hydrogen, lower alkoxy, trifluoromethyl or halogen, alk is alkylene or hydroxyalkylene which is uninterrupted or interrupted by oxygen, of the formulae

$$-(CH_2)_m- \ (Ia), \quad -(CH_2)_{n'}-[O-(CH_2)_n]_k \ (Ib)$$

$$\text{or} \quad -[(CH_2)_{l'}-O]_o-CH_2-CH(OH)-CH_2-[O-(CH_2)_l]_p \ (Ic),$$

in which k is 1,2 or 3,1 and 1' independently of one another are 2 or 3, m is an integer from 2 to not more than 9, n and n' independently of one another are 2, 3 or 4 and o and p independently of one another are 0 or 1, one of the radicals $R_4$, $R_5$ and $R_7$ is a group of the formula $-NH-C(=O)-R_8$, a radical $R_4$ or $R_5$ which differs from this is a radical $R_9$ and a radical $R_7$ which differs from this is a radical $R_{10}$, $R_6$ is hydrogen, lower alkyl, trifluoromethyl, halogen, lower alkanoyl, carboxyl, lower alkoxycarbonyl, cyano, carbamyl or N-mono- or N,N-di-lower alkylcarbamyl, $R_8$ is on the one hand carboxyl, lower alkoxycarbonyl, N,N-di-lower alkylamino-lower alkoxycarbonyl, N,N-lower alkyleneamino-lower alkoxycarbonyl, carbamyl, N-mono- or N,N-di-lower alkylcarbamyl or N,N-lower alkylene- or N,N-(aza)-lower alkylene-, N,N-(oxa)-lower alkylene- or N,N-(thia)-lower alkylenecarbamyl, or on the other hand 5-tetrazolyl, $R_9$ is hydrogen, lower alkyl, lower alkoxy, halogen or trifluoromethyl and $R_{10}$ is hydrogen, lower alkyl, lower alkoxy, halogen, trifluoromethyl, carboxyl, lower alkoxycarbonyl, cyano, carbamyl or N-mono- or N,N-di-lower alkylcarbamyl, it being possible for halogen to have an atomic number of not more than 53 and for "lower" groups to have not more than 7 C atoms, or a salt thereof.

3. A compound according to claim 1, in which $R_1$ is lower alkyl having not more than 4 C atoms, $R_2$ is ω-fluoro-, ω,ω-difluoro- or ω,ω,ω-trifluoro-lower alkyl having not more than 4 C atoms, $R_3$ is hydrogen, $R_4$ is hydrogen, lower alkyl having not more than 4 C atoms, trifluoromethyl or halogen with an atomic number of from 17 to not more than 53, $R_5$ is a group of the formula $-NH-C(=O)-R_8$, $R_6$ is hydrogen, lower alkyl having not more than 4 C atoms, halogen with an atomic number of from 17 to not more than 53, trifluoromethyl, lower alkoxycarbonyl having not more than 5 C atoms, cyano or carboxyl, $R_7$ is hydrogen, lower alkyl having not more than 4 C atoms, halogen with an atomic number of from 17 to not more than 53, carbamyl or cyano, $R_8$ is carboxyl, lower alkoxycarbonyl having not more than 5 C atoms or 5-tetrazolyl and alk is straight-chain lower alkylene having 2 to not more than 7 C atoms, or hydroxy-lower alkylene having 3 to not more than 7 C atoms, in which the hydroxyl group is bonded in a position higher than the α-position and lower than the ω-position, or a salt thereof.

4. A compound according to claim 1, in which $R_1$ is lower alkyl having not more than 4 C atoms, $R_2$ is ω-fluoro-, ω,ω-difluoro- or ω,ω,ω-trifluoro-lower alkyl having not more than 4 C atoms, $R_3$ is hydrogen, $R_4$ is hydrogen, lower alkyl having not more than 4 C atoms, trifluoromethyl or halogen with an atomic number of from 17 to not more than 53, $R_5$ is a group of the formula $-NH-C(=O)-R_8$, $R_6$ is hydrogen, lower alkyl having not more than 4 C atoms, halogen with an atomic number of from 17 to not more than 53, trifluoromethyl or cyano, $R_7$ is hydrogen, lower alkyl having not more than 4 C atoms, cyano or halogen with an atomic number of from 17 to not more than 53, $R_8$ is carboxyl, lower alkoxycarbonyl having not more than 5 C atoms or 5-tetrazolyl, and alk is straight-chain lower alkylene having 2 to not more than 7 C atoms, or hydroxy-lower alkylene having 3 to not more than 7 C atoms, in which the hydroxyl group is bonded in a position higher than the α-position and lower than the ω-position, or a salt thereof.

5. A compound according to claim 1, in which $R_1$ is lower alkyl having not more than 4 C atoms, $R_2$ is ω,ω,ω-trifluoro-lower alkyl having not more than 3 C atoms, $R_3$ is hydrogen and $R_5$ is a group of the formula $-NH-C(=O)-R_8$, $R_4$ is lower alkyl having not more than 4 C atoms, $R_6$ is halogen with an atomic number of from 17 to not more than 53, or cyano, $R_7$ is hydrogen, $R_8$ is carboxyl, lower alkoxycarbonyl having not more than 5 C atoms or 5-tetrazolyl, and alk is straight-chain lower alkylene having 2 to not more than 7 C atoms, or hydroxy-lower alkylene having 3 to not more than 7 C atoms, in which the hydroxyl group is bonded in a position higher than the α-position and lower than the ω-position, or a salt thereof.

6. A compound according to claim 1, in which $R_1$ is lower alkyl having not more than 4 C atoms, $R_2$ is ω-fluoro-, ω,ω-difluoro- or ω,ω,ω-trifluoro- lower alkyl having not more than 3 C atoms, $R_3$ and $R_7$ are hydrogen, one of the radicals $R_4$ and $R_6$ is lower alkyl having not more than 4 C atoms, and the other is halogen with an atomic number of from 17 to not more than 53 , $R_5$ is a group of the formula $-NH-C-(=O)-R_8$, $R_8$ is carboxyl or 5-tetrazolyl, and alk is straight-chain lower alkylene having 2 to not more than 7 C atoms, or hydroxy-lower alkylene having 3 to not more than 7 C atoms, in which the hydroxyl group is bonded in a position higher than the α-position and lower than the ω-position, or a salt thereof.

7. N-{{3-{3-[4-Acetyl-3-hydroxy-2-(3,3,3-trifluoropropyl)-phenoxy]-propoxy}-4-chloro-6-methyl-phenyl}}-1H-tetrazole-5-carboxamide or a salt thereof according to claim 1.

8. N-{{3-{3-[4-Acetyl-3-hydroxy-2-(3,3,3-trifluoropropyl)-phenoxy]-propoxy}-4-bromo-6-methyl-phenyl}}-1H-tetrazole-5-carboxamide or a salt thereof according to claim 1.

9. N-{{3{-3-[4-Acetyl-3-hydroxy-2-(3,3,3-trifluoropropyl)-phenoxy]-propoxy}-4-bromo-6-methyl-phenyl}}-oxamic acid or a salt thereof according to claim 1.

10. N-{{3-(3-[4-Acetyl-3-hydroxy-2-(3,3,3-trifluoropropyl)-phenoxy]-2-hydroxy-propoxy}-4-bromo-6-methyl-phenyl}}-oxamic acid or a salt thereof according to claim 1.

11. N-{{3-{3-[4-Acetyl-3-hydroxy-2-(3,3,3-trifluoropropyl)-phenoxy]-2-hydroxy-propoxy}-4-bromo-6-methyl-phenyl}}-1H-tetrazole-5-carboxamide or a salt thereof according to claim 1.

12. N-{{3-{3-[4-Acetyl-3-hydroxy-2-(3,3,3-trifluoropropyl)-phenoxy]-2-hydroxy-propoxy}-4-chloro-6-methyl-phenyl}}-1H-tetrazole-5-carboxamide or a salt thereof according to claim 1.

13. N-{{3-{3-[4-Acetyl-3-hydroxy-2-(3,3,3-trifluoropropyl)-phenoxy]-2-hydroxy-propoxy}-4-cyano-6-methyl-phenyl}}-1H-tetrazole-5-carboxamide or a salt thereof according to claim 1.

14. Methyl N-{{3-{3-[4-Acetyl-3-hydroxy-2-(3,3,3-trifluoropropyl)-phenoxy]-propoxy}-4-bromo-6-methyl-phenyl}}-oxamate, N-{{3-{3-[4-acetyl-3-hydroxy-2-(3,3,3-trifluoropropyl)-phenoxy]-propoxy}-4-fluoro-6-methyl-phenyl}}-1-H-tetrazole-5-carboxamide or a salt thereof, N-{{3-{3-[4-acetyl-3-hydroxy-2-(3,3,3-trifluoropropyl)-phenoxy]-2-hydroxy-propoxy}-4-fluoro-6-methyl-phenyl}}-1H-tetrazole-5-carboxamide or a salt thereof, ethyl N-{{3-{3-[4-acetyl-3-hydroxy-2-(3,3,3-trifluoropropyl)-phenoxy]-propoxy}-4-bromo-6-methyl-phenyl}}-oxamate or ethyl N-{{3-{3-[4-acetyl-3-hydroxy-2-(3,3,3-trifluoropropyl)-phenoxy]-2-hydroxy-propoxy}-4-bromo-6-methyl-phenyl}}-oxamate according to claim 1.

15. N-{{3-{3-[4-Acetyl-3-hydroxy-2-(3,3,3-trifluoropropyl)-phenoxy]-propoxy}-4-chloro-6-methyl-phenyl}}-oxamic acid or a salt thereof, N-{{3-(3-[4-acetyl-3-hydroxy-2-(3,3,3-trifluoropropyl)-phenoxy]-propoxy}-4-fluoro-6-methyl-phenyl}}-oxamic acid or a salt thereof, N-{{3-{3-[4-acetyl-3-hydroxy-2-(3,3,3-trifluoropropyl)-phenoxy]-propoxy}-4-cyano-6-methyl-phenyl}}-oxamic acid or a salt thereof, methyl N-{{3-{3-[4-acetyl3-hydroxy-2-(3,3,3-trifluoropropyl)-phenoxy]-propoxy}-4-chloro-6-methyl-phenyl}}-oxamate, methyl N-{{3-{3-[4-acetyl-3-hydroxy-2-(3,3,3-trifluoropropyl)-phenoxy]-propoxy}-4-fluoro-6-methyl-phenyl}}-oxamate, ethyl N-{{3-{3-[4-acetyl-3-hydroxy-2-(3,3,3-trifluoropropyl)-phenoxy]-propoxy}-4-chloro-6-methyl-phenyl}}-oxamate or ethyl N-{{3-{3-[4-acetyl-3-hydroxy-2-(3,3,3-trifluoropropyl)-phenoxy]-propoxy}-4-fluoro-6-methyl-phenyl}}-oxamate according to claim 1.

16. A salt-forming compound according to any one of claims 1, 2 and 6 to 14 in the form of the sodium, potassium, triethanolammonium, diethanolammonium, tris(hydroxymethyl)methylammonium or diethylammonium salt.

17. A salt-forming compound according to any one of claims 3 to 5 and 15 in the form of the sodium, potassium, triethanolammonium, diethanolammonium, tris(hydroxymethyl)methylammonium or diethylammonium salt.

18. A compound according to any one of claims 1 to 17 for use in a method of therapeutic treatment of the human or animal body.

19. A pharmaceutical product containing a compound according to any one of claims 1, 2, 6 to 14 and 16, in addition to customary pharmaceutical adjuncts and/or carriers.

20. A pharmaceutical product containing a compound according to any one of claims 3 to 5, 15 and 17 in addition to customary pharmaceutical adjuncts and/or carriers.

21. A process for the preparation of a 2-fluoroalkylated 4-acylresorcinol ether of the formula I or a salt thereof according to any one of claims 1 to 17, which comprises

a) rearranging a compound of the formula

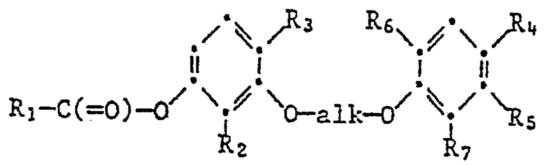

(II)

or

b) reacting a compound of the formula

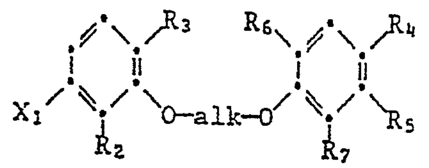

(III)

in which $X_1$ is free or etherified hydroxyl, with a compound of the formula $R_1$–$X_2$ (IV), in which $X_2$ is free or functionally modified carboxyl, or
c) [lacuna] a compound of the formula

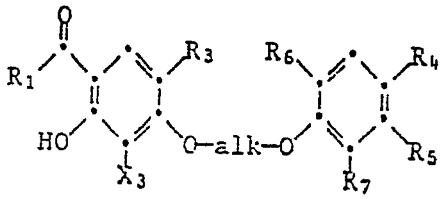

(V)

in which $X_3$ is a radical which can be converted into $R_2$, or in a salt thereof, converting $X_3$ into $R_2$, or
d) converting $X_4$ in a compound of the formula

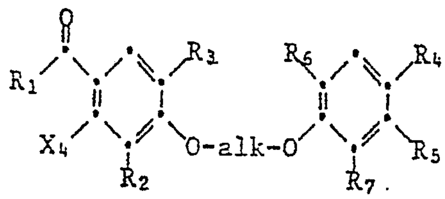

(VI)

in which $X_4$ is a radical which can be converted into hydroxyl or
e) reacting compounds of the formulae

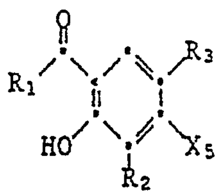

(VII) and

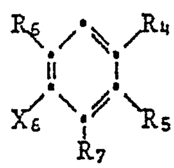

(VIII)

in which one of the radicals $X_5$ and $X_6$ is hydroxyl which is free or in salt form and the other is a radical –O–alkH substituted by reactive esterified hydroxyl or epoxy, i.e. an alkoxy or mono-, di- or trioxaalkoxy radical substituted by reactive esterified hydroxyl or an alkoxy or mono- or dioxaalkoxy radical substituted by epoxy, with one another or
f) reacting a compound of the formula

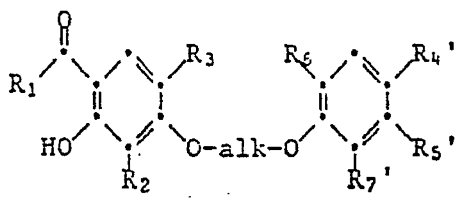

(IX)

in which one of the radicals $R_4$' $R_5$' and $R_7$' is the amino group, a radical $R_4$' or $R_5$' which differs from this is a radical $R_9$ and a radical $R_7$' which differs from this is a radical $R_{10}$, or a salt thereof, with a compound of the formula

$$X_7 - R_8' \qquad (X)$$

in which $R_8$' is a carboxyl group which is free, esterified or amidated or 5-tetrazolyl which is protected, if appropriate, in the 1-position and $X_7$ is a carboxyl group which is free, esterified, amidated, converted into an anhydride or, if $R_8$' is 5-tetrazolyl protected in the 1-position, in salt form, and, if appropriate, detaching the protective group in the 1-position of a 5-tetrazolyl group $R_8$, or
g) converting $X_8$ in a compound of the formula

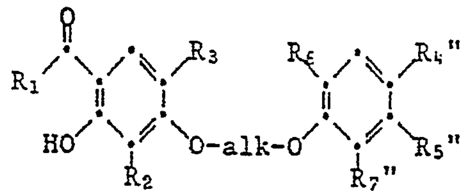

(XI)

in which one of the radicals $R_4$", $R_5$" and $R_7$" is a radical $X_8$, a radical $R_4$" or $R_5$" which differs from this is a radical $R_9$ and a radical $R_7$" which differs from this is a radical $R_{10}$ and $X_8$ is a radical which can be converted into the group of the formula $-NH-C(=O)-R_8$, into this group, or
h) converting alk' in a compound of the formula

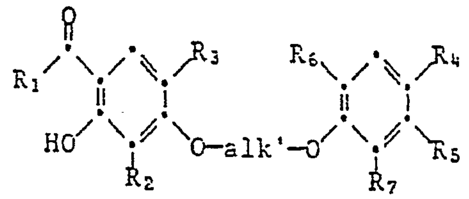

(I')

in which alk' is a radical which can be converted into the group alk, into alk, and, if desired, in each case converting a compound of the formula I obtainable according to the process into another compound of the formula I, separating an isomer mixture obtainable according to the process and isolating the desired isomer(s) of the formula I, and/or converting a free compound of the formula I obtainable according to the process into a salt or a salt obtainable according to the process into the free compound of the formula I or into another salt.
22. A compound of the formula

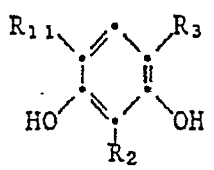

(XXXIII)

in which $R_2$ is fluorinated lower alkyl, $R_3$ is hydrogen, lower alkoxy, trifluoromethyl or halogen and $R_{11}$ is hydrogen or a group of the formula $R_1-C(=O)-$, in which $R_1$ is lower alkyl, it being possible for halogen to have an atomic number of not more than 53 and for "lower" groups to have not more than 7 C atoms, or a salt thereof.

23. 2-(3,3,3-Trifluoropropyl)-resorcinol or a salt thereof or 4-acetyl-2-(3,3,3-trifluoropropyl)-resorcinol or a salt thereof according to claim 22.

24. A process for the preparation of a compound of the formula XXXIII or a salt thereof according to claim 22 or 23, which comprises, in a compound of the formula

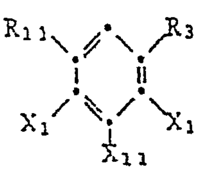

(XXIV)

in which at least one of the radicals $X_1$ is etherified hydroxyl R and a radical $X_1$ which differs from this is free hydroxyl and $X_{11}$ is a group which can be replaced by $R_2$ or converted into $R_2$, replacing $X_{11}$ by $R_2$ or converting it into $R_2$, splitting etherified hydroxyl R to give hydroxyl and, if desired, converting the compound of the formula XXXIII obtainable according to the process into another compound of the formula XXXIII, in particular introducing a group of the formula $R_1-C(=O)-$ instead of hydrogen $R_{11}$ and/or converting a free compound of the formula XXXIII obtainable according to the process into a salt or a salt obtainable according to the process into the free compound of the formula XXXIII.

25. A process for the preparation of a compound of the formula XXXIII or a salt thereof according to claim 22 or 23, in which $R_2$ is 3,3,3-trifluoropropyl, which comprises reacting a compound of the formula

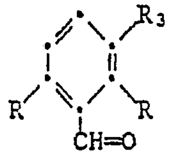

(XX)

in which R is etherified hydroxyl, such as lower alkoxy having not more than 4 C atoms, for example methoxy, in an inert solvent with an etherate of 2,2,2-trifluoro-1,1-dichloro-ethyl-zinc chloride of the formula $CF_3CCl_2ZnClx2(C_2H_5)_2O$ (XXI) to give the corresponding compound of the formula

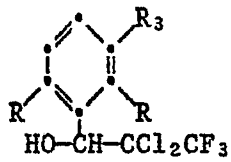

(XXII)

acylating this in the side chain, converting the reaction product of the formula

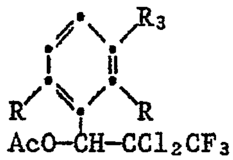

(XXIIa),

in which the radical Ac is the acyl group introduced, by treatment with a metallic reducing agent, into the corresponding compound of the formula

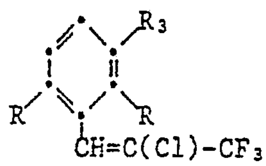

(XXIII)

detaching hydrogen chloride from this product by treatment with a metal baser, hydrogenating the side chain in the resulting compound of the formula

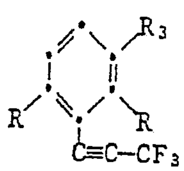

(XXIV)

and splitting the etherified hydroxyl groups R to give hydroxyl, if desired introducing a radical $R_{11}$ of the formula $R_1$–C(=O)– and if desired converting a resulting free compound of the formula XXXIII into a salt or a resulting salt into the free compound of the formula XXXIII.

26. The process according to claim 24 or 25 for the preparation of 2-(3,3,3-trifluoropropyl)-resorcinol or 4-acetyl-2-(3,3,3-trifluoropropyl)-resorcinol, or in each case a salt thereof.

27. The use of a compound according to any one of claims 1 to 18 for the preparation of allergic and/or antiinflammatory medicaments.

**Claims for the Contracting State AT**

1. A process for the preparation of a 2-fluoroalkylated 4-acylresorcinol ether of the formula

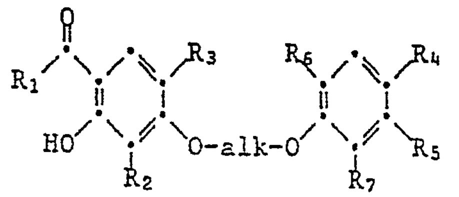

(I)

in which $R_1$ is lower alkyl, $R_2$ is fluorinated lower alkyl, $R_3$ is hydrogen, lower alkoxy, trifluoromethyl or halogen, alk is an alkylene radical having from 2 to not more than 9 chain members and from 2 to not more than 9 C atoms, a hydroxyalkylene radical having from 3 to not more than 7 chain members and from 3 to not more than 7 C atoms, an alkylene radical which is interrupted by oxygen and has from 5 to not more than 19 chain members and from 4 to not more than 16 C atoms, or a hydroxyalkylene radical which is interrupted by oxygen and has from 6 to not more than 11 chain members and from 5 to not more than 9 C atoms, one of the radicals $R_4$, $R_5$ and $R_7$ is a group of the formula $-NH-C(=O)-R_8$, a radical $R_4$ or $R_5$ which differs from this is a radical $R_9$ and a radical $R_7$ which differs from this is a radical $R_{10}$, $R_6$ is hydrogen, lower alkyl, trifluoromethyl, halogen, carboxyl which is free, esterified or amidated, cyano or lower alkanoyl, $R_8$ is carboxyl which is free, esterified or amidated or 5-tetrazolyl, $R_9$ is hydrogen, lower alkyl, lower alkoxy, halogen or trifluoromethyl and $R_{10}$ is hydrogen, lower alkyl, lower alkoxy, halogen, trifluoromethyl, cyano or carboxyl which is free, esterified or amidated, it being possible for halogen to have an atomic number of not more than 53 and for "lower" groups to have not more than 7 C atoms, or a salt thereof, which comprises

a) rearranging a compound of the formula

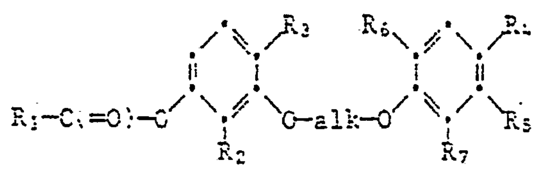

(II)

b) reacting a compound of the formula

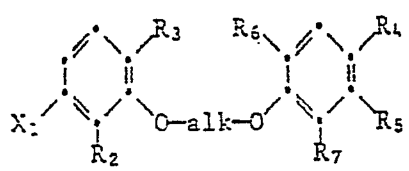

(III)

in which $X_1$ is free or etherified hydroxyl, with a compound of the formula $R_1-X_2$ (IV), in which $X_2$ is free or functionally modified carboxyl, or

c) [lacuna] a compound of the formula

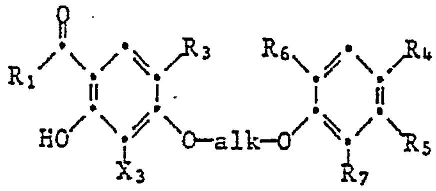

(V)

in which $X_3$ is a radical which can be converted into the radical $R_2$, or in a salt thereof, converting $X_3$ into $R_2$, or

d) converting $X_4$ in a compound of the formula

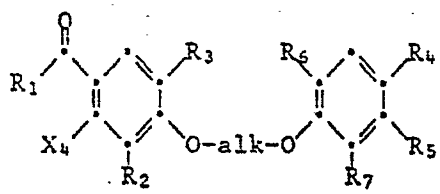

(VI)

in which $X_4$ is a radical which can be converted into hydroxyl, into hydroxyl, or

e) reacting compounds of the formulae

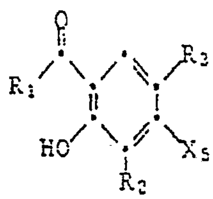

(VII) and

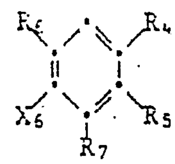

(VIII)

in which one of the radicals $X_5$ and $X_6$ is hydroxyl which is free or in salt form and the other is a radical –O–alkH substituted by reactive esterified hydroxyl or epoxy, i.e. an alkoxy or mono-, di- or trioxaalkoxy radical substituted by reactive esterified hydroxyl or an alkoxy or mono- or dioxaalkoxy radical substituted by epoxy, with one another or

f) reacting a compound of the formula

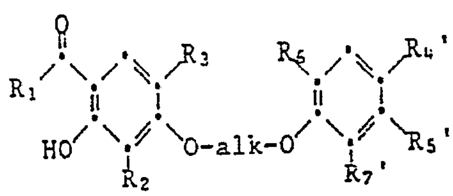

(IX)

in which one of the radicals $R_4'$ $R_5'$ and $R_7'$ is the amino group, a radical R4' or $R_5'$ which differs from this is a radical $R_9$ and a radical $R_7'$ which differs from this is a radical $R_{10}$, or a salt thereof, with a compound of the formula

$$X_7 - R_8' \qquad (X)$$

in which $R_8'$ is a carboxyl group which is free, esterified or amidated or 5-tetrazolyl which is protected, if appropriate, in the 1-position and $X_7$ is a carboxyl group which is free, esterified, amidated, converted into an anhydride or, if $R_8'$ is 5-tetrazolyl protected in the 1-position, in salt form, and, if appropriate, detaching the protective group in the 1position of a 5-tetrazolyl group $R_8$, or

g) converting $X_8$ in a compound of the formula

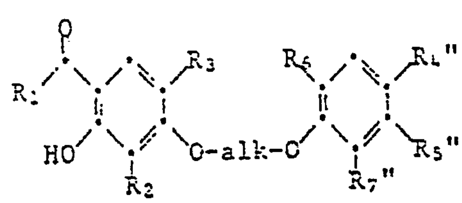

(XI)

in which one of the radicals $R_4''$, $R_5''$ and $R_7''$ is a radical $X_8$, a radical $R_4''$ or $R_5''$ which differs from this is a radical $R_9$ and a radical R7" which differs from this is a radical $R_{10}$ and $X_8$ is a radical which can be converted into the group of the formula –NH–C(=O)–$R_8$, into this group, or

h) converting alk' in a compound of the formula

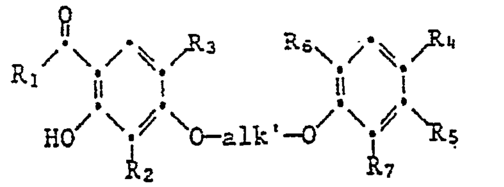

(I')

in which alk' is a radical which can be converted into the group alk, into alk, and, if desired, in each case converting a compound of the formula I obtainable according to the process into another compound of the formula I, separating an isomer mixture obtainable according to the process and isolating the desired isomer(s) of the formula I, and/or converting a free compound of the formula I obtainable according to the process into a salt or a salt obtainable according to the process into the free compound of the formula I or into another salt.

2. A process according to claim 1 for the preparation of a compound of the formula I, in which $R_1$ is lower alkyl, $R_2$ is fluorinated lower alkyl having not more than 3 fluorine atoms, $R_3$ is hydrogen, lower alkoxy, trifluoromethyl or halogen, alk is alkylene or hydroxyalkylene which is uninterrupted or interrupted by oxygen, of the formulae

$$-(CH_2)_m- \ (Ia), \quad -(CH_2)_{n'}-[O-(CH_2)_n]_k \ (Ib)$$

$$\text{or} \quad -[(CH_2)_{l'}-O]_o-CH_2-CH(OH)-CH_2-[O-(CH_2)_l]_p \ (Ic) \qquad (Ic),$$

in which k is 1,2 or 3,1 and 1' independently of one another are 2 or 3, m is an integer from 2 to not more than 9, n and n' independently of one another are 2, 3 or 4 and o and p independently of one another are 0 or 1, one of the radicals $R_4$, $R_5$ and $R_7$ is a group of the formula –NH–C(=O)–$R_8$, a radical $R_4$ or $R_5$ which differs from this is a radical $R_9$ and a radical $R_7$ which differs from this is a radical $R_{10}$, $R_6$ is hydrogen, lower alkyl, trifluoromethyl, halogen, lower alkanoyl, carboxyl, lower alkoxycarbonyl, cyano, carbamyl or N-mono- or N,N-di-lower alkylcarbamyl, $R_8$ is on the one hand carboxyl, lower alkoxycarbonyl, N,N-di-lower alkylamino-lower alkoxycarbonyl, N,N-lower alkyleneamino-lower alkoxycarbonyl, carbamyl, N-mono- or N,N-dilower alkylcarbamyl or N,N-lower alkylene- or N,N-(aza)-lower alkylene-, N,N-(oxa)-lower alkylene- or N,N-(thia)-lower alkylenecarbamyl, or on the other hand 5-tetrazolyl, $R_9$ is hydrogen, lower alkyl, lower alkoxy, halogen or trifluoromethyl and $R_{10}$ is hydrogen, lower alkyl, lower alkoxy, halogen, trifluoromethyl, carboxyl, lower alkoxycarbonyl, cyano, carbamyl or N-mono- or N,N-di-lower alkylcarbamyl, it being possible for halogen to have an atomic number of not more than 53 and for "lower" groups to have not more than 7 C atoms, or a salt thereof.

3. A process according to claim 1 for the preparation of a compound of the formula I, in which $R_1$ is lower alkyl having not more than 4 C atoms, $R_2$ is ω-fluoro-, ω,ω-difluoro- or ω,ω,ω-trifluoro-lower alkyl having not more than 4 C atoms, $R_3$ is hydrogen, $R_4$ is hydrogen, lower alkyl having not more than 4 C atoms, trifluoromethyl or halogen with an atomic number of from 17 to not more than 53, $R_5$ is a group of the formula –NH–C(=O)–$R_8$, $R_6$ is hydrogen, lower alkyl having not more than 4 C atoms, halogen with an atomic number of from 17 to not more than 53, trifluoromethyl, lower alkoxycarbonyl having not more than 5 C atoms, cyano or carboxyl, $R_7$ is hydrogen, lower alkyl having not more than 4 C atoms, halogen with an atomic number of from 17 to not more than 53, carbamyl or cyano, $R_8$ is carboxyl, lower alkoxycarbonyl having not more than 5 C atoms or 5-tetrazolyl and alk is straight-chain lower alkylene having 2 to not more than 7 C atoms, or hydroxy-lower alkylene having 3 to not more than 7 C atoms, in which the hydroxyl group is bonded in a position higher than the α-position and lower than thew ω-position, or a salt thereof.

4. A process according to claim 1 for the preparation of a compound of the formula I, in which $R_1$ is lower alkyl having not more than 4 C atoms, $R_2$ is ω-fluoro-, ω,ω-difluoro- or ω,ω,ω-trifluoro-lower alkyl having not more than 4 C atoms, $R_3$ is hydrogen, $R_4$ is hydrogen, lower alkyl having not more than 4 C atoms, trifluoromethyl or halogen with an atomic number of from 17 to not more than 53, $R_5$ is a group of the formula –NH–C(=O)–$R_8$, $R_6$ is hydrogen, lower alkyl having not more than 4 C atoms, halogen with an atomic number of from 17 to not more than 53, trifluoromethyl or cyano, $R_7$ is hydrogen, lower alkyl having not more than 4 C atoms, cyano or halogen with an atomic number of from 17 to not more than 53, $R_8$ is carboxyl, lower alkoxycarbonyl having not more than 5 C atoms or 5-tetrazolyl, and alk is straight-chain lower alkylene having 2 to not more than 7 C atoms, or hydroxy-lower alkylene having 3 to not more than 7 C atoms, in which the hydroxyl group is bonded in a position higher than the α-position and lower than the ω-position, or a salt thereof.

5. A process according to claim 1 for the preparation of a compound of the formula I, in which $R_1$ is lower alkyl having not more than 4 C atoms, $R_2$ is ω,ω,ω-trifluoro-lower alkyl having not more than 3 C atoms, $R_3$ is hydrogen and $R_5$ is a group of the formula –NH–C(=O)–$R_8$, $R_4$ is lower alkyl having not more

than 4 C atoms, $R_6$ is halogen with an atomic number of from 17 to not more than 53, or cyano, $R_7$ is hydrogen, $R_8$ is carboxyl, lower alkoxycarbonyl having not more than 5 C atoms or 5-tetrazolyl, and alk is straight-chain lower alkylene having 2 to not more than 7 C atoms, or hydroxy-lower alkylene having 3 to not more than 7 C atoms, in which the hydroxyl group is bonded in a position higher than the α-position and lower than the ω-position, or a salt thereof.

6. A process according to claim 1 for the preparation of a compound of the formula I, in which $R_1$ is lower alkyl having not more than 4 C atoms, $R_2$ is ω-fluoro-, ω,ω-difluoro- or ω,ω,ω-trifluoro-lower alkyl having not more than 3 C atoms, $R_3$ and $R_7$ are hydrogen, one of the radicals $R_4$ and $R_6$ is lower alkyl having not more than 4 C atoms, and the other is halogen with an atomic number of from 17 to not more than 53, $R_5$ is a group of the formula –NH–C–(=O)–$R_8$, $R_8$ is carboxyl or 5-tetrazolyl, and alk is straight-chain lower alkylene having 2 to not more than 7 C atoms, or hydroxy-lower alkylene having 3 to not more than 7 C atoms, in which the hydroxyl group is bonded in a position higher than the α-position and lower than the ω-position, or a salt thereof.

7. A process according to claim 1 for the preparation of N-{{3-{3-[4-Acetyl-3-hydroxy-2-(3,3,3-trifluoropropyl)-phenoxy]-propoxy}-4-chloro-6-methyl-phenyl}}-1H-tetrazole-5-carboxamide or a salt thereof.

8. A process according to claim 1 for the preparation of N-{{3-{3-[4-Acetyl-3-hydroxy-2-(3,3,3-trifluoropropyl)-phenoxy]-propoxy}-4-bromo-6-methyl-phenyl}}-1H-tetrazole-5-carboxamide or a salt thereof.

9. A process according to claim 1 for the preparation of N-{{3-{3-[4-Acetyl-3-hydroxy-2-(3,3,3-trifluoropropyl)-phenoxy]-propoxy}-4-bromo-6-methyl-phenyl}}-oxamic acid or a salt thereof.

10. A process according to claim 1 for the preparation of N-{{3-{3-[4-Acetyl-3-hydroxy-2-(3,3,3-trifluoropropyl)-phenoxy]-2-hydroxy-propoxy}-4-bromo-6-methyl-phenyl}}-oxamic acid or a salt thereof.

11. A process according to claim 1 for the preparation of N-{{3-{3-[4-Acetyl-3-hydroxy-2-(3,3,3-trifluoropropyl)-phenoxy]-2-hydroxy-propoxy}-4-bromo-6-methyl-phenyl}}-1H-tetrazole-5-carboxamide or a salt thereof.

12. A process according to claim 1 for the preparation of N-{{3-{3-[4-Acetyl-3-hydroxy-2-(3,3,3-trifluoropropyl)-phenoxy]-2-hydroxy-propoxy}-4-chloro-6-methyl-phenyl}}-1H-tetrazole-5-carboxamide or a salt thereof.

13. A process according to claim 1 for the preparation of N-{{3-{3-[4-Acetyl-3-hydroxy-2-(3,3,3-trifluoropropyl)-phenoxy]-2-hydroxy-propoxy}-4-cyano-6-methyl-phenyl}}-1H-tetrazole-5-carboxamide or a salt thereof.

14. A process according to claim 1 for the preparation of Methyl N-{{3-{3-[4-Acetyl-3-hydroxy-2-(3,3,3-trifluoropropyl)-phenoxy]-propoxy}-4-bromo-6-methyl-phenyl}}-oxamate, N-{{3-{3-[4-acetyl-3-hydroxy-2-(3,3,3-trifluoropropyl)-phenoxy]-propoxy}-4-fluoro-6-methyl-phenyl}}-1H-tetrazole-5-carboxamide or a salt thereof, N-{{3-{3-[4-acetyl-3-hydroxy-2-(3,3,3-trifluoropropyl)-phenoxy}-2-hydroxy-propoxy}-4-fluoro-6-methyl-phenyl}}-1H-tetrazole-5-carboxamide or a salt thereof, ethyl N-{{3-{3-[4-acetyl-3-hydroxy-2-(3,3,3-trifluoropropyl)-phenoxy]-propoxy}-4-bromo-6-methyl-phenyl}}-oxamate or ethyl N-{{3-{3-[4-acetyl-3-hydroxy-2-(3,3,3-trifluoropropyl)-phenoxy]-2-hydroxy-propoxy}-4-bromo-6-methyl-phenyl}}-oxamate, N-{{3-{3-[4-acetyl-3-hydroxy-2-(3,3,3-trifluoropropyl)-phenoxy]propoxy}-4-chloro-6-methyl-phenyl}}-oxamic acid or a salt thereof, N-{{3{3-[4-acetyl-3-hydroxy-2-(3,3,3-trifluoropropyl)-phenoxy]-propoxy}-4-fluoro-6-methyl-phenyl}}-oxamic acid or a salt thereof, N-{{3-{3-[4-acetyl-3-hydroxy-2-(3,3,3-trifluoropropyl)-phenoxy]-propoxy}-4-cyano-6-methyl-phenyl}}-oxamic acid or a salt thereof, methyl N-{{3-{3-[4-acetyl-3-hydroxy-2-(3,3,3-trifluoropropyl)-phenoxy]-propoxy}-4-chloro-6-methyl-phenyl}}-oxamate, methyl N-{{3-{3-[4-acetyl-3-hydroxy-2-(3,3,3-trifluoropropyl)-phenoxy]-propoxy}-4-fluoro-6-methyl-phenyl}}-oxamate, ethyl N-{{3-{3-[4-acetyl-3-hydroxy-2-(3,3,3-trifluoropropyl)-phenoxy]-propoxy}-4-chloro-6-methyl-phenyl}}-oxamate or ethyl N-{{3-{3-[4-acetyl-3-hydroxy-2-(3,3,3-trifluoropropyl)-phenoxy]-propoxy}-4-fluoro-6-methyl-phenyl}}-oxamate.

15. A salt-forming compound according to any one of claims 1 to 14 in the form of the sodium, potassium, triethanolammonium, diethanolammonium, tris(hydroxymethyl)methylammonium or diethylammonium salt.

16. A process for the preparation of a pharmaceutical product which comprises mixing a compound obtainable according to any one of claims 1 to 15 with customary pharmaceutical adjuncts and/or carriers.

17. A process for the preparation of a compound of the formula

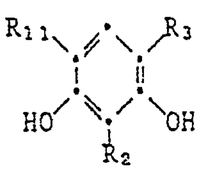

(XXXIII)

in which $R_2$ is fluorinated lower alkyl, $R_3$ is hydrogen, lower alkoxy, trifluoromethyl or halogen and $R_{11}$ is hydrogen or a group of the formula $R_1$–C(=O)–, in which $R_1$ is lower alkyl, it being possible for halogen to

have an atomic number of not more than 53 and for "lower" groups to have not more than 7 C atoms, or a salt thereof, which comprises, in a compound of the formula

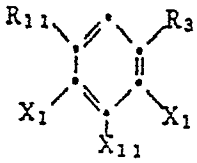

(XXIV)

in which at least one of the radicals $X_1$ is etherified hydroxyl R and a radical $X_1$ which differs from this is free hydroxyl and $X_{11}$ is a group which can be replaced by $R_2$ or converted into $R_2$, replacing $X_{11}$ by $R_2$ or converting it into $R_2$, splitting etherified hydroxyl R to give hydroxyl and, if desired, converting the compound of the formula XXXIII obtainable according to the process into another compound of the formula XXXIII, in particular introducing a group of the formula $R_1$–C(=O)– instead of hydrogen $R_{11}$ and/or converting a free compound of the formula XXXIII obtainable according to the process into a salt or a salt obtainable according to the process into the free compound of the formula XXXIII.

18. A process for the preparation of a compound of the formula

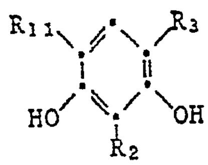

(XXXIII)

in which $R_2$ is 3,3,3-trifluoropropyl, $R_3$ is hydrogen, lower alkoxy, trifluoromethyl or halogen and $R_{11}$ is hydrogen or a group of the formula $R_1$–C(=O)–, in which $R_1$ is lower alkyl, it being possible for halogen to have an atomic number of not more than 53 and for "lower" groups to have not more than 7 C atoms, or a salt thereof, which comprises reacting a compound of the formula

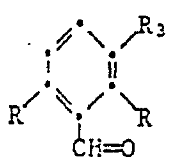

(XX)

in which R is etherified hydroxyl, such as lower alkoxy having not more than 4 C atoms, for example methoxy, in an inert solvent with an etherate of 2,2,2-trifluoro-1,1-dichloro-ethyl-zinc chloride of the formula $CF_3CCl_2ZnClx2(C_2H_5)_2O$ (XXI) to give the corresponding compound of the formula

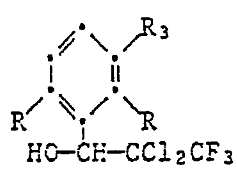

(XXII)

acylating this in the side chain, converting the reaction product of the formula

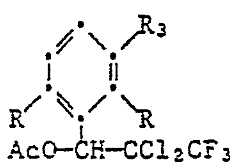

(XXIIa)

in which the radical Ac is the acyl group introduced, by treatment with a metallic reducing agent, into the corresponding compound of the formula

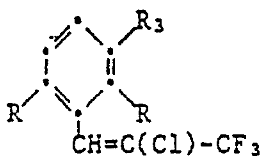

(XXIII)

detaching hydrogen chloride from this product by treatment with a metal base, hydrogenating the side chain in the resulting compound of the formula

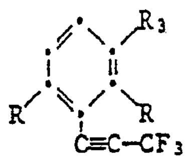

(XXIV)

and splitting the etherified hydroxyl groups R to give hydroxyl, if desired introducing a radical $R_{11}$ of the formula $R_1-C(=O)-$ and if desired converting a resulting free compound of the formula XXXIII into a salt or a resulting salt into the free compound of the formula XXXIII.

19. A process according to claim 17 for the preparation of a compound of the formula XXXIII in which $R_1$, if present, is lower alkyl having not more than 4 C atoms, $R_2$ is ω-fluoro-, ω,ω-difluoro- or ω,ω,ω-trifluorolower alkyl having not more than 4, for example 1 or 3, C atoms and $R_3$ is hydrogen, or a salt thereof.

20. A process according to claim 17 or 18 for the preparation of a compound of the formula XXXIII in which $R_1$, if present, is lower alkyl having not more than 4 C atoms, $R_2$ is ω,ω,ω-trifluoro-lower alkyl having not more than 4 C atoms and $R_3$ is hydrogen, or a salt thereof.

21. The process according to claim 17 or 18 for the preparation of 2-(3,3,3-trifluoropropyl)-resorcinol or a salt thereof.

22. The process according to claim 17 or 18 for the preparation of 4-acetyl-2-(3,3,3-trifluoropropyl)-resorcinol or a salt thereof.

**Revendications pour les Etats contractants: BE, CH, DE, FR, GB, IT, LI, LU, NL, SE**

1. Ether de 4-acylrésorcine 2-fluoroalkylé de formule

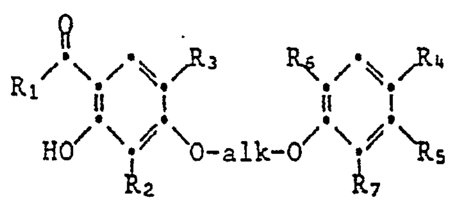

(I),

où $R_1$ représente un alkyle inférieur, $R_2$ représente un alkyle inférieur fluoré, $R_3$ représente l'hydrogène, un alcoxy inférieur, le trifluorométhyle ou un halogène, alk représente un reste alkylène ayant 2 à 9 chaînons inclus et 2 à 9 atomes de C inclus, un reste hydroxyalkylène ayant 3 à 7 chaînons inclus et 3 à 7 atomes de C inclus, un reste alkylène interrompu par l'oxygène ayant 5 à 19 chaînons inclus et 4 à 16 atomes de C inclus ou un reste hydroxyalkylène interrompu par l'oxygène ayant 6 à 11 chaînons inclus et 5 à 9 atomes de C inclus, un des restes $R_4$, $R_5$ et $R_7$ représente un groupe de formule $-NH-C(=O)-R_8$, un reste $R_4$ ou $R_5$ différent de celui-ci représente un reste $R_9$, et un reste $R_7$ différent de celui-ci représente un reste $R_{10}$, $R_6$ représente l'hydrogène, un alkyle inférieur, le trifluorométhyle, un halogène, un carboxy éventuellement estérifié ou sous forme d'amide, un cyano ou un alcanoyle inférieur, $R_8$ représente un carboxy éventuellement estérifié ou sous forme d'amide ou le 5-tétrazolyle, $R_9$ représente l'hydrogène, un alkyle inférieur, un alcoxy inférieur, un halogène ou le trifluorométhyle et $R_{10}$ représente l'hydrogène, un alkyle inférieur, un alcoxy inférieur, un halogène, le trifluorométhyle, un cyano ou un carboxy éventuellement estérifié ou sous forme d'amide, l'halogène pouvant présenter un numéro atomique allant jusqu'à 53 inclus et les groupes "inférieurs" pouvant avoir jusqu'à 7 atomes de C inclus ou l'un de ses sels.

2. Composé selon la revendication 1, où $R_1$ représente un alkyle inférieur, $R_2$ un alkyle inférieur fluoré ayant jusqu'à 3 atomes de fluor inclus, $R_3$ représente l'hydrogène, un alcoxy inférieur le trifluorométhyle ou un halogène, alk représente un alkylène ou un hydroxyalkylène éventuellement interrompu par l'oxygène

de formule $-(CH_2)_m-$ (Ia), $-(CH_2)_{n'}-[O-(CH_2)_n]_k$ (Ib) ou $-[(CH_2)_{l'}-O]_o-CH_2-CH(OH)-CH_2-[O-(CH_2)_l]_p$ (Ic)

où k est égal à 1, 2 ou 3, 1 et 1' sont, indépendamment l'un de l'autre, 2 ou 3, m est un nombre entier allant de 2 à 9 inclus, n et n, sont, indépendamment l'un de l'autre, 2, 3 ou 4 et o et p sont, indépendamment l'un de l'autre, 0 ou 1, un des restes $R_4$, $R_5$ et $R_7$ représente un groupe de formule $-NH-C(=O)-R_8$, un reste $R_4$ ou $R_5$ différent de celui-ci représente un reste $R_9$ et un reste $R_7$ différent de celui-ci représente un reste $R_{10}$, $R_6$ représente l'hydrogène, un alkyle inférieur, le trifluorométhyle, un halogène, un alcanoyle inférieur, le carboxy, un alcoxy inférieur carbonyle, le cyano, le carbamyle, un N-mono- ou N,N-dialkyle inférieur carbamyle, $R_8$ représente, d'une part, le carboxy, un alcoxy inférieur carbonyle, un N,N-dialkyles inférieurs aminoalcoxy inférieur carbonyle, un N,N-alkylène inférieur aminoalcoxy inférieur carbonyle, le carbamyle, un N-mono- ou N,N-dialkyles inférieurs carbamyle, un N,N-alkylène inférieur carbamyle ou un N,N-(aza)alkylène inférieur carbamyle, un N,N-(oxa)alkylène inférieur carbamyle ou un N,N-(thia) alkylène inférieur carbamyle et, d'autre part, le 5-tétrazolyle, $R_9$ représente l'hydrogène, un alkyle inférieur, un alcoxy inférieur, un halogène ou le trifluorométhyle et $R_{10}$ représente l'hydrogène, un alkyle inférieur, un alcoxy inférieur, un halogène, le trifluorométhyle, le carboxy, un alcoxy inférieur carbonyle, le cyano, le carbamyle, un N-mono- ou N,N-dialkyles inférieurs carbamyle, l'halogène pouvant présenter un numéro atomique allant jusqu'à 53 inclus et les groupes "inférieurs" pouvant avoir jusqu'à 7 atomes de C inclus, ou l'un de ses sels.

3. Composé selon la revendication 1, où $R_1$ est un alkyle inférieur ayant jusqu'à 4 atomes de C inclus, $R_2$ représente un ω-fluoro, un ω,ω-difluoro ou un ω,ω,ω-trifluoroalkyle inférieur ayant jusqu'à 4 atomes de C inclus, $R_3$ représente l'hydrogène, $R_4$ représente l'hydrogène, un alkyle inférieur ayant jusqu'à 4 atomes de C inclus, le trifluorométhyle ou un halogène ayant un numéro atomique allant de 17 à 53 inclus, $R_5$ représente un groupe de formule $-NH-C(=O)-R_8$, $R_6$ représente l'hydrogène, un alkyle inférieur ayant jusqu'à 4 atomes de C inclus, un halogène ayant un numéro atomique allant de 17 à 53 inclus, le trifluorométhyle, un alcoxy inférieur carbonyle ayant jusqu'à 5 atomes de C inclus, le cyano ou le carboxy, $R_7$ représente l'hydrogène, un alkyle inférieur ayant jusqu'à 4 atomes de C inclus, un halogène ayant un numéro atomique allant de 17 à 53 inclus, un carbamyle ou un cyano, $R_8$ représente un carboxy, un alcoxy inférieur carbonyle ayant jusqu'à 5 atomes de C inclus ou le 5-tétrazolyle et alk représente un alkylène inférieur à chaîne linéaire ayant 2 à 7 atomes de C inclus ou un hydroxyalkylène inférieur ayant 3 à 7 atomes de C inclus, dans lequel le groupe hydroxy est lié au-delà de la position α et en deçà de la position ω ou l'un de ses sels.

4. Composé selon la revendication 1, où $R_1$ est un alkyle inférieur ayant jusqu'à 4 atomes de C inclus, $R_2$ représente un ω-fluoro, un ω,ω-difluoro- ou un ω,ω,ω-trifluoroalkyle inférieur ayant jusqu'à 4 atomes de C inclus, $R_3$ représente l'hydrogène, $R_4$ représente l'hydrogène, un alkyle inférieur ayant jusqu'à 4 atomes de C inclus, le trifluorométhyle ou un halogène ayant un numéro atomique allant de 17 à 53 inclus, $R_5$ est un groupe de formule $-NH-C(=O)-R_8$, $R_6$ représente l'hydrogène, un alkyle inférieur ayant jusqu'à 4 atomes de C inclus, un halogène ayant un numéro atomique allant de 17 à 53 inclus, le trifluorométhyle ou le cyano, $R_7$ représente l'hydrogène, un alkyle inférieur ayant jusqu'à 4 atomes de C inclus, le cyano ou un halogène ayant un numéro atomique allant de 17 à 53 inclus, $R_8$ représente un carboxy, un alcoxy inférieur carbonyle ayant jusqu'à 5 atomes de C inclus ou le 5-tétrazolyle et alk représente un alkylène inférieur à chaîne linéaire ayant 2 à 7 atomes de C inclus ou un hydroxyalkylène inférieur ayant 3 à 7 atomes de C inclus, dans lequel le groupe hydroxy est lié au-delà de la position α et en-deçà de la position ω ou l'un de ses sels.

5. Composé selon la revendication 1, où $R_1$ représente un alkyle inférieur ayant jusqu'à 4 atomes de C inclus, $R_2$ représente un ω,ω,ω-trifluoroalkyle inférieur ayant jusqu'à 3 atomes de C inclus, $R_3$ représente l'hydrogène, $R_5$ représente un groupe de formule $-NH-C(=O)-R_8$, $R_4$ représente un alkyle inférieur ayant jusqu'à 4 atomes de C inclus, $R_6$ représente un halogène ayant un numéro atomique allant de 17 à 53 inclus ou le cyano, $R_7$ est l'hydrogène, $R_8$ représente le carboxy, un alcoxy inférieur carbonyle ayant jusqu'à 5 atomes de C inclus ou le 5-tétrazolyle et alk représente un alkylène inférieur à chaîne linéaire ayant 2 à 7 atomes de C inclus ou un hydroxyalkylène inférieur ayant 3 à 7 atomes de C inclus, dans lequel le groupe hydroxy est lié au-delà de la position α et en-deçà de la position ω ou l'un de ses sels.

6. Composé selon la revendication 1, où $R_1$ représente un alkyle inférieur ayant jusqu'à 4 atomes de C inclus, $R_2$ représente un ω-fluoro-, un ω,ω-difluoro- ou un ω,ω,ω-trifluoroalkyle inférieur ayant jusqu'à 3 atomes de C inclus, $R_3$ et $R_7$ représentent l'hydrogène, un des restes $R_4$ et $R_6$ représente un alkyle inférieur ayant jusqu'à 4 atomes de C inclus et l'autre reste un halogène ayant un numéro atomique allant de 17 à 53 inclus, $R_5$ est un groupe de formule $-NH-C(=O)-R_8$, $R_8$ représente un carboxy ou le 5-tétrazolyle et alk représente un alkylène inférieur à chaîne droite ayant 2 à 7 atomes de C inclus ou un hydroxyalkylène inférieur ayant 3 à 7 atomes de C inclus, dans lequel le groupe hydroxy est lié au-delà de la position α et en-deçà de la position ω ou l'un de ses sels.

7. N-{{3-{3-[4-acétyl-3-hydroxy-2-(3,3,3-trifluoropropyl)-phénoxy]-propyloxy}}-4-chloro-6-méthylphényl}}-1H-tétrazol-5-carboxamide ou l'un de ses sels selon la revendication 1.

8. N-{{3-{3-[4-acétyl-3-hydroxy-2-(3,3,3-trifluoropropyl)-phénoxy]-propyloxy}-4-bromo-6-méthylphényl}}-1H-tétrazol-5-carboxamide ou l'un de ses sels selon la revendication 1.

9. AcideN-{{3-{3-[4-acétyl-3-hydroxy-2-(3,3,3-trifluoropropyl)-phénoxy]-propyloxy}-4-bromo-6-méthylphényl}}-oxamique ou l'un de ses sels selon la revendication 1.

10. AcideN-{{3-{3-[4-acétyl-3-hydroxy-2-(3,3,3-trifluoropropyl)-phénoxy]-2-hydroxy-propoxy}-4-bromo-6-méthyl-phényl}}-oxamique ou l'un de ses sels selon la revendication 1.

11. N-{{3-{3-[4-acétyl-3-hydroxy-2-(3,3,3-trifluoropropyl)-phénoxy]-2-hydroxy-propoxy}-4-bromo-6-méthyl-phényl}}-1H-tétrazol-5-carboxamide ou l'un de ses sels selon la revendication 1.

12. N-{{3-{3-[4-acétyl-3-hydroxy-2-(3,3,3-trifluoropropyl)-phénoxy]-2-hydroxy-propoxy}-4-chloro-6-méthyl-phényl}}-1H-tétrazol-5-carboxamide ou l'un de ses sels selon la revendication 1.

13. N-{{3-{3-[4-acétyl-3-hydroxy-2-(3,3,3-trifluoropropyl)-phénoxy]-2-hydroxy-propoxy}-4-cyano-6-méthyl-phényl}}-1H-tétrazol-5-carboxamide ou l'un de ses sels selon la revendication 1.

14. Ester méthylique de l'acide N-{{3-{3-[4-acétyl-3-hydroxy-2-(3,3,3-trifluoropropyl)-phénoxy]-propyloxy}-4-bromo-6-méthyl-phényl}}-oxamique, N-{{3{3-[4-acétyl-3-hydroxy-2-(3,3,3-trifluoropropyl)-phénoxy]-propyloxy}-4-fluoro-6-méthyl-phényl}}-1H-tétrazol-5-carboxamide ou l'un de ses sels, N-{{3-{3-[4-acétyl-3-hydroxy-2-(3,3,3-trifluoropropyl)-phénoxy]-2-hydroxypropyloxy}-4-fluoro-6-méthylphényl}}-1H-tétrazol-5-carboxamide ou l'un de ses sels, ester éthylique de l'acide N-{{3-{3-[4-acétyl-3-hydroxy-2-(3,3,3-trifluoropropyl)-phénoxy]-propyloxy}-4-bromo-6-méthyl-phényl}}oxamique ou ester éthylique de l'acide N-{{3-{3-[4-acétyl-3-hydroxy-2-(3,3,3-trifluoropropyl)-phénoxy]-2-hydroxy-propoxy}-4-bromo-6-méthyl-phényl}}-oxamique selon la revendication 1.

15. AcideN-{{3-{3-[4-acétyl-3-hydroxy-2-(3,3,3-trifluoropropyl)-phénoxy]-propoxy}-4-chloro-6-méthyl-phényl}}-oxamique ou l'un de ses sels, acide N-{{3-{3[4-acétyl-3-hydroxy-2-(3,3,3-trifluoropropyl)-phénoxy]-propoxy}-4-fluoro-6-méthyl-phényl}}-oxamique ou l'un de ses sels, acide N-{{3-{3-[4-acétyl-3-hydroxy-2-(3,3,3-trifluoropropyl)-phénoxy]-propoxy}-4-cyano-6-méthyl-phényl}}-oxamique ou l'un de ses sels, ester méthylique de l'acide N-{{3-{3-[4-acétyl-3-hydroxy-2-(3,3,3-trifluoropropyl)-phénoxy]-propoxy}-4-chloro-6-méthyl-phényl}}-oxamique, ester méthylique de l'acide N-{{3-{3-[4-acétyl-3-hydroxy-2-(3,3,3-trifluoropropyl)-phénoxy]-propoxy}-4-fluoro-6-méthyl-phényl}}-oxamique, ester éthylique de l'acide N-{{3-{3-[4-acétyl-3-hydroxy-2-(3,3,3-trifluoropropyl)-phénoxy]-propoxy}-4-chloro-6-méthyl-phényl}}-oxamique ou ester éthylique de l'acide N-{{3-{3-[4-acétyl-3-hydroxy-2-(3,3,3-trifluoropropyl}-phénoxy]-propoxy}-4-fluoro-6-méthyl-phényl}}-oxamique selon la revendication 1.

16. Un composé formant un sel selon l'une des revendications 1, 2 et 6 à 14 sous la forme d'un sel de sodium, de potassium, de triéthanolammonium, de diéthanolammonium, de Tris(hydroxyméthyl)méthylammonium ou de diéthylammonium.

17. Un composé formant un sel selon l'une des revendications 3 à 5 et 15 sous la forme d'un sel de sodium, de potassium, de triéthanolammonium, de diéthanolammonium, de Tris(hydroxyméthyl)méthylammonium ou de diéthylammonium.

18. Un composé selon l'une des revendications 1 à 17 destiné à être utilisé dans un procédé pour le traitement thérapeutique du corps humain ou animal.

19. Une préparation pharmaceutique contenant un composé selon l'une des revendications 1, 2, 6 à 14 et 16 à côte des adjuvants et/ou supports pharmaceutiques usuels.

20. Une préparation pharmaceutique contenant un composé selon l'une des revendications 3 à 5, 15 et 17 à côté des adjuvants et/ou supports pharmaceutiques usuels.

21. Procédé de préparation d'un éther de 4-acylrésorcine 2-fluoro-alkylé de formule I ou de l'un de ses sels selon l'une des revendications 1 à 17, caractérisé

a) en ce que l'on transpose un composé de formule

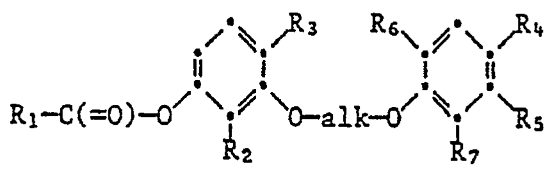

(II)

où

b) en ce que l'on fait réagir un composé de formule

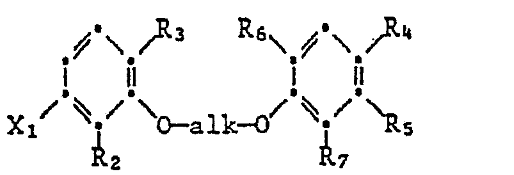

(III),

où X représente un hydroxy éventuellement éthérifié, avec un composé de formule $R_1$–$X_2$ (IV), où $X_2$ représente un carboxy éventuellement fonctionnellement modifié, ou
c) en ce que, dans un composé de formule

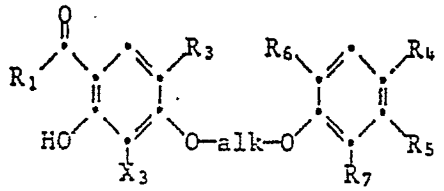

(V)

où $X_3$ représente un reste transformable en $R_2$ ou dans un sel de ce composé, l'on transforme $X_3$ en $R_2$, ou
d) en ce que, dans un composé de formule

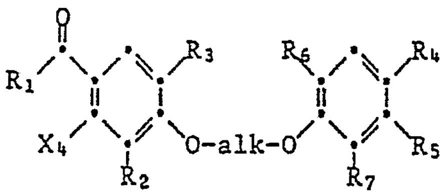

(VI),

où $X_4$ représente un reste transformable en hydroxy, l'on transforme $X_4$ en hydroxy, ou
e) en ce que l'on fait réagir ensemble des composés de formule

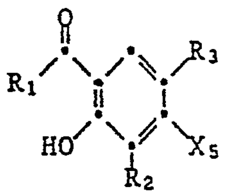

(VII) et

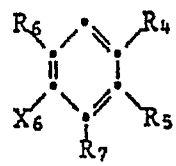

(VIII)

où un des restes $X_5$ et $X_6$ représente un hydroxy se présentant éventuellement sous forme de sel et l'autre reste un reste –Q–alkH substitué par un hydroxy ou un époxy estérifié réactif, c'est-à-dire un reste alcoxy- ou mono-, di- ou trioxaalcoxy substitué par un hydroxy estérifié réactif ou un reste alcoxy- ou mono- ou dioxaalcoxy substitué par un époxy, ou
f) en ce que l'on fait réagir un composé de formule

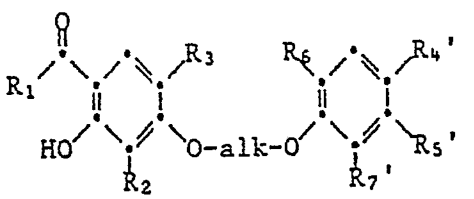

(IX),

où un des restes $R'_4$, $R'_5$ et $R'_7$ représente un groupe amino, un reste $R'_4$ ou $R'_5$ différent de celui-ci représente un reste $R_9$ et un reste $R'_7$ différent de celui-ci représente un reste $R_{10}$ ou un de ses sels, avec un composé de formule

$$X_7-R'_8 \qquad (X),$$

où $R'_8$ représente un groupe carboxy éventuellement estérifié ou mis sous forme d'amide ou le 5-tétrazolyle éventuellement protégé en position 1 et $X_7$ représente un groupe carboxy éventuellement estérifié, mis sous forme d'amide, d'anhydride, ou si $R'_8$ représente le 5-tétrazolyle protégé en position 1, se présentant sous la forme d'un sel et, éventuellement, en ce que l'on clive le groupe protecteur en position 1 du groupe 5-tétrazolyle $R_8$, ou
g) en ce que, dans un composé de formule

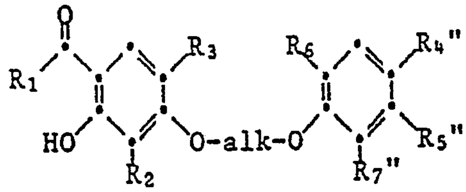

(XI),

où l'un des restes $R''_4$, $R''_5$ et $R''_7$ représente un reste $X_8$, un reste $R''_4$ ou $R''_5$ différent de celui-ci représente un reste $R_9$ et un reste $R''_7$ différent de celui-ci représente un reste $R_{10}$ et $X_8$ représente un reste transformable en un groupe de formule $-NH-C(=O)-R_8$, l'on transforme $X_8$ en ce groupe ou

h) en ce que, dans un composé de formule

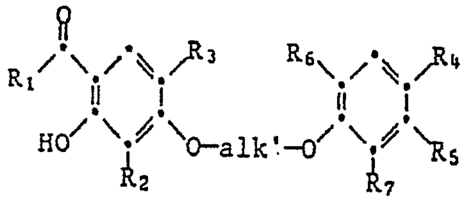

(I'),

où alk, représente un reste transformable en groupe alk, l'on transforme alk' en alk et, si désiré, en ce que l'on transforme un composé de formule I obtenu par le procédé conforme à l'invention en un autre composé de formule I, en ce que l'on sépare le mélange d'isomères obtenu par le procédé conforme à l'invention, en ce que l'on isole le ou les isomère(s) de formule I et/ou, si désiré en ce que l'on transforme un composé libre de formule I obtenu par le procédé conforme à l'invention en un sel ou un sel, obtenu par le procédé conforme à l'invention, en un composé libre de formule I ou en un autre sel.

22. Un composé de formule

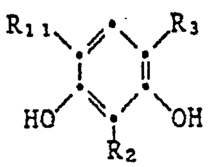

(XXXIII),

où $R_2$ représente un alkyle inférieur fluoré, $R_3$ représente l'hydrogène, un alcoxy inférieur, le trifluorométhyle ou un halogène et $R_{11}$ représente l'hydrogène ou un groupe de formule $R_1-C(=O)-$, dans laquelle $R_1$ représente un alkyle inférieur, l'halogène pouvant présenter un numéro atomique allant jusqu'à 53 inclus et les groupes "inférieurs" pouvant avoir jusqu'à 7 atomes de C inclus ou l'un de ses sels.

23. La 2-(3,3,3-trifluoropropyl)-résorcine ou l'un de ses sels ou la 4-acétyl-2-(3,3,3-trifluoropropyl)-résorcine ou l'un de ses sels selon la revendication 22.

24. Procédé de préparation d'un composé de formule XXXIII ou de l'un de ses sels selon la revendication 22 ou 23, caractérisé en ce que, dans un composé de formule

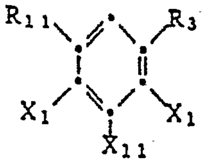

(XXIV),

où au moins l'un des restes $X_1$ représente un hydroxy R éthérifié et un reste $X_1$ différent de celui-ci représente un hydroxy libre et $X_{11}$ représente un groupe échangeable contre $R_2$ ou transformable en $R_2$, on échange $X_{11}$ contre $R_2$ ou on le transforme en $R_2$, on clive l'hydroxy éthérifié R en hydroxy et, si désiré, on transforme le composé de formule XXXIII obtenu conformément au procédé en un autre composé de formule XXXIII, on introduit un groupe de formule $R_1-C(=O)-$, en particulier à la place de l'hydrogène $R_{11}$ et/ou on transforme un composé libre de formule XXXIII obtenu conformément au procédé en un sel ou le sel obtenu conformément au procédé en un composé libre de formule XXXIII.

25. Procédé de préparation d'un composé de formule XXXIII ou de l'un de ses sels selon la revendication 22 ou 23, où $R_2$ représente le 3,3,3-trifluoropropyle, caractérisé en ce que l'on transforme un composé de formule

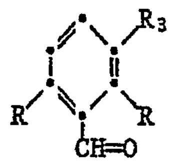

(XX),

où R représente un hydroxy éthérifié tel qu'un alcoxy inférieur ayant jusqu'à 4 atomes de C inclus, par exemple le méthoxy, dans un solvant inerte avec un éthérate de 2,2,2-trifluoro-1,1-dichloro-éthyle chlorure de zinc de formule $CF_3CCl_2ZnClx2(C_2H_5)_2O$ (XXI) en un composé correspondant de formule

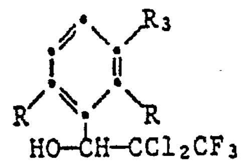

(XXII),

en ce que l'on acyle celui-ci dans la chaîne latérale, en ce que l'on transforme le produit de la réaction de formule

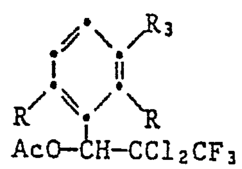

(XXIIa),

où le reste Ac représente le groupe acyle introduit, par traitement avec un agent réducteur métallique en un composé correspondant de formule

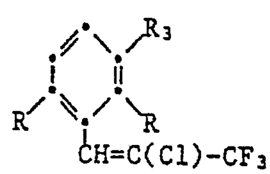

(XXIII)

en ce que l'on clive sur celui-ci le chlorure d'hydrogène, par traitement avec une base métallique, en ce que l'on hydrogène dans le composé obtenu de formule

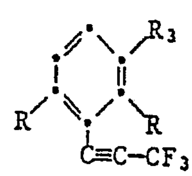

(XXIV),

la chaîne latérale et en ce que l'on clive les groupes hydroxy éthérifiés R en hydroxy et, si désiré, en ce que l'on introduit un reste $R_{11}$ de formule $R_1$–C(=O)– et, si désiré, en ce que l'on transforme le composé libre obtenu de formule XXXIII en un sel ou le sel obtenu en un composé libre de formule XXXIII.

26. Procédé selon la revendication 24 ou 25 pour la préparation de la 2-(3,3,3-trifluoropropyl)-résorcine ou de la 4-acétyl-2-(3,3,3-trifluoropropyl)-résorcine ou l'un de leurs sels.

27. Utilisation d'un composé selon l'une des revendications 1 à 18 pour la préparation de médicaments anti-allergiques et/ou anti-inflammatoires.

**Revendications pour l'Etat contractant: AT**

1. Procédé de préparation d'un éther de 4-acrylrésorcine 2-fluoroalkylé de formule

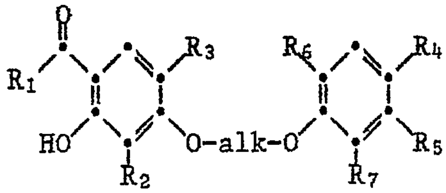

(I),

où $R_1$ représente un alkyle inférieur, $R_2$ représente un alkyle inférieur fluoré, $R_3$ représente l'hydrogène, un alcoxy inférieur, le trifluorométhyle ou un halogène, alk représente un reste alkylène ayant 2 à 9 chaînons inclus et 2 à 9 atomes de C inclus, un reste hydroxyalkylène ayant 3 à 7 chaînons inclus et 3 à 7 atomes de C inclus, un reste alkylène interrompu par l'oxygène ayant 5 à 19 chaînons inclus et 4 à 16 atomes de C inclus ou un reste hydroxyalkylène interrompu par l'oxygène ayant 6 à 11 chaînons inclus et 5 à 9 atomes de C inclus, un des restes $R_4$, $R_5$ et $R_7$ représente un groupe de formule $-NH-C(=O)-R_8$, un reste $R_4$ ou $R_5$ différent de celui-ci représente un reste $R_9$, et un reste $R_7$ différent de celui-ci représente un reste $R_{10}$, $R_6$ représente l'hydrogène, un alkyle inférieur, le trifluorométhyle, un halogène, un carboxy éventuellement estérifié ou sous forme d'amide, un cyano ou un alcanoyle inférieur, $R_8$ représente un carboxy éventuellement estérifié ou sous forme d'amide ou le 5-tétrazolyle, $R_9$ représente l'hydrogène, un alkyle inférieur, un alcoxy inférieur, un halogène ou le trifluorométhyle et $R_{10}$ représente l'hydrogène, un alkyle inférieur, un alcoxy inférieur, un halogène, le trifluorométhyle, un cyano ou un carboxy éventuellement estérifié ou sous forme d'amide, l'halogène pouvant présenter un numéro atomique allant jusqu'à 53 inclus et les groupes "inférieurs" pouvant avoir jusqu'à 7 atomes de C inclus et de son sel, caractérisé

a) en ce que l'on transpose un composé de formule

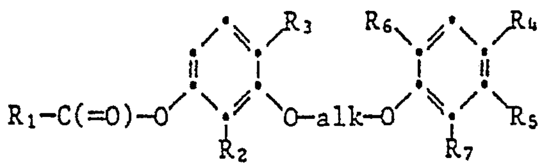

(II),

ou

b) en ce que l'on fait réagir un composé de formule

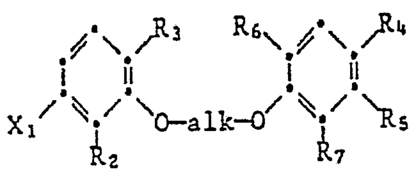

(III),

où $X_1$ représente un hydroxy éventuellement éthérifié, avec un composé de formule $R_1-X_2$ (IV), où $X_2$ représente un carboxy éventuellement fonctionnellement modifié, ou

c) en ce que, dans un composé de formule

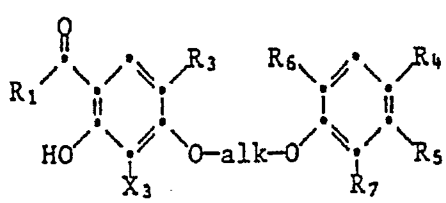

(V),

où $X_3$ représente un reste transformable en $R_2$ ou dans un sel de ce composé, l'on transforme $X_3$ en $R_2$, ou

d) en ce que, dans un composé de formule

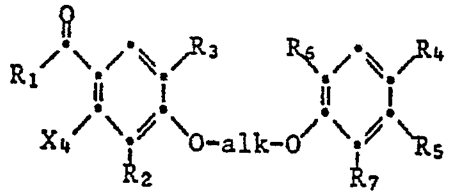

(VI),

où $X_4$ représente un reste transformable en hydroxy, l'on transforme $X_4$ en hydroxy, ou
e) en ce que l'on fait réagir ensemble des composés de formule

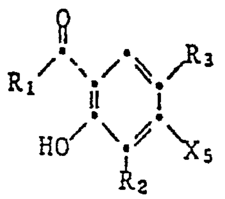

(VII) et

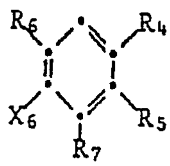

(VIII),

où l'un des restes $X_5$ et $X_6$ représente un hydroxy se présentant éventuellement sous forme de sel et l'autre reste un reste –O–alkH substitué par un hydroxy ou un époxy estérifié réactif, c'est-à-dire un reste alcoxy- ou mono-, di- ou trioxaalcoxy substitué par un hydroxy estérifié réactif ou un reste alcoxy- ou mono- ou dioxaalcoxy substitué par un époxy, ou
f) en ce que l'on fait réagir un composé de formule

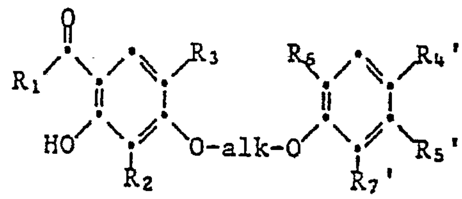

(IX),

où l'un des restes $R'_4$, $R'_5$ et $R'_7$ représente un groupe amino, un reste $R'_4$ ou $R'_5$ différent de celui-ci représente un reste $R_9$ et un reste $R'_7$ différent de celui-ci représente un reste $R_{10}$ ou un de ses sels, avec un composé de formule

$$X_7-R'_8 \qquad (X)$$

où $R'_8$ représente un groupe carboxy éventuellement estérifié ou mis sous forme d'amide ou le 5-tétrazolyle éventuellement protégé en position 1 et $X_7$ représente un groupe carboxy éventuellement estérifié, mis sous forme d'amide, d'anhydride, ou si $R'_8$ représente le 5-tétrazolyle protégé en position 1, se présentant sous la forme d'un sel et, éventuellement, en ce que l'on clive le groupe protecteur en position 1 du groupe 5-tétrazolyle $R_8$, ou
g) en ce que, dans un composé de formule

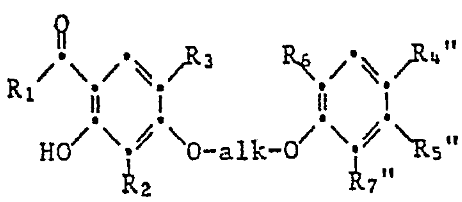

(XI),

où l'un des restes $R''_4$, $R''_5$ et $R''_7$ représente un reste $X_8$, un reste $R''_4$ ou $R''_5$ différent de celui-ci représente un reste $R_9$ et un reste $R''_7$ différent de celui-ci représente un reste $R_{10}$ et $X_8$ représente un reste transformable en un groupe de formule $-NH-C(=O)-R_8$, l'on transforme $X_8$ en ce groupe, ou
h) en ce que, dans un composé de formule

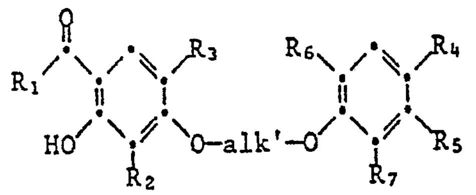

(I'),

où alk, représente un reste transformable en groupe alk, l'on transforme alk' en alk et, si désiré, en ce que l'on transforme un composé de formule I obtenu par le procédé conforme à l'invention en un autre composé de formule I, en ce que l'on sépare le mélange d'isomères obtenu par le procédé conforme à l'invention, en ce que l'on isole le ou les isomère(s) de formule I, et/ou, si désiré, en ce que l'on transforme un composé libre de formule I obtenu par le procédé conforme à l'invention en un sel ou un sel, obtenu par le procédé conforme à l'invention, en un composé libre de formule I ou en un autre sel.

2. Procédé selon la revendication 1 pour la préparation d'un composé de formule I où $R_1$ représente un alkyle inférieur, $R_2$ un alkyle inférieur fluoré ayant jusqu'à 3 atomes de fluor inclus, $R_3$ représente l'hydrogène, un alcoxy inférieur, le trifluorométhyle ou un halogène, alk represente un alkylène ou un hydroxyalkylène éventuellement interrompu par l'oxygène de formule

$$-(CH_2)_m- \ (Ia), \ -(CH_2)_{n'}-[O-(CH_2)_n]_k \ (Ib) \ ou$$
$$-[(CH_2)_{l'}-O]_o-CH_2-CH(OH)-CH_2-[O-(CH_2)_l]_p \ (Ic)$$

où k est égal à 1, 2 ou 3, l et l', sont, indépendamment l'un de l'autre, 2 ou 3, m est un nombre entier allant de 2 à 9 inclus, n et n' sont, indépendamment l'un de l'autre, 2, 3 ou 4 et o et p sont, indépendamment l'un de l'autre, 0 ou 1, un des restes $R_4$, $R_5$ et $R_7$ représente un groupe de formule $-NH-C(=O)-R_8$, un reste $R_4$ ou $R_5$ différent de celui-ci représente un reste $R_9$ et un reste $R_7$ différent de celui-ci représente un reste $R_{10}$, $R_6$ représente l'hydrogène, un alkyle inférieur, le trifluorométhyle, un halogène, un alcanoyle inférieur, le carboxy, un alcoxy inférieur carbonyle, le cyano, le carbamyle, un N-mono- ou N,N-dialkyle inférieur carbamyle, $R_8$ représente, d'une part le carboxy, un alcoxy inférieur carbonyle, un N,N-dialkyles inférieurs aminoalcoxy inférieur carbonyle, un N,N-alkylène inférieur aminoalcoxy inférieur carbonyle, le carbamyle, un N-mono- ou N,N-dialkyles inférieurs carbamyle, un N,N-alkylène inférieur carbamyle ou un N,N-(aza)alkylène inférieur carbamyle, un N,N-(oxa)alkylène inférieur carbamyle ou un N,N-(thia) alkylène inférieur carbamyle et, d'autre part le 5-tétrazolyle, $R_9$ représente l'hydrogène, un alkyle inférieur, un alcoxy inférieur, un halogène ou le trifluorométhyle et $R_{10}$ représente l'hydrogène, un alkyle inférieur, un alcoxy inférieur, un halogène, le trifluorométhyle, le carboxy, un alcoxy inférieur carbonyle, le cyano, le carbamyle, un N-mono- ou N,N-dialkyles inférieurs carbamyle, l'halogène pouvant présenter un numéro atomique allant jusqu'à 53 inclus et les groupes "inférieurs" pouvant avoir jusqu'à 7 atomes de C inclus, ou de l'un de ses sels.

3. Procédé selon la revendication 1, pour la préparation d'un composé de formule I où $R_1$ est un alkyle inférieur ayant jusqu'à 4 atomes de C inclus, $R_2$ représente un ω-fluoro-, un ω,ω-difluoro- ou un ω,ω,ω-trifluoroalkyle inférieur ayant jusqu'à 4 atomes de C inclus, $R_3$ représente l'hydrogène, $R_4$ représente l'hydrogène, un alkyle inférieur ayant jusqu'à 4 atomes de C inclus, le trifluorométhyle ou un halogène ayant un numéro atomique allant de 17 à 53 inclus, $R_5$ représente un groupe de formule $-NH-C(=O)-R_8$, $R_6$ représente l'hydrogène, un alkyle intérieur ayant jusqu'à 4 atomes de C inclus, un halogène ayant un numéro atomique allant de 17 à 53 inclus, le trifluorométhyle, un alcoxy inférieur carbonyle ayant jusqu'à 5 atomes de C inclus, le cyano ou le carboxy, $R_7$ représente l'hydrogène, un alkyle inférieur ayant jusqu'à 4 atomes de C inclus, un halogène ayant un numéro atomique allant de 17 à 53 inclus, un carbamyle ou un cyano, $R_8$ représente un carboxy, un alcoxy inférieur carbonyle ayant jusqu'à 5 atomes de C inclus ou le 5-tétrazolyle et alk représente un alkylène inférieur à chaîne linéaire ayant 2 à 7 atomes de C inclus ou un hydroxyalkylène inférieur ayant 3 à 7 atomes de C inclus, dans lequel le groupe hydroxy est lié au-delà de la position $\alpha$ et en-deçà de la position $\omega$ ou de l'un de ses sels.

4. Procédé selon la revendication 1 pour la préparation d'un composé de formule I où $R_1$ est un alkyle inférieur ayant jusqu'à 4 atomes de C inclus, $R_2$ représente un ω-fluoro-, un ω,ω-difluoro- ou un ω,ω,ω-trifluoroalkyle inférieur ayant jusqu'à 4 atomes de C inclus, $R_3$ représente l'hydrogène, $R_4$ représente l'hydrogène, un alkyle inférieur ayant jusqu'à 4 atomes de C inclus, le trifluorométhyle ou un halogène ayant un numéro atomique allant de 17 à 53 inclus, $R_5$ est un groupe de formule $-NH-C(=O)-R_8$, $R_6$ représente l'hydrogène, un alkyle inférieur ayant jusqu'à 4 atomes de C inclus, un halogène ayant un numéro atomique allant de 17 à 53 inclus, le trifluorométhyle ou le cyano, $R_7$ représente l'hydrogène, un alkyle inférieur ayant jusqu'à 4 atomes de C inclus, le cyano ou un halogène ayant un numéro atomique allant de 17 à 53 inclus, $R_8$ représente un carboxy, un alcoxy inférieur carbonyle ayant jusqu'à 5 atomes de C inclus ou le 5-tétrazolyle et alk représente un alkylène inférieur à cha¨ne linéaire ayant 2 à 7 atomes de C inclus ou un hydroxyalkylène inférieur ayant 3 à 7 atomes de C inclus, dans lequel le groupe hydroxy est lié au-delà de la position a et en-deçà de la position ω ou de l'un de ses sels.

5. Procédé selon la revendication 1 pour la préparation d'un composé de formule I où $R_1$ représente un alkyle inférieur ayant jusqu'à 4 atomes de C inclus, $R_2$ représente un ω,ω,ω-trifluoroalkyle inférieur ayant jusqu'à 3 atomes de C inclus, $R_3$ représente l'hydrogène, $R_5$ représente un groupe de formule –NH–C(=O)$R_8$, $R_4$ représente un alkyle inférieur ayant jusqu'à 4 atomes de C inclus, $R_6$ représente un halogène ayant un numéro atomique allant de 17 à 53 inclus ou le cyano, $R_7$ est l'hydrogène, $R_8$ représente le carboxy, un alcoxy inférieur carbonyle ayant jusqu'à 5 atomes de C inclus ou le 5-tétrazolyle et alk représente un alkylène inférieur à chaîne linéaire ayant 2 à 7 atomes de C inclus ou un hydroxyalkylène inférieur ayant 3 à 7 atomes de C inclus, dans lequel le groupe hydroxy est lié au-delà de la position α et en-deçà de la position ω, ou de l'un de ses sels.

6. Procédé selon la revendication 1 pour la préparation d'un composé de formule I où $R_1$ représente un alkyle inférieur ayant jusqu'à 4 atomes de C inclus, $R_2$ représente un ω-fluoro-, un ω,ω-difluoro- ou un ω,ω,ω-trifluoroalkyle inférieur ayant jusqu'à 3 atomes de C inclus, $R_3$ et $R_7$ représentent l'hydrogène, un des restes $R_4$ et $R_6$ représente un alkyle inférieur ayant jusqu'à 4 atomes de C inclus et l'autre reste un halogène ayant un numéro atomique allant de 17 à 53 inclus, $R_5$ est un groupe de formule –NH–C(=O)–$R_8$, $R_8$ représente un carboxy ou le 5-tétrazolyle et alk représente un alkylène inférieur à chaîne droite ayant 2 à 7 atomes de C inclus ou un hydroxyalkylène inférieur ayant 3 à 7 atomes de C inclus, dans lequel le groupe hydroxy est lié au-delà de la position α et en-deçà de la position ω, ou de l'un de ses sels.

7. Procédé selon la revendication 1 pour la préparation du N-{{3-{3-[4-acétyl-3-hydroxy-2-(3,3,3-trifluoropropyl)-phénoxy]-propyloxy}-4-chloro-6-méthylphényl}}-1H-tétrazol-5-carboxamide ou de l'un de ses sels.

8. Procédé selon la revendication 1 pour la préparation du N-{{3-{3-[4-acétyl-3-hydroxy-2-(3,3,3-trifluoropropyl)-phénoxy]-propyloxy}-4-bromo-6-méthylphényl}}-1H-tétrazol-5-carboxamide ou de l'un de ses sels.

9. Procédé selon la revendication 1 pour la préparation de l'acide N-{{3-{3-[4-acétyl-3-hydroxy-2-(3,3,3-trifluoropropyl)-phénoxy]-propyloxy}-4-bromo-6méthyl-phényl}}-oxamique ou de l'un de ses sels.

10. Procédé selon la revendication 1 pour la préparation de l'acide N-{{3-{3-[4-acétyl-3-hydroxy-2-(3,3,3-trifluoropropyl)-phénoxy]-2-hydroxy-propoxy}-4-bromo-6-méthyl-phényl}}-oxamique ou de l'un de ses sels.

11. Procédé selon la revendication 1 pour la préparation du N-{{3-{3-[4-acétyl-3-hydroxy-2-(3,3,3-trifluoropropyl)-phénoxy]-2-hydroxy-propoxy}-4-bromo-6-méthyl-phényl}}-1H-tétrazol-5-carboxamide ou de l'un de ses sels.

12. Procédé selon la revendication 1 pour la préparation du N-{{3-{3-[4-acétyl-3-hydroxy-2-(3,3,3-trifluoropropyl)-phénoxy]-2-hydroxy-propoxy}-4-chloro-6-méthyl-phényl}}-1H-tétrazol-5-carboxamide ou de l'un de ses sels.

13. Procédé selon la revendication 1 pour la préparation du N-{{3-{3-[4-acétyl-3-hydroxy-2-(3,3,3-trifluoropropyl}-phénoxy]-2-hydroxy-propoxy}-4-cyano-6-méthyl-phényl}}-1H-tétrazol-5-carboxamide ou de l'un de ses sels.

14. Procédé selon la revendication 1 pour la préparation de l'ester méthylique de l'acide N-{{3-{3[4-acétyl-3-hydroxy-2-(3,3,3-trifluoropropyl)-phénoxy]-propyloxy}-4-bromo-6-méthyl-phényl}}-oxamique, du N-{{3-{3-[4-acétyl-3-hydroxy-2-(3,3,3-trifluoropropyl)-phénoxyl-propyloxy}-4-fluoro-6-méthyl-phényl}}-1H-tétrazol-5-carboxamide ou de l'un de ses sels, du N-{{3-{3-[4-acétyl-3-hydroxy-2-(3,3,3-trifluoropropyl)-phénoxy]-2-hydroxy-propyloxy}-4-fluoro-6-méthyl-phényl}}-1H-tétrazol-5-carboxamide ou de l'un de ses sels, de l'ester éthylique de l'acide N-{{3-{3-[4-acétyl-3-hydroxy-2-(3,3,3-trifluoropropyl)-phénoxy]-propyloxy}-4-bromo-6-méthyl-phényl}}-oxamique, de l'ester éthylique de l'acide N-{{3-{3-[4-acétyl-3-hydroxy-2-(3,3,3-trifluoropropyl)-phénoxy]-2-hydroxy-propoxy}-4-bromo-6-méthyl-phényl}}-oxamique, de l'acide N-{{3-{3-[4-acétyl-3-hydroxy-2-(3,3,3-trifluoropropyl)-phénoxy]-propoxy}-4-chloro-6-méthyl-phényl}}-oxamique ou de l'un de ses sels, de l'acide N-{{3-{3-[4-acétyl-3-hydroxy-2-(3,3,3-trifluoropropyl)-phénoxy-propoxy}-4-fluoro-6-méthyl-phényl}}-oxamique ou de l'un de ses sels, de l'acide N-{{3-{3-[4-acétyl-3-hydroxy-2-(3,3,3-trifluoropropyl)-phénoxy]-propoxy}-4-cyano-6-méthyl-phényl}}-oxamique ou de l'un de ses sels, de l'ester méthylique de l'acide N-{{3-{3-[4-acétyl-3-hydroxy-2-(3,3,3-trifluoropropyl)-phénoxy]-propoxy}-4-chloro-6-méthyl-phényl}}-oxamique, de l'ester méthylique de l'acide N-{{3-{3-[4-acétyl-3-hydroxy-2-(3,3,3-trifluoropropyl)-phénoxy]-propoxy}--4-fluoro-6-méthyl-phényl}}-oxamique, de l'ester éthylique de l'acide N-{{3-{3-[4-acétyl-3-hydroxy-2-(3,3,3-trifluoropropyl)-phénoxy]-propoxy}-4-chloro-6-méthyl-phényl}}-oxamique ou de l'ester éthylique de l'acide N-{{3-{3-[4-acétyl-3-hydroxy-2-(3,3,3-trifluoropropyl)-phénoxy]-propoxy}-4-fluoro-6-méthyl-phényl}}-oxamique.

15. Un composé formant un sel selon l'une des revendications 1 à 14 sous la forme d'un sel de sodium, de potassium, de triéthanolammonium, de diéthanolammonium, de Tris(hydroxyméthyl)méthylammonium ou de diéthylammonium.

16. Procédé de préparation d'un composé pharmaceutique, caractérisé en ce que l'on mélange un composé obtenu selon l'une des revendications 1 à 15, avec des adjuvants et/ou des supports pharmaceutiques usuels.

17. Procédé de préparation d'un composé de formule

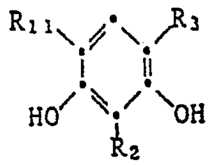

(XXXIII),

où $R_2$ représente un alkyle inférieur fluoré, $R_3$ représente l'hydrogène, un alcoxy inférieur, le trifluorométhyle ou un halogène et $R_{11}$ représente l'hydrogène ou un groupe de formule R1–C(=O)–, dans laquelle $R_1$ représente un alkyle inférieur, l'halogène pouvant présenter un numéro atomique jusqu'à 53 inclus et les groupes "inférieurs " pouvant avoir jusqu'à 7 atomes de C inclus ou l'un de ses sels, caractérisé en ce que, dans un composé de formule

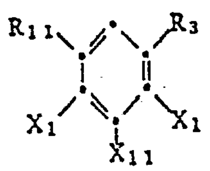

(XXIV),

où au moins l'un des restes $X_1$ représente un hydroxy R éthérifié et un reste $X_1$ différent de celui-ci représente un hydroxy libre et $X_{11}$ représente un groupe échangeable contre $R_2$ ou transformable en $R_2$, on échange $X_{11}$ contre $R_2$ ou on le transforme en $R_2$, on clive l'hydroxy éthérifié R en hydroxy et, si désiré, on transforme le composé de formule XXXIII obtenu conformément au procédé en un autre composé de formule XXXIII, on introduit un groupe de formule $R_1$–C(=O)–, en particulier à la place de l'hydrogène $R_{11}$ et/ou on transforme un composé libre de formule XXXIII obtenu conformément au procédé en un sel ou le sel obtenu conformément au procédé en un composé libre de formule XXXIII.

18. Procédé de préparation d'un composé de formule,

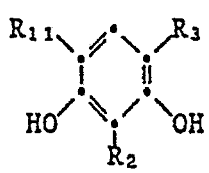

(XXXIII)

où $R_2$ représente le 3,3,3-trifluoropropyle, $R_3$ représente l'hydrogène, un alcoxy inférieur, le trifluorométhyle ou un halogène et $R_{11}$ représente l'hydrogène ou un groupe de formule $R_1$–C(=O)-, dans laquelle $R_1$ représente un alkyle inférieur, l'halogène pouvant présenter un numéro atomique allant jusqu'à 53 inclus et des groupes "inférieurs" pouvant avoir jusqu'à 7 atomes de C inclus ou l'un de ses sels, caractérisé en ce que

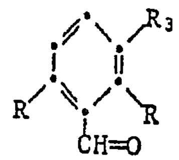

(XX),

où R représente un hydroxy éthérifié tel qu'un alcoxy inférieur ayant jusqu'à 4 atomes de C inclus, par exemple le méthoxy, dans un solvant inerte avec un éthérate de 2,2,2-trifluoro-1,1-dichloro-éthyle chlorure de zinc de formule $CF_3CCl_2ZnClx2(_2H_5)_2O$ (XXI) en un composé correspondant de formule

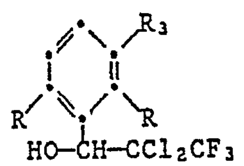

(XXII),

en ce que l'on acyle celui-ci dans la chaîne latérale, en ce que l'on transforme le produit de la réaction de formule

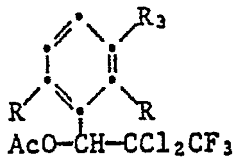

(XXIIa),

où le reste Ac représente le groupe acyle introduit, par traitement avec un agent réducteur métallique en un composé correspondant de formule

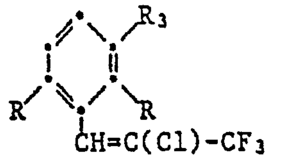

(XXIII),

en ce que l'on clive sur celui-ci le chlorure d'hydrogène, par traitement avec une base métallique, en ce que l'on hydrogène dans le composé obtenu de formule

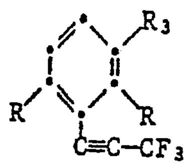

(XXIV),

la chaîne latérale et en ce que l'on clive les groupes hydroxy éthérifiés R en hydroxy et, si désiré, en ce que l'on introduit un reste $R_{11}$ de formule $R_1$–C(=O)– et, si désiré, en ce que l'on transforme le composé libre obtenu de formule XXXIII en un sel ou le sel obtenu en un composé libre de formule XXXIII.

19. Procédé selon la revendication 17 pour la préparation d'un composé de formule XXXIII où $R_1$ s'il est présent, représente un alkyle inférieur ayant jusqu'à 4 atomes de C inclus, $R_2$ représente un ω-fluoro-, un ω,ω-difluoro- ou un ω,ω,ω-trifluoroalkyle inférieur ayant jusqu'à 4 atomes de C inclus, par exemple 1 ou 3 et $R_3$ représente l'hydrogène ou de l'un de ses sels.

20. Procédé selon la revendication 17 ou 18 pour la préparation d'un composé de formule XXXIII où $R_1$, s'il est présent, représente un alkyle inférieur ayant jusqu'à 4 atomes de C inclus, $R_2$ représente un ω,ω,ω-trifluoroalkyle inférieur ayant jusqu'à 4 atomes de C inclus et $R_3$ représente l'hydrogène ou de l'un de ses sels.

21. Procédé selon la revendication 17 ou 18 pour la préparation de la 2-(3,3,3-trifluoropropyl)résorcine ou de l'un de ses sels.

22. Procédé selon la revendication 17 ou 18 pour la préparation de la 4-acétyl-2-(3,3,3-trifluoropropyl)-résorcine ou de l'un de ses sels.